# EUROPEAN PATENT SPECIFICATION

(11) **EP 2 364 369 B1**
(45) Date of publication and mention of the grant of the patent: **29.07.2015**
(21) Application number: 09795744.3
(22) Date of filing: 10.12.2009
(51) Int. Cl.: C12Q 1/68

(54) **NEUROBLASTOMA PROGNOSTIC MULTIGENE EXPRESSION SIGNATURE**
MULTIGEN-EXPRESSIONSSIGNATUR ZUR PROGNOSE VON NEUROBLASTOM
SIGNATURE D EXPRESSION MULTIGÉNIQUE POUR LE PRONOSTIC DE NEUROBLASTOME

(30) Priority: 10.12.2008 EP 08171163
(43) Date of publication of application: 14.09.2011
(73) Proprietor: Universiteit Gent, 9000 Gent (BE)
(72) Inventor: VANDESOMPELE, Joke, B-9870 Zulte (BE); DE PRETER, Katleen, B-Sint-Niklaas 9100 (BE); MESTDAGH, Pieter, B-8000 Brugge (BE); SPELEMAN, Franki, B-2600 Berchem (BE); VERMEULEN, Joëlle, B-1082 Brussel (BE)
(74) Representative: Vanhalst, Koen
(86) International application number: PCT/EP2009/066866
(87) International publication number: WO 2010/066851

(56) References cited:
- US-A1- 2006 105 360
- US-A1- 2008 026 951
- CHEN YONGXIN ET AL: "Differential patterns of microRNA expression in neuroblastoma are correlated with prognosis, differentiation, and apoptosis" CANCER RESEARCH, vol. 67, no. 3, February 2007 (2007-02), pages 976-983, XP002540200 ISSN: 0008-5472 cited in the application
- GRANDINETTI KATHRYN B ET AL: "MicroRNA expression patterns differ between human neuroblastoma cell types with different malignant potentials and N-myc amplification status." PROCEEDINGS OF THE AMERICAN ASSOCIATION FOR CANCER RESEARCH ANNUAL MEETING, vol. 47, April 2006 (2006-04), pages 641-642, XP002540201 & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- SCHULTE J H ET AL: "MicroRNAs in the pathogenesis of neuroblastoma" CANCER LETTERS, NEW YORK, NY, US, vol. 274, no. 1, 17 July 2008 (2008-07-17) , pages 10-15, XP025817755 ISSN: 0304-3835 [retrieved on 2008-07-17] cited in the application
- TAJIRI ET AL: "Biological diagnosis for neuroblastoma using the combination of highly sensitive analysis of prognostic factors" JOURNAL OF PEDIATRIC SURGERY, W. B. SAUNDERS COMPANY, US, vol. 41, no. 3, 1 March 2006 (2006-03-01), pages 560-566, XP005312152 ISSN: 0022-3468
- OHIRA M ET AL: "Expression profiling and characterization of 4200 genes cloned from primary neuroblastomas: identification of 305 genes differentially expressed between favorable and unfavorable subsets" ONCOGENE, NATURE PUBLISHING GROUP, GB BASINGSTOKE, HANTS, vol. 22, 1 August 2003 (2003-08-01), pages 5525-5536, XP002984256 ISSN: 0950-9232
- WEI J S ET AL: "Prediction of Clinical Outcome Using Gene Expression Profiling and Artificial Neural Networks for Patients with Neuroblastoma" CANCER RESEARCH, AMERICAN ASSOCIATION FOR CANCER RESEARCH, BALTIMORE, MD., US, vol. 64, 1 October 2004 (2004-10-01), pages 6883-6891, XP002997547 ISSN: 0008-5472
- OBERTHUER ANDRÉ ET AL: "Customized oligonucleotide microarray gene expression-based classification of neuroblastoma patients outperforms current clinical risk stratification." JOURNAL OF CLINICAL ONCOLOGY : OFFICIAL JOURNAL OF THE AMERICAN SOCIETY OF CLINICAL ONCOLOGY 1 NOV 2006 LNKD- PUBMED:17075126, vol. 24, no. 31, 1 November 2006 (2006-11-01), pages 5070-5078, XP002578135 ISSN: 1527-7755 cited in the application
- WANG QUN ET AL: "Integrative genomics identifies distinct molecular classes of neuroblastoma and shows that multiple genes are targeted by regional alterations in DNA copy number" CANCER RESEARCH, vol. 66, no. 12, June 2006 (2006-06), pages 6050-6062, XP002578136 ISSN: 0008-5472
- SCHRAMM ALEXANDER ET AL: "Prediction of clinical outcome and biological characterization of neuroblastoma by expression profiling" ONCOGENE, vol. 24, no. 53, November 2005 (2005-11), pages 7902-7912, XP002578137 ISSN: 0950-9232
- BERWANGER B ET AL: "Loss of a FYN-regulated differentiation and growth arrest pathway in advanced stage neuroblastoma" CANCER CELL, CELL PRESS, US LNKD- DOI:10.1016/S1535-6108(02)00179-4, vol. 2, no. 5, 1 November 2002 (2002-11-01), pages 377-386, XP002423895 ISSN: 1535-6108
- OHIRA MIKI ET AL: "Expression profiling using a tumor-specific cDNA microarray predicts the prognosis of intermediate risk neuroblastomas" CANCER CELL, vol. 7, no. 4, April 2005 (2005-04), pages 337-350, XP002578138 ISSN: 1535-6108
- DE PRETER KATLEEN ET AL: "Human fetal neuroblast and neuroblastoma transcriptome analysis confirms neuroblast origin and highlights neuroblastoma candidate genes." GENOME BIOLOGY 2006 LNKD- PUBMED:16989664, vol. 7, no. 9, 2006, page R84, XP002578139 ISSN: 1465-6914
- MCARDLE L ET AL: "Oligonucleotide microarray analysis of gene expression in neuroblastoma displaying loss of chromosome 11q" CARCINOGENESIS (OXFORD), vol. 25, no. 9, September 2004 (2004-09), pages 1599-1609, XP002578140 ISSN: 0143-3334
- CHEN QING-RONG ET AL: "An integrated cross-platform prognosis study on neuroblastoma patients" GENOMICS, vol. 92, no. 4, October 2008 (2008-10), pages 195-203, XP002578141 ISSN: 0888-7543
- "GeneAnnot search results of platforms HG-U95, HG-U133, HG-U133 Plus 2.0", , Retrieved from the Internet: URL:http://www.genecards.weizmann.ac.il/cg i-bin/geneannot/GA_search.pl [retrieved on 2012-03-08]
- NCBI: "Gene Expression Omnibus, Platforms GPL91, GPL96, GPL570.", , Retrieved from the Internet: URL:http://www.ncbi.nlm.nih.gov/geo/query/ acc.cgi [retrieved on 2012-03-09]

## Description

### FIELD OF THE INVENTION

The current invention lies in the medical field, more particularly in the field of medical diagnostics. The invention provides tools and methods for prognostic assessment of neuroblastoma patients.

### BACKGROUND OF THE INVENTION

Neuroblastoma (NB) is one of the most frequent solid tumors in children. The neoplasm is characterized by a remarkable genetic heterogeneity that underlies the observed clinical variability, ranging from spontaneous regression to widespread metastasis and fatal outcome for the patient. Current therapeutic stratification of NB patients is based on risk estimation according to a combination of parameters such as age at diagnosis, tumor stage, *MYCN* gene copy number, DNA ploidy status and histopathology. Clinical experience with this system suggests that this stratification of patients for treatment is useful, but patients with the same clinicopathological parameters, receiving the same treatment, can have markedly different clinical courses. Consequently, patients with an intrinsic poor prognosis classified as low risk with current stratification system will receive inappropriate mild treatment and this could lead to a loss of valuable time prior to installing the required, more intensive treatment. Within the current high-risk treatment group, survival rates remain disappointingly low. Therefore the challenge is to identify additional tumor-specific prognostic markers to improve risk estimation at the time of diagnosis and to refine therapeutic decision making. Only then, patients will receive the most appropriate therapy, can be monitored more intensively if needed, and become eligible for new experimental therapies.

As differences in final patient outcome are considered to reflect underlying genetic and biological characteristics that can be analysed at the mRNA gene expression level, several microarray expression profiling studies have been undertaken in order to identify prognostic signatures that can predict neuroblastoma patient outcome. However, an important limitation of these published gene expression profiling studies is the lack of statistical power to identify reliable prognostic markers or classifiers. Typically, around 30,000-40,000 genes are tested in a relatively small subset of tumors (20-100), generating enormous amounts of data for prognostic assessment. As such, there are a few inherent but often overlooked statistical issues, such as data over-fitting, unstable gene lists and lack of study power. Consequently, for any small set of tumors, a gene classifier can be easily established which works fine as a prognostic classifier on the sample set for which it was designed, but often has little or no utility for an independent set of tumor samples. This is clearly illustrated by the lack of overlap between the different published gene sets. Most published prognostic classifiers indeed have not been tested in independent datasets that were obtained on other analysis platforms.

In US patent application US 2006/0263784 for instance, a prognostic signature of 37 genes is disclosed, which was built on a very small set of tumor samples (i.e. 23 samples, 8HR and 15 LR) using a micro-array comprising 10.000 genes. Using PAM they selected 37 differently expressed genes. These were validated on the same 23 samples and only 6 independent samples

Similarly, in US patent application US 2005/0059001, a 366-gene signature is claimed, tested on far less samples, resulting in poor statistical relevance.

Further, European Patent application EP 1 683 862 discloses a gene signature of 25 to 45 probes for good and 25 to 45 probes for poor prognosis out of 96 genes. These were derived from a 200 element cDNA chip set of which 96 genes were up-regulated in good prognosis en 104 genes were up-regulated in poor prognosis neuroblastoma samples. Again, these gene signatures were not validated in an independent sample set or on another measurement platform resulting in unproven prognostic power and accuracy.

Wei et al. (Clinical Cancer Research 2004:6883) discloses a validated set of 19 markers for predicting the clinical outcome in neuroblastoma.

The present invention has overcome these drawbacks by testing an unprecedented large number of relatively rare tumor samples with a rationally selected restricted number of prognostic genes and by validating the signature on a large independent sample set in a blind study. This has significantly improved the statistical power which is lacking in most current gene expression studies. Furthermore the present invention discloses 12 mRNA genes which have, surprisingly, never been linked to neuroblastoma patient's prognosis. Six (6) of these 12 genes are part of preferred signatures which are linked to prognosis and involve a total of 42 or 59 genes. The other six (6) of the latter 12 genes are part of one of said preferred signatures: namely the one involving 59 mRNA genes.

Underlying genetic and biological characteristics of tumors can also be obtained using miRNA/ncRNAs (microRNA/non-coding RNA) expression profiling analysis as demonstrated in other cancer entities. Moreover cumulating evidence indicates that alterations in miRNAs expression play a critical role in tumorigenesis. For example, Chen et al. (Cancer Research 2007: 976), Schulte et al. (Cancer Letters 2009: 10) and Bray et al. (Plos One 2009: e7850) disclose the relationship between miRNA expression patterns and prognosis/pathogenesis of neuroblastoma. In this regard, the present invention further discloses 7 miRNAs which have, surprisingly, never been linked to neuroblastoma. All 7 miRNAs are part of a preferred signature which is linked to prognosis and involves a total of 25 miRNAs. Furthermore, two (2) and five (5) of said 7 miRNAs are part of other preferred signatures linked to neuroblastoma and involving 13 and 12 miRNAs, respectively.

Furthermore, a combined signature including both mRNAs and miRNAs can result in a highly accurate prognostic classification of tumors. The present invention also discloses signatures combining both of said profiles.

### SUMMARY OF THE INVENTION

The invention is defined in the appended claims.

The inventors have developed molecular signatures to predict progression free and overall survival in patients with neuroblastoma (NB). These signatures involve at least 6 of 12 mRNA's and/or at least 2 of 7 miRNAs which have never been linked to neuroblastoma and which can be used to accurately predict neuroblastoma patients' outcome, i.e. progression, relapse as well as death of disease.

In particular, the inventors have developed signatures of 42 mRNAs involving 6 of the above-cited 12 genes and of 59 genes involving all 12 of said genes to be linked with neuroblastoma's prognosis.

The inventors further established a list of 7 miRNAs and signatures of 12, 13 or 25 miRNAs including at least 2 of said list of 7 miRNAs which significantly correlate to the patients' outcome.

Moreover, both of the above-cited mRNA-based signatures and miRNA-based signatures can be integrated or combined to predict the outcome of neuroblastoma.

In essence the invention provides several neuroblastoma prognostic methods and tools with improved statistical power, resulting in independence from the tumor sample pool that was made to design them. The major strength of the prognostic tools provided by the current invention is that they can be easily implemented in the clinic, that they were validated on an independent series of tumors in a blind study, that they have high accuracy in predicting patient outcome, that they have independent prognostic power compared to current clinical risk factors such as age, *MYCN* amplification and tumor stage, and that they can identify patients with an increased risk for poor outcome within the current treatment groups which will help in fine tuning the treatment regimes of neuroblastoma patients.

The invention thus provides a kit for prognostic stratification of neuroblastoma patients comprising means for analyzing the expression level of the following 6 mRNAs: *CAMTA2, EPHA5, EPN2, PLAGL1, PTPRN2* and *SNAPC1,* and/or means for analyzing the expression level of the following 2, 5 or 7 /miRNAs, respectively : hsa-mir-628 and hsa-mir-500, or hsa-mir-345, hsa-mir-320, hsa-mir-485-5p, hsa-mir-542-3p and hsa-mir-192, or the combined list (2+5) hsa-mir-628, hsa-mir-345, hsa-mir-500, hsa-mir-320, hsa-mir-485-5p, hsa-mir-542-3p and hsa-mir-192. Hence, the latter kit relates to the expression of at least 6 mRNAs and/or at least 2, 5 or 7 miRNAs.

In a preferred embodiment, said kit of the invention further comprises means for analyzing the expression level of mRNA from all of the following 6 additional mRNA genes: *MAP2K4, MTSS1, PLAT, PRDM2, PTPRF* and *QPCT.* Hence, the latter kit relates to the expression of at least 12 mRNA genes.

In a further preferred embodiment, said kit of the invention further comprises means for analyzing the expression level of mRNA from all of the following 36 additional coding mRNA genes: AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2 and WSB1. Hence, the latter kit relates to the expression of at least 42 mRNA genes.

In still another preferred embodiment, the latter kit of the invention further comprises means for analyzing the expression level of mRNA from all of the following 17 additional genes: CAMTA1, CD44, CHD5, ELAVL4, MAP2K4, MTSS1, NHLH2, PDE4DIP, PIK3R1, PLAT, PRAME, PRDM2, PTPRF, PTPRH, QPCT, SLC6A8 and TNFRSF25. Hence, the latter kit relates to the expression of at least 59 mRNA genes.

In another specific embodiment the kit of the invention further comprises, besides the means for analyzing the expression of 5 miRNAs: hsa-mir-345, hsa-mir-320, hsa-mir-485-5p, hsa-mir-542-3p and hsa-mir-192, also means for analyzing the expression level of the following 7 additional miRNAs: hsa-mir-25, hsa-mir-15b, hsa-mir-326, hsa-mir-93, hsa-mir-572, hsa-mir-17-5p and hsa-mir-20b. Hence, the latter kit relates to the expression of at least 12 miRNAs.

In another specific embodiment the kit of the invention further comprises, besides the means for analyzing the expression of hsa-mir-628 and hsa-mir-500, also means for analyzing the expression level of the following 11 additional miRNAs: hsa-mir-92, hsa-mir-26a, hsa-mir-30c, hsa-mir-190, hsa-mir-204, hsa-mir-488, hsa-mir-125b, hsa-mir-18a*, hsa-mir-20a, hsa-mir-19a and hsa-mir-18a. Hence, the latter kit relates to the expression of at least 13 miRNAs.

In another specific embodiment the kit of the invention further comprises, besides the means for analyzing the expression of hsa-mir-628, hsa-mir-345, hsa-mir-500, hsa-mir-320, hsa-mir-485-5p, hsa-mir-542-3p and hsa-mir-192, also means for analyzing the expression level of the following 18 additional miRNAs: hsa-mir-92, hsa-mir-26a, hsa-mir-30c, hsa-mir-190, hsa-mir-204, hsa-mir-488, hsa-mir-125b, hsa-mir-18a*, hsa-mir-20a, hsa-mir-19a, hsa-mir-18a, hsa-mir-25, hsa-mir-15b, hsa-mir-326, hsa-mir-93, hsa-mir-572, hsa-mir-17-5p and hsa-mir-20b. Hence, the latter kit relates to the expression of at least 25 miRNAs.

In any of the above embodiments of the kit said means for detecting the expression level comprises one or more oligonucleotide(s) which is (are) specific for each of the target genes and/or miRNAs for use in hybridization-based analysis, microarray, digital gene expression (DGE), RNA-in-situ hybridization (RISH), Northern-blot analysis and the like. Alternatively, said means for detecting the expression level can be a primer pair specific for each of the target genes and/or miRNAs for use in PCR, RT-PCR, RT-qPCR, end-point PCR, digital PCR or the like. In a further alternative embodiment, said means for detecting the expression level is suitable for sequence-analysis based expression analysis specific for each of the target genes and/or miRNAs selected from the group of: Supported oligonucleotide detection, Pyrosequencing, Polony Cyclic Sequencing by Synthesis, Simultaneous Bi-directional Sequencing, Single-molecule sequencing, Single molecule real time sequencing, True Single Molecule Sequencing, Hybridization-Assisted Nanopore Sequencing and Sequencing by synthesis.

In addition to kits as defined above, the invention further provides for a method for prognostic stratification of neuroblastoma patients comprising the steps of:
a) providing a sample from the patient,
b) analyzing the expression level of the mRNAs and/or miRNAs which are part of the kits as indicated above,
c) comparing the expression level obtained in step b) with the expression level as established for neuroblastoma tumors with poor prognosis and with the expression level as established for neuroblastoma tumors with good prognosis, and
d) determining whether the expression level(s) as determined in step b) correlate(s) with the expression levels in neuroblastoma tumors with either good or poor prognosis, thereby stratifying the patient into the group of good prognosis or poor prognosis.

In the method of the invention the sample can be selected from the group comprising: tumor tissue (fresh frozen or formalin-fixed and paraffin-embedded), bone-marrow, bodily fluids, blood, serum, plasma, cerebrospinal fluid, peritoneal fluid and intraperitoneal fluid, wherein tumor samples, blood components and bone-marrow are preferred and tumor samples are the most preferred embodiments.

In the method of the invention, the expression analysis is performed using any one of the technologies selected from the group comprising: Polymerase Chain Reaction (PCR), Real-Time quantitative PCR (RT-qPCR), End-Point PCR, digital PCR (dPCR), RNA or cDNA hybridization techniques, microarrays, RNA-in-situ hybridization (RISH), Northern-Blotting, digital gene expression (DGE), sequence-analysis based expression analysis, Supported oligonucleotide detection, Pyrosequencing, Polony Cyclic Sequencing by Synthesis, Simultaneous Bi-directional Sequencing, Single-molecule sequencing, Single molecule real time sequencing, True Single Molecule Sequencing, Hybridization-Assisted Nanopore Sequencing or Sequencing by synthesis, wherein microarrays and PCR are the preferred embodiments and RT-qPCR is the most preferred embodiment.

The method according to any one of the above defined embodiments can be performed on samples of patients that were previously diagnosed as being high risk patients, intermediate risk patients, low risk patients based on currently available risk factors or were not classified previously.

In addition, the invention further provides a neuroblastoma prognostic stratifying tool comprising:
a) a neuroblastoma prognostic classifier comprising information regarding the neuroblastoma expression levels of the mRNAs and/or miRNAsmiRNAs which are part of any of the kits as indicated above and which are all correlated with poor- or good prognosis when overexpressed, and
b) means for assessing the expression level of one or more of the genes and/or miRNAs which are part of any of the kits according to the present invention in a sample from a patient.

Additionally, the known neuroblastoma stratification or classifying tools such as tumor stage, age and *MYCN* copy number and the like can be combined with the methods, kits and tools according to the invention.

### BRIEF DESCRIPTION OF THE DRAWINGS

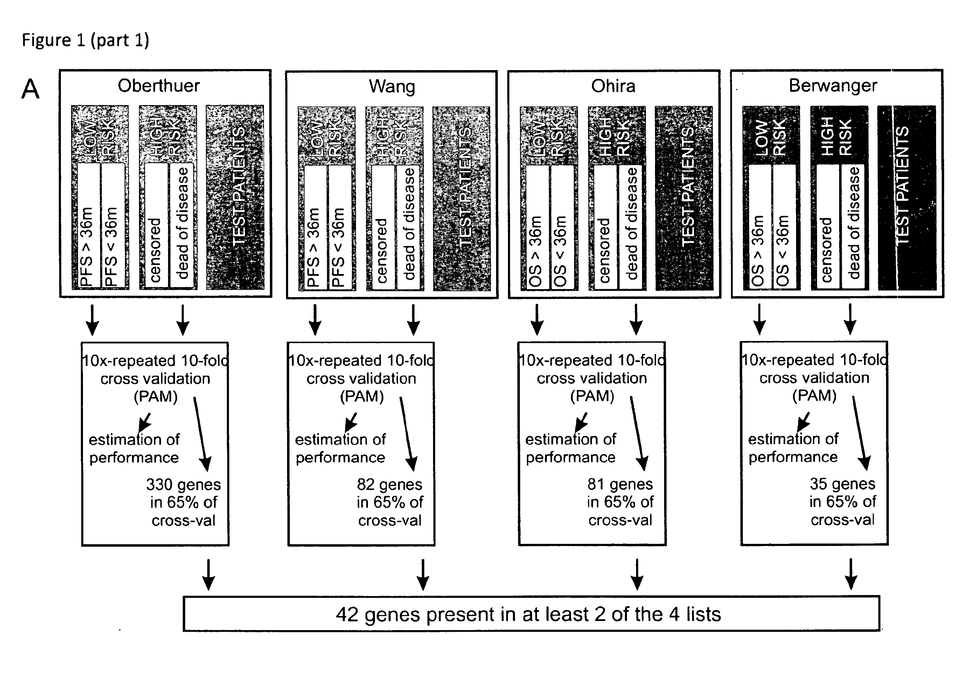

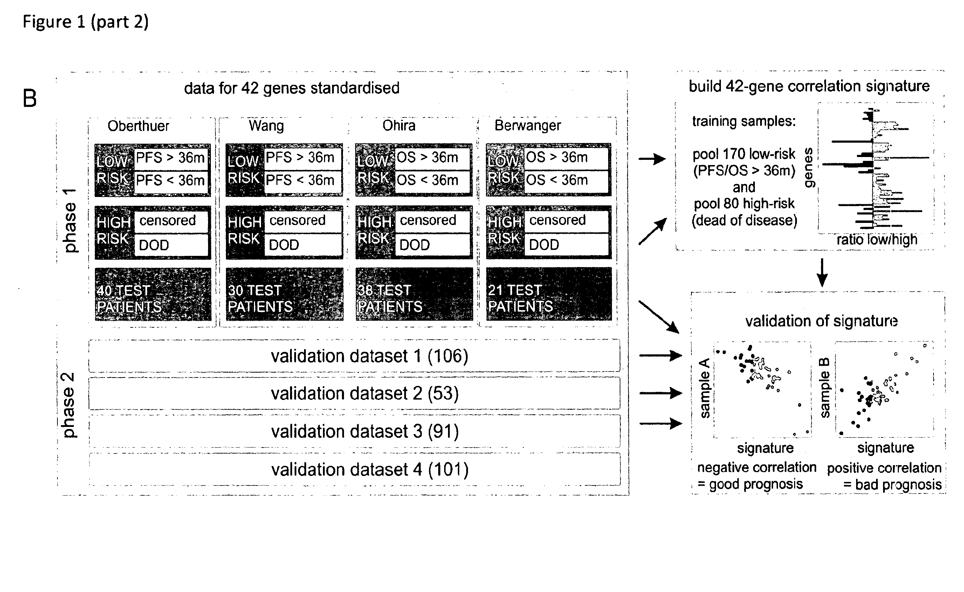

**Figure 1****:** Outline of the strategy used for prioritization of the 42 prognostic gene list (A) and construction of a 42-gene correlation signature and validation on independent test samples from phase 1 studies and phase 2 validation datasets (legend: m = months, OS = overall survival, PFS= progression free survival) (B)

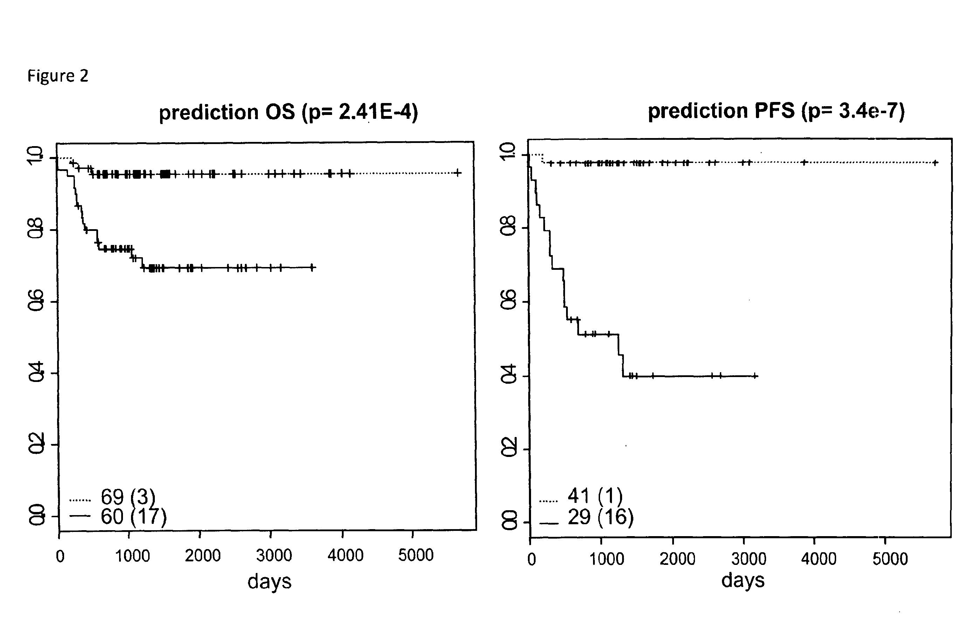

**Figure 2****:** Kaplan-Meier and log-rank analysis of 129 test patients (overall survival) and 70 test patients (progression-free survival) from the four published phase 1 studies classified using the prognostic correlation signature (legend: number of patients in predicted subgroups, between brackets: number of patients with event (relapse, progression or death))

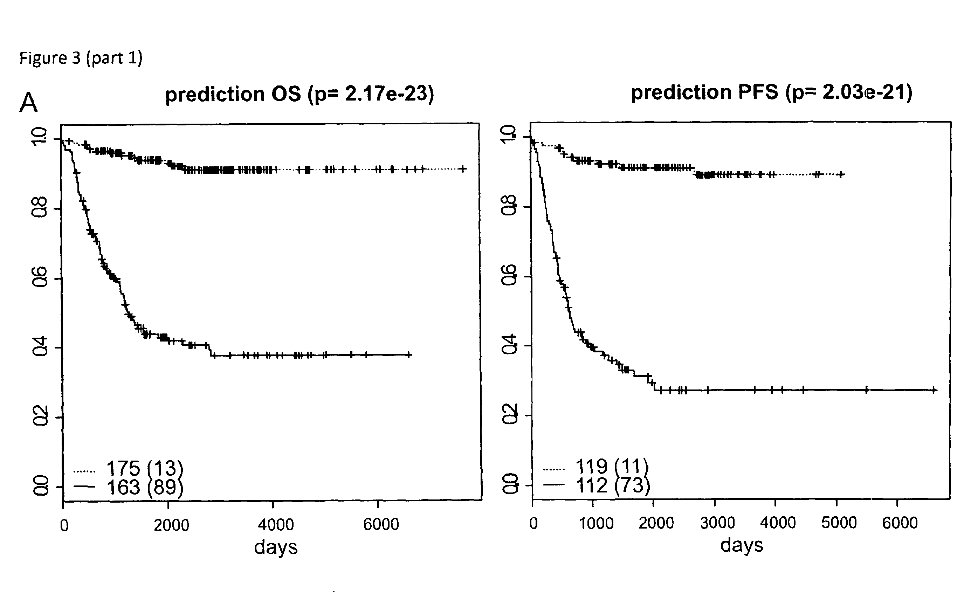

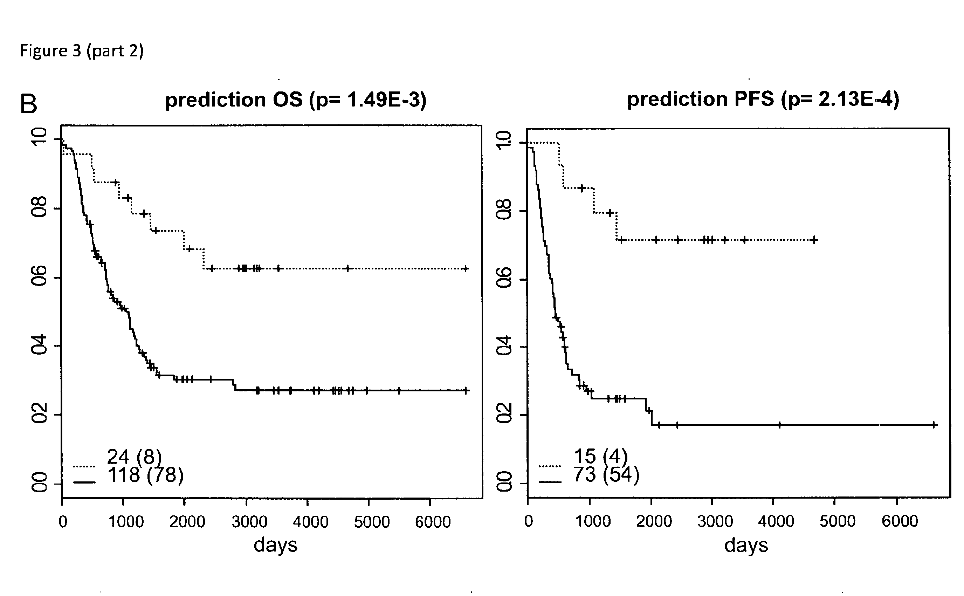

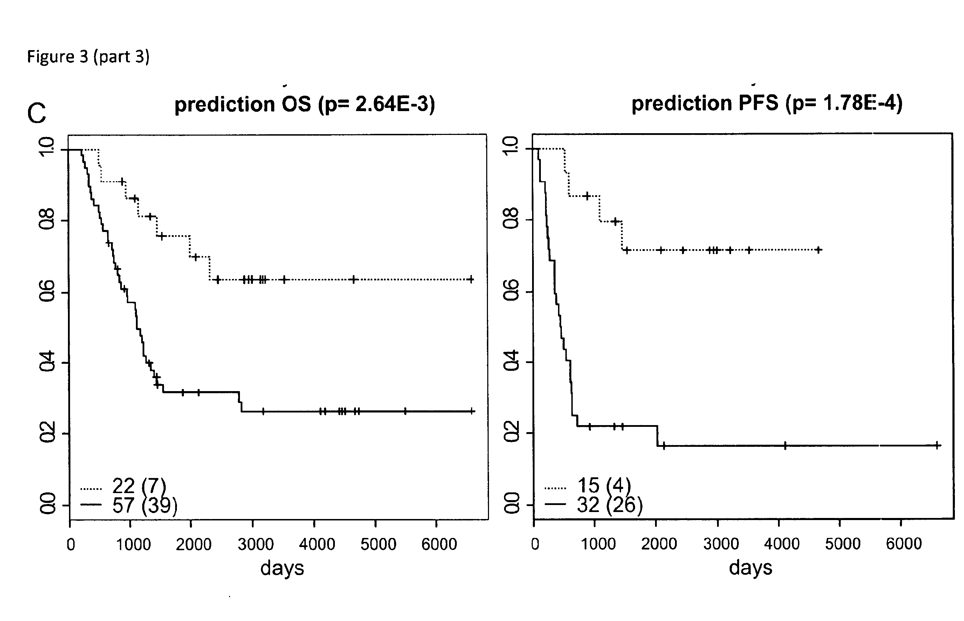

**Figure 3****:** Kaplan-Meier and log-rank analysis of the patients from 4 independent unpublished phase 2 validation datasets classified using the prognostic correlation signature for all patients together (5y OS: 93.9% (95%Cl 90.2-97.6) for low molecular risk (LR) vs 43.1% (95%Cl 35.6-52.2) for high molecular risk (HR) and 5y PFS: 91.1% (95%Cl 86.0-96.6) for LR vs 30.4% (95%Cl 22.1-41.8) for HR) (A), for the common high-risk subgroup (B) and for the common high-risk subgroup without MYCN amplification (C) (legend: number of patients in predicted subgroups, between brackets: number of patients with event (relapse, progression or death))

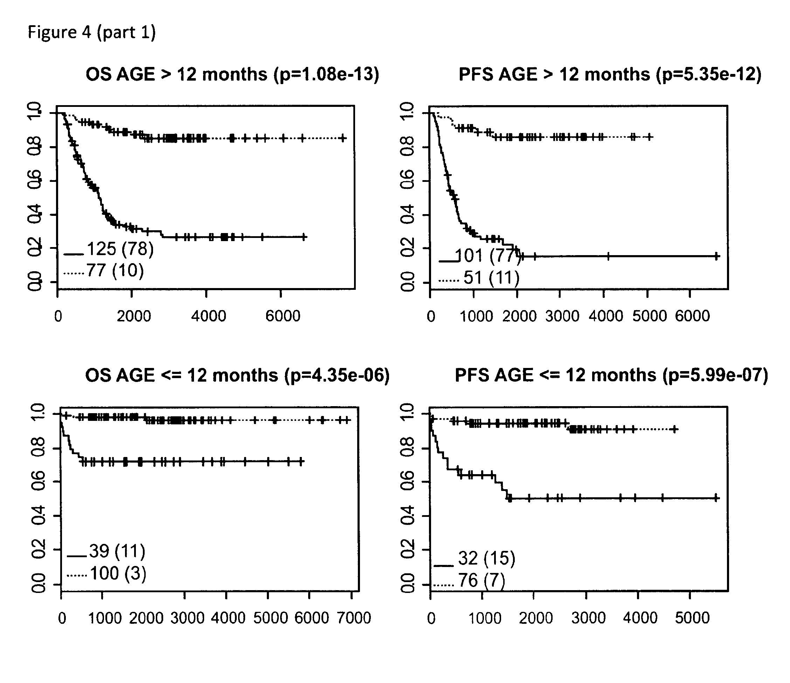

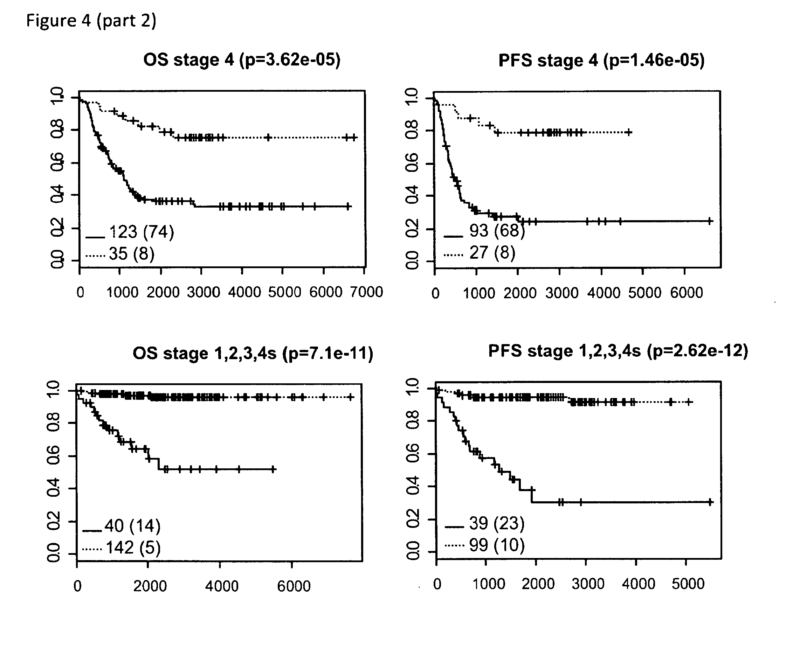

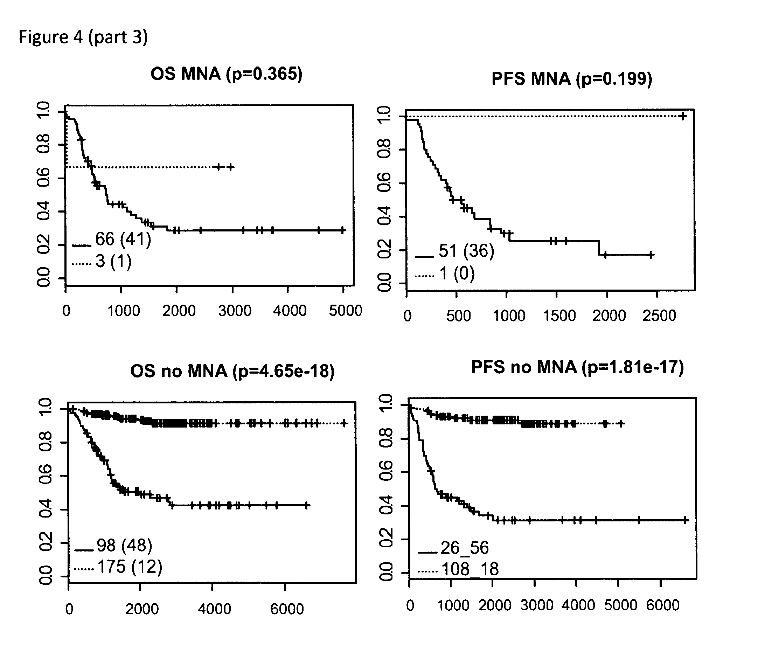

**Figure 4****:** Kaplan-Meier and log-rank analysis for overall survival (OS) and progression free survival (PFS) of the patients from the four independent unpublished phase 2 validation datasets classified using the prognostic correlation signature and stratified according to the known risk factors (stage, age and MYCN status) (legend: number of patients in predicted subgroups, between brackets: number of patients with event (relapse, progression or death), MNA = MYCN amplification)

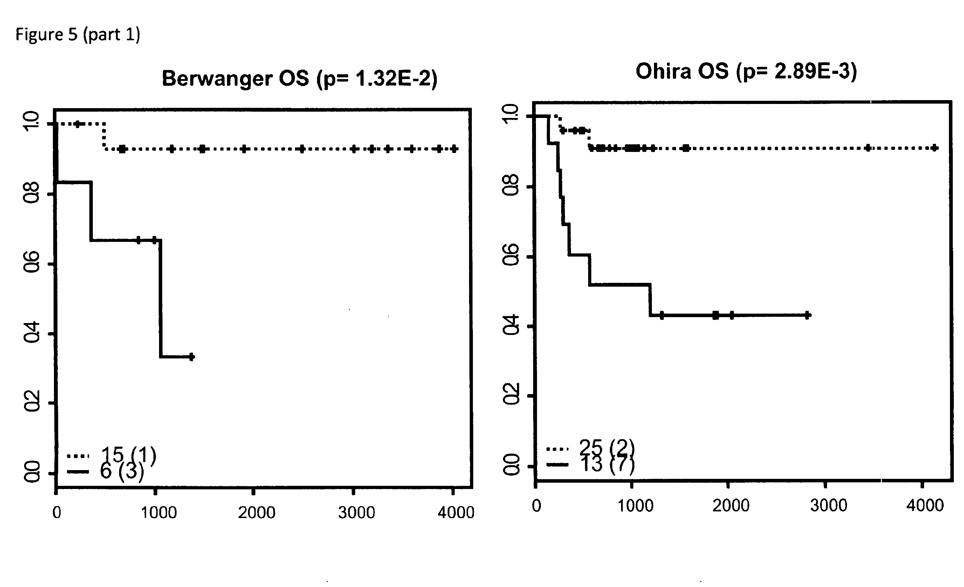

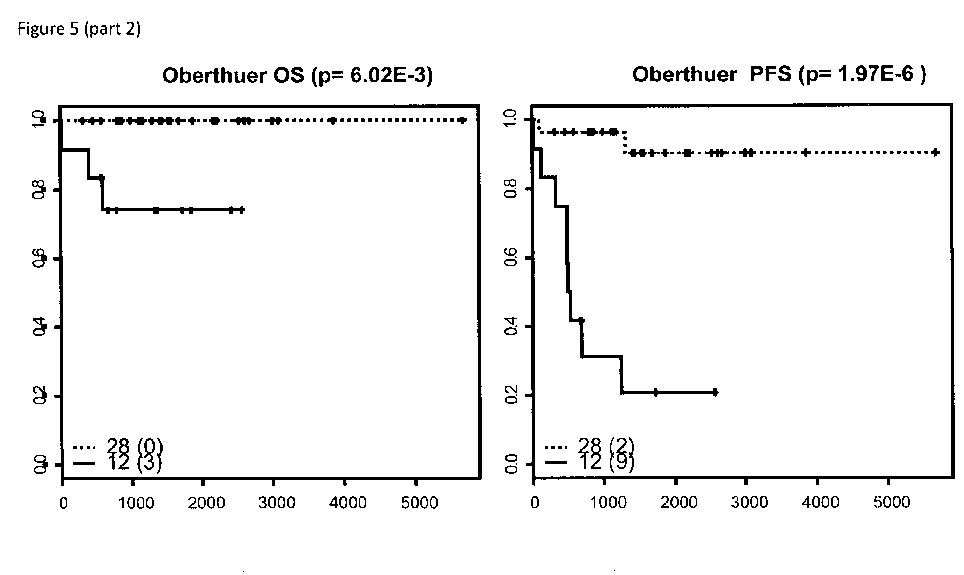

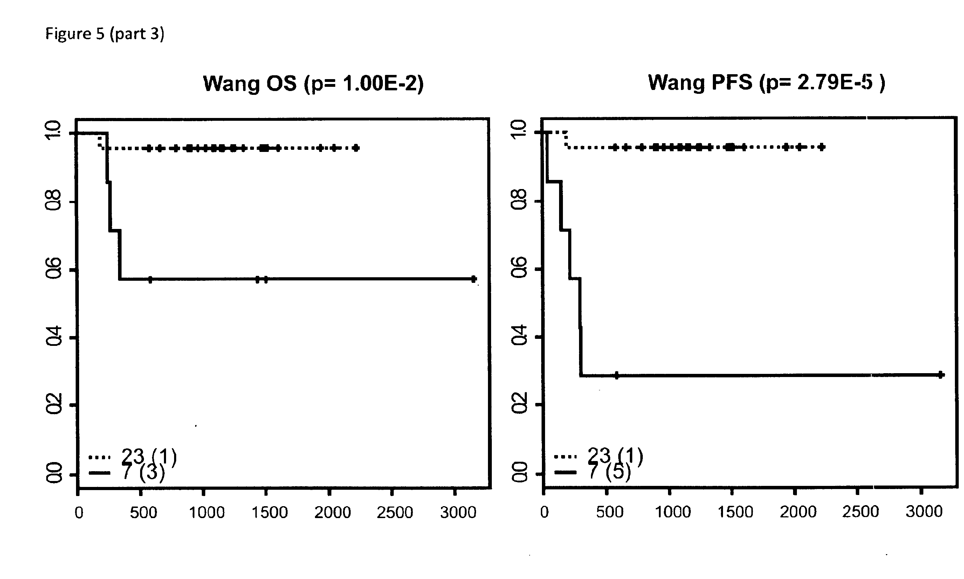

**Figure 5****:** Kaplan-Meier and log-rank analysis of the test patients from the four published phase 1 studies classified using the 42-gene PAM-classifier (legend: number of patients in predicted subgroups, between brackets: number of patients with event (relapse, progression or death))

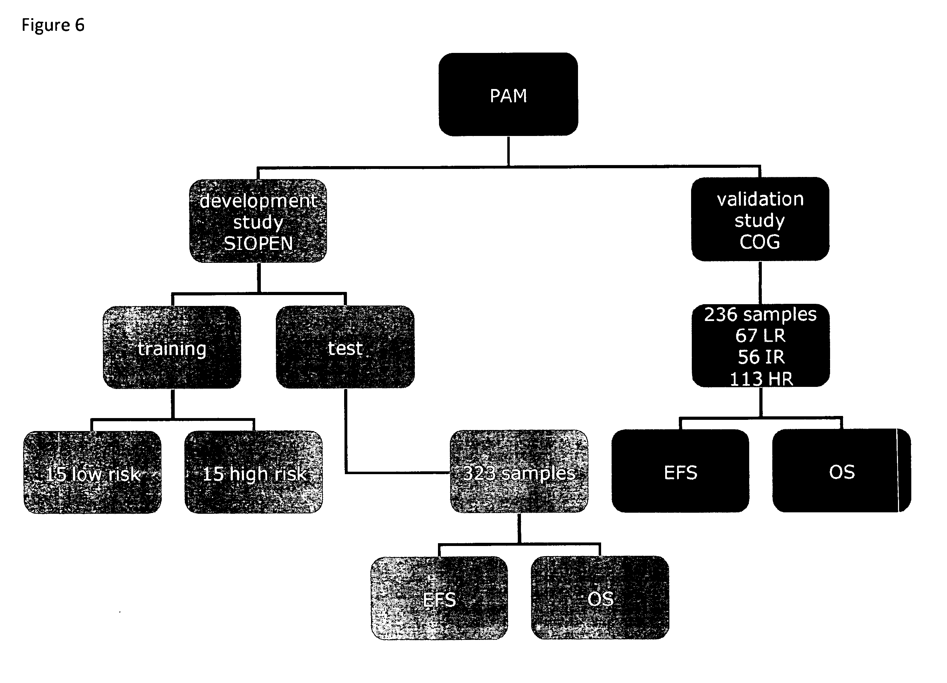

**Figure 6****:** Data-analysis of the 59-gene classifier. For the establishment of the multigene expression signature, SIOPEN (Society International Oncology Pediatric European Neuroblastoma) tumor samples were divided into a training set and a test set. The training set comprised 30 samples from 2 subgroups of patients with maximally divergent clinical courses selected ad random: 15 low risk patients with stage 1, 2 or 4S without MYCN amplification and with an progression free survival time (PFS) of at least 1000 days and 15 deceased high risk patients with age of diagnosis higher than 1 year with stage 4 tumor (irrespective of the MYCN status) or with stage 2 and 3 tumor with MYCN amplification. The multigene expression signature was built for these 30 training samples using the Prediction Analysis of Microarrays (PAM). This analysis resulted in a classifier of which the expression levels best characterized each risk group enabling class prediction of the remaining SIOPEN samples and validated in a blind manner on the COG (Children Oncology Group) samples.
   OS: Overall Survival, Low risk: stage 1, 2 or 4s, MYCN single copy, alive (PFS > 1000 d), High risk: stage 2/3, MYCN amplified, > 1 year, dead of disease or stage 4, > 1 year, dead of disease.
**Figure 7****:**
   Kaplan-Meier and log-rank analysis for progression-free (a) and overall (b) survival of the entire SIOPEN/GPOH cohort
   * missing relapse date for one HR case
   LR: low molecular risk
   HR: high molecular risk
   PFS: progression-free survival
   OS: overall survival
   (Legend: number of patients in predicted subgroups, between brackets: number of patients with event)
**Figure 8****:**
   Kaplan-Meier and log-rank analysis for progression-free (a) and overall (b) survival of the stratified SIOPEN cohort according to currently used risk factors
   * missing relapse date for one HR case
   LR: low molecular risk
   HR: high molecular risk
   PFS: progression-free survival
   OS: overall survival
   (Legend: number of patients in predicted subgroups, between brackets: number of patients with event)
   Missing MYCN status for 1 HR case
**Figure 9****:**
   Kaplan-Meier and log-rank analysis for progression-free (a) and overall (b) survival of the stratified SIOPEN cohort according to treatment group
   * missing relapse date for one HR case
   LR: low molecular risk
   HR: high molecular risk
   PFS: progression-free survival
   OS: overall survival
   (Legend: number of patients in predicted subgroups, between brackets: number of patients with event)
**Figure 10****:**
   Kaplan-Meier and log-rank analysis for overall (OS) and progression-free survival (EFS) of the total cohort of 208 test patients, and for the patients in the different risk groups (LR = low risk, HR = high risk, IR = intermediate risk (patients not belonging to LR or HR)) classified using the PAM 25 miRNA classifier.

### DETAILED DESCRIPTION OF THE PREFERRED EMBODIMENTS

In search for a new and innovative tool for prognosing the outcome of neuroblastoma patients in a more accurate manner, the inventors have embarked on an innovative route of re-analysing published results of micro-array gene expression studies in neuroblastoma. Strikingly, the lists that were published as comprising an important combination of prognostic genes in the past show very little overlap, indicating that their predictive power is not that high. Indeed, most studies show very good results on the tumor sample set used for their identification, but show little or poor prognostic power on unrelated tumor samples.

The inventors used these studies as a starting point to compose a more condensed list of genes, which were present in at least two of the newly generated prognostic gene lists. The finding of these genes in at least 2 independent studies indicates their power and robustness as prognostic markers. These genes, an original selection of 42 genes and a further selection of 59 genes, later reduced to only 12 and 6 genes were tested for their prognostic power on a large group of several hundreds of both published or unpublished (i.e. available at the lab or through collaborations) neuroblastoma tumor samples. This ratio of "less genes than tumor samples" resulted in very robust classifiers, which do not only work on a single set of tumors, but can be generally applied in the clinic.

The classifiers of the invention are thus valuable tools for predicting patient outcome (risk of relapse, progression or death).

The invention enables the prognosis of neuroblastoma patients by analysing the expression of the genes comprised in the 42 gene classifier according to the invention in a sample of the patient. This classifier was established purely based on in silico analysis.

In addition, the invention enables the prognosis of neuroblastoma patients by analysing the gene expression of the genes comprised in the 59, 42, 12 or 6 gene lists according to the invention in a sample of the patient (cf Table 1A). In this regard, especially the 12 and 6 gene lists are part of the invention. This gene list can be expanded with additional genes to obtain the 59 gene list of the invention. Although this gene list was also established by an in silico analysis, its prognostic power was tested by analysing a large set of several hundreds of tumor samples using the very accurate RT-qPCR analysis of tumor samples from patients, resulting in a classifier based on the expression levels of the different genes in the list.

### Establishing the 42 gene classifier according to the invention

In contrast to several published studies, the inventors performed high-throughput meta-analysis of 4 different micro-array studies (obtained on 4 different platforms: such as commercial and home-made oligonucleotide or cDNA arrays) to identify a list of top candidate prognostic markers for neuroblastoma (phase 1 datasets). This gene list of 42 genes (cf. Table 5) as well as subsets of the list were validated by re-analyzing 4 independent published studies (phase 2 datasets) and more importantly through analysis of more than 700 independent neuroblastoma tumors on our qPCR platform (2 independent series).

### Gene-list

Comparison of the performance of the 42-gene list with the lists that were generated showed that the classifier based on the 42-gene list has the highest overall accuracy. Upon comparison of our 42-gene signature with the published 144-gene classifier (Oberthuer A et al., J Clin Oncol 24:5070-8, 2006), the 42-gene signature clearly outperforms in all but one published study, i.e. the study for which the 144-gene classifier was built. For this dataset, both the 42- and 144-gene classifier have comparable performance. The high prognostic classification performance of the 42 gene list is undoubtedly due to the unique meta-analysis approach. First, annotations of the probes on the different platforms were updated according the latest genome build. Second, a uniform risk definition was applied to select training patients across the different studies. Only patients with maximally divergent courses were used for the training. Third, the same powerful algorithm with cross-validation was used for all studies, enabling the generation of relatively stable prognostic gene lists with high overlap.

### Classifier

This list of 42 prognostic genes was subsequently used to build a cross-platform classification signature. Since the PAM (prediction analysis of microarrays) algorithm is not suitable for cross-platform classification, a more intuitive, alternative method was used for building a 42-gene classifier. In this invention, a prognostic correlation signature based on the expression data of the 42 genes in all training samples of the four phase 1 datasets was generated. The signature was subsequently applied on independent test samples from the phase 1 datasets and on 4 independent phase 2 datasets, generated on different expression profiling platforms, for a total of 351 patients. The excellent prognostic performance of the 42-gene list in the test samples and the 4 independent datasets (Table 6) further demonstrates the validity of the meta-analysis approach and the utility of the recognized prognostic markers for NB. The classifier allows to predict overall (OS) and progression free survival (PFS) for the test patients that could not be unequivocally classified in the low or high risk training subgroups with a sensitivity of 85% (17 of 20 patients that died classified as prognostically unfavorable) for OS and 94% (16/17) for PFS, and a specificity of 61% (66/109) for OS and 77% (27/35) for PFS (for the specificity only patients with a follow-up time of at least 36 months were taken into account) (Table 6). Important to note is that for this particular group of patients, no definitive outcome is available, as there are censored patients with insufficient follow-up time. Performance data thus might improve over time. Importantly, it is shown that the classifier is, together with age at diagnosis, an independent predictor for PFS as well as OS.

Recently, Chen and colleagues presented a 160-gene classifier (Chen et al.: Genomics. 2008 Oct, 92(4):195-203) that could predict outcome in an independent dataset with an accuracy of 71%. Using our 42-gene classifier (i.e. comprising 4-times less genes), we obtained a similar accuracy of 70% (a difference of 1 sample misclassified) on the same set of samples. In addition, we applied our 42-gene classifier on the dataset that was used to build the 160-gene classifier and demonstrated an accuracy of 75%. In contrast to the study of Chen and colleagues, the inventors additionally demonstrated an excellent performance of the 42-gene classifier on a total of 5 independent datasets involving patients from different study protocols (German, European and COG) by using a smaller gene set and a more intuitive classification method. This indicates that the 42 gene set of the invention is independent from the sample set analysed and generally usable as a prognostic classifier.

### The 59 gene classifier according to the invention:

### Gene-list

Using a unique and powerful data-mining strategy, the inventors re-analysed 7 published micro-array gene-expression studies on neuroblastoma (NB) (cf. Oberthuer et al., J Clin Oncol. 2006 Nov 1;24(31):5070-8; Wang et al., Cancer research 2006, 66(12):6050-6062; Schramm et al., Oncogene 2005, 24(53):7902-7912; Berwanger et al., Cancer Cell 2002, 2(5):377-386; Ohira et al., Cancer Cell. 2005 Apr;7(4):337-50; De Preter et al., Genome Biology 2006, 7(9):R84; McArdle et al., Carcinogenesis 2004 25:1599-1609).

In a first step of the procedure, the inventors used patient subgroups with maximally divergent clinical courses, i.e. high-risk NB patients who deceased from disease and low risk patients with a long progression or relapse free survival time (>1000 days).

For each published micro-array study, prediction analysis of micro-arrays (PAM) with a 10 times repeated 10 fold cross-validation was performed in order to identify the genes that allow risk classification of patients. Inspection of the generated prognostic marker lists showed that there was a significant overlap between the different studies, in contrast to similar studies on other tumor entities. The main reasons for significant overlap are the use of an identical risk definition of patients, identical data-mining method, updated probe annotation, and updated clinical patient info where possible.

The inventors further compared these genes with a list of 48 genes with reported prognostic value upon extensive review of literature screening for single candidate prognostic genes.

In total, the inventors could establish a list of 59 prognostic markers (cf. Table 8) that were identified independently in at least 2 of the 7 prognostic gene sets or literature gene set.

### Classifier

Subsequently, a robust multigene expression prognostic signature was built using 30 training samples (i.e. 15 high risk and 15 low risk samples) and further tested on a large set of SIOPEN tumors from uniformly treated patients and validated on an independent set of COG tumors covering 579 patients in total.

What is unique in this invention is that a carefully selected gene set (59) was tested on a large panel of tumor samples (579) from uniformly treated patients, thus increasing statistical power and robustness through this high patient/gene ratio. Several previous studies have attempted to identify prognostic signatures in neuroblastoma based on genome-wide mRNA expression profiles. However, an important limitation of most published gene expression studies is the lack of statistical power due to extremely low patient/gene ratio. As such, there are inherent but often overlooked statistical issues, such as data over-fitting, unstable gene lists, and lack of study power. Consequently, for any small set of tumours, a gene classifier can be easily established, with little or no utility if not validated on an independent patient cohort.

As an essential step in the validation procedure of the developed multigene expression signature the inventors tested it on an independent set of COG tumors in a blind study. Similar performances of the gene expression based signature could be obtained indicating that the multigene expression signature can yield reproducible results in an independent patient cohort. Furthermore, the data obtained reflect the potential sources of variability in broad practice. Indeed, patients were of different geographic background and treated with other drugs, the RNA samples were extracted with other protocols and there were important differences in analytic methods.

Of further interest is the value of this multigene expression signature beyond the use of known risk factors as demonstrated by the survival analyses after stratification of the patients based on the current known risk factors and by the multivariate logistic regression analysis. Based on this signature, patients with an 19 fold higher risk for death of disease or 4-fold higher risk for relapse or progression can be identified which clearly exceeds the other risk factors. This clearly demonstrates the potential of this gene expression signature for improving management of NB patients.

Importantly, survival analyses within the groups of patients treated according to the current European treatment protocols clearly demonstrate that the multigene signature can make a difference between survivors and non-survivors. Consequently patients who have a poor gene signature and who are currently treated with surgery alone (LNEGS1) or with mild chemotherapy for children below 1 year of age (INES) might benefit from more appropriate therapies, i.e. according to the current HR-NBL1 protocol. On the other hand, patients who have a poor gene signature and who are currently already treated with intensive multi-modal anti-cancer therapies (HR-NBL1) might be excellent candidates for the addition of a more targeted agent in their treatment.

Gene Ontology analysis on the top ranking list of 59 prognostic genes tabulated in Table 1 In addition to known prognostic markers as MYCN and NTRK1, this gene set also contains positional candidate genes as CAMTA1 and CHD5 on 1p, BIRC5 (Survivin) on 17q, CADM1 (IGSF4) on 11q, etc which clearly demonstrates that the marker selection strategy to establish a top ranking list of prognostic genes identified those genes which are linked to underlying gene defects. Additional Gene Ontology analysis of the prognostic gene list showed that genes implicated in neuronal differentiation such as *PTN, NRCAM, DPYSL3, SCG2, DDC, FYN, NTRK1, MAPT, PMP22, CHD5,* and *MTSS1,* are enriched amongst the genes higher expressed in low-risk tumours.

Instead of microarrays which were used in most published gene-expression studies, the inventors used high-throughput RT-qPCR. The applied quantification strategy (including RNA quality control, RNA amplification and gene expression analysis by qPCR), is perfectly suitable for routine lab tests because it is a fast and sensitive PCR based assay, requiring only minimal amounts of RNA. RT-qPCR is the fastest, most accurate, precise and cost-efficient method to quantify mRNA expression of selected genes. Compared to microarrays, PCR technology is cheaper and the amount of required RNA as starting material is much lower. This is important, especially in paediatric cancers because most of the time biopsies are very small and the material available is poor. Moreover, a robust sample pre-amplification method (WT-Ovation from NuGEN) has extensively been validated for this application and allows preparing microgram quantities of amplified cDNA from 5-50 ng of total RNA enabling gene expression profiling of up to 1000 target genes (Vermeulen et al, BMC Res Notes. 2009 Nov 25;2(1):235). This significantly eased the increase of sample size in this collaborative study and the generated cDNA library might be a source for future qPCR gene expression studies. Another advantage of RT-qPCR versus microarrays is the possibility to use universally applicable, quantifiable and absolute standards (Vermeulen et al, Nucleic Acids Res. 2009 Sep 4). They consist of synthetic controls that need to be run in parallel with the patient samples for each gene to ensure reproducibility and validation of the result across labs and experiments. A standard was designed for all genes containing the forward primer sequence, a stuffer sequence (random sequence consisting of an actg repeat) in the middle and the reverse complement sequence of the reverse primer at the end (total length of 55 nucleotides). All standards were pooled together and a dilution series consisting of 5 serial dilution points, starting from 150,000 molecules down to 15 molecules was created using yeast tRNA as carrier. This innovative strategy not only allows careful monitoring and correction of inter-run variation, but also enables the exchange of data between different laboratories, even when not using the same PCR instrument or commercial master mixes. In that case average deltaCq of the absolute standards (15 in total) between the different instruments is calculated and Cq-values of all samples obtained with instrument 2 are corrected based on this value. This strategy truly enables to test the multigene expression signature on a single sample from any patient over the world accompanied by robust meta-analysis of the generated data in the future. Important advantages of this signature compared to previously published gene expression classifiers are thus the need of smaller amounts of starting material, the lower number of genes, higher cost-efficiency and speed of the quantification method, and the possibility of cross lab data comparison

In this study the inventors paid special attention to get all possible technical problems sorted out in order to make data more reliable (Bustin et al., 2008 Biomarkers Med. 2:201-207). For primer design the inventors used an *in silico* analysis pipeline, incorporating BLAST specificity, amplicon secondary structure, SNP presence and splice variant analysis and only primers with optimal efficiency were used for RT-qPCR (Lefever et al., Nucleic Acids Res. 2008, Oct 23.). To handle all the data and the calculations the inventors used powerful algorithms (Hellemans et al., Genome Biol. 2007;8(2):R19) and multiple internal control genes, being an indispensable element for proper data analysis (Vandesompele et al., Genome Biology 2002, 3(7)). However, the main technical critical issue of this approach is the RNA quality. The accuracy of gene-expression profiling is indeed highly dependent on mRNA quality (Nolan et al., Nat Protoc. 1(3), 1559-1582). In order not to compromise the outcome of this study, the inventors were very strict at RNA quality and purity and discarded almost 10% of the samples based on the presence of enzymatic inhibitors (SPUD-assay [Nolan et al 1]) or suboptimal RNA quality (low RNA Quality Index as established by the capillary gel electrophoresis analysis). As it unacceptable not being able to assess the prognosis of a child with neuroblastoma due to inferior RNA quality, the inventors further aim to evaluate the impact of RNA quality on classification performance and to establish a cut-off designating sufficient quality for proper class prediction.

### Reducing the 59 gene set.

As indicated above already, the inventors identified a 42 genes list tested for their prognostic power on published data-sets and here also tested using RT-qPCR on an independent set of 313 samples (the SIOPEN tumors), showing similar performances (cf. Table 1A, gene set "42") (after training with 15 low risk patients that survived and 15 high risk patients that died of disease).

In the complete 59 gene set, 12 genes were identified that had not previously been linked to neuroblastoma prognosis at all. The predictive power of the group of 12 genes was also tested (cf. Table 1A, gene set "12") and shown to have good prognostic power, but performs inferior to the best subsets (i.e. the 59 and 42 gene lists).

Six genes out of the 12 (unpublished) prognostic marker genes were also present in the 42 gene list. Also this gene list was tested for its performance (cf. Table 1A, gene list "6").

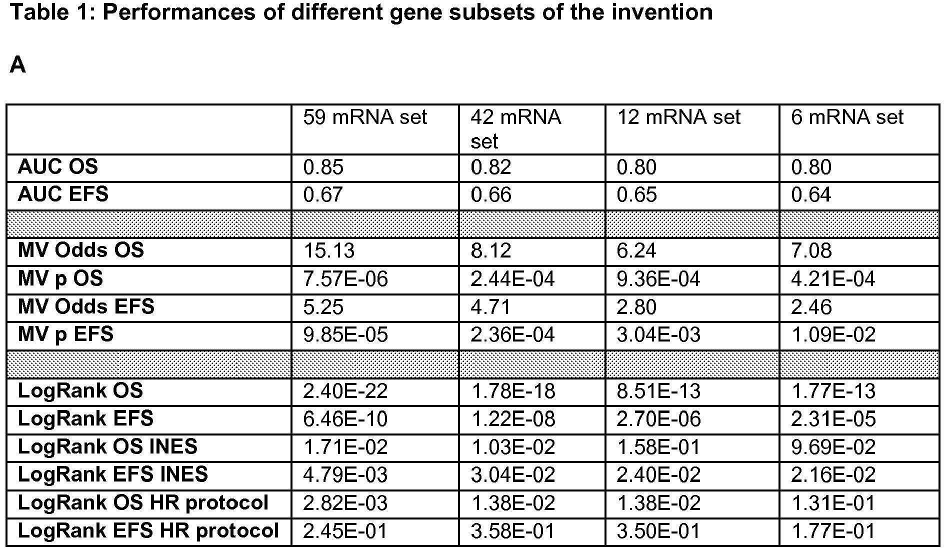

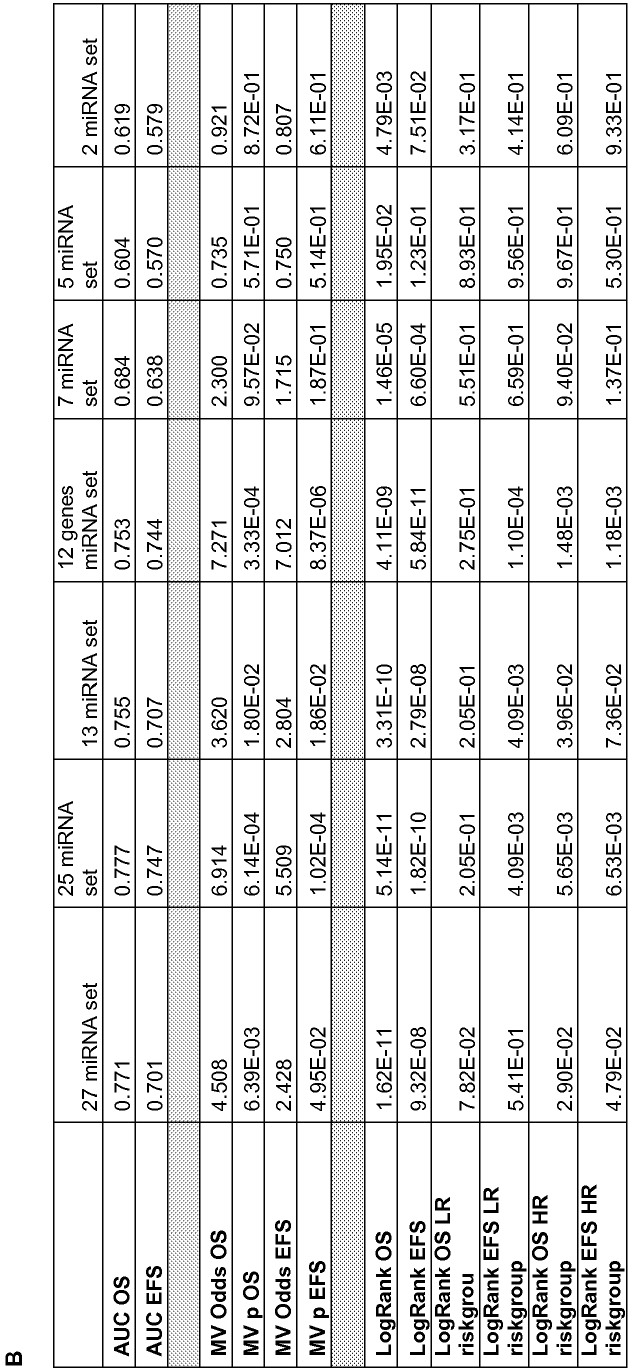

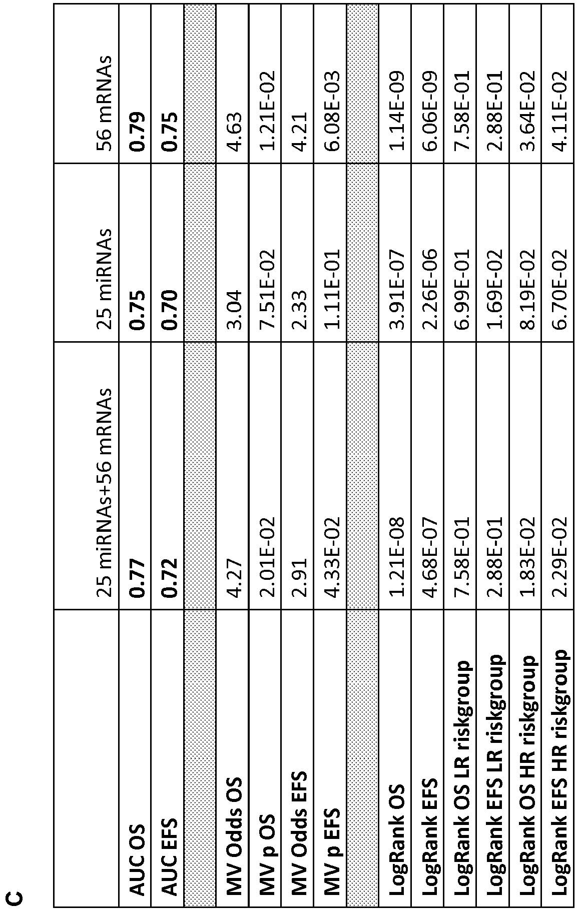

### miRNA classifiers for neuroblastoma

In addition to mRNA expression levels, non-coding RNAs (ncRNAs) such as micro RNAs (miRNAs) were recently recognized as important molecules in gene regulation and oncogenesis. Moreover, their importance in regulation of normal cellular growth and differentiation intuitively makes them good oncogene or tumor suppressor gene candidates in pediatric oncogenesis. The authors therefore established a neuroblastoma classifier based on expression profiling of miRNA molecules linked to prognosis. Moreover, miRNA expression levels are better suited to be used on formalin-fixed or paraffin-embedded samples.

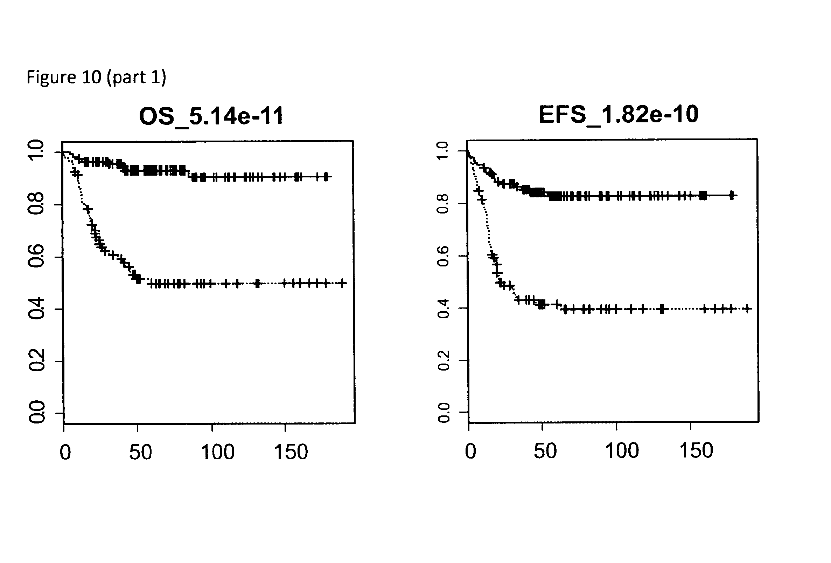

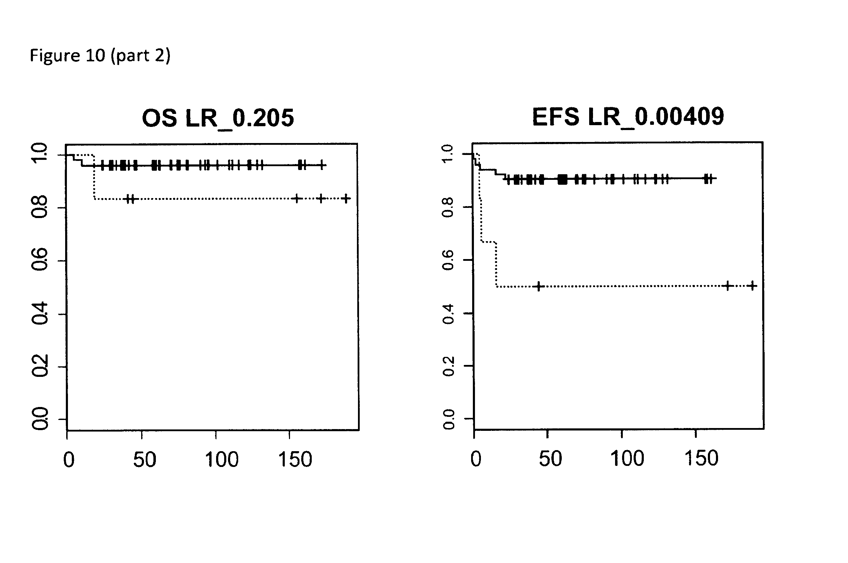

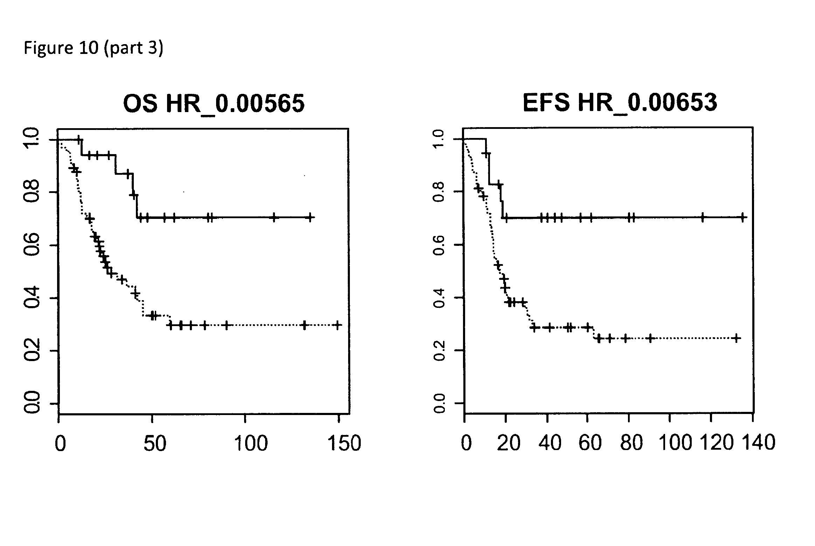

Using whole miRNAome profiling using the stem-loop RT-qPCR platform, we obtained the miRNA expression profile of 268 primary neuroblastoma tumour samples. Based on logistic regression analysis on 30 high risk (died of disease) and 30 low risk (survivors) training samples, the top 25 miRNAs with highest correlation to overall survival were selected. The performance of a 25 miRNA PAM classifier for the prognostic classification of the remaining 208 samples is summarised in Table 2B and Figur 10. In addition we tested the performance of a classifier based on a subselection of 12 genes, as well as the remaining 13 genes. 7 miRNAs of the 25 miRNA set were identified as not being previously linked to neuroblastoma prognosis at all. The predictive power of this list as well as the 5 miRNAs of the 7 miRNA list that overlap with the 12 gene list and the 2 miRNAs of the 7 miRNA list that overlap with the 13 gene list is summarised in the Table. Overall these analyses show that the 25 miRNA list performs best and that all other list, except from the 2 gene list perform relatively good.

These classifiers provide easy tools for clinical assessment of neuroblastoma prognosis.

They can be used in isolation or in combination with each other and/or in combination with the mRNA classifiers. In such a combination, one can for example use the 6, 12, 42 or 59 gene set in combination with any one of miRNA sets of the present invention..

For 178 samples, mRNA expression profiling data (on the Affymetrix platforms) as well as miRNA expression profiling data (stem-loop RT-qPCR platform) were available. mRNA expression data of 56 of the claimed 59 mRNA list and all of the 25 miRNA list were present in the profiles. In Table 1C performance results are shown for a PAM classifier combining the expression data of the 59 mRNA list and the 25 miRNA list, showing that comparable results could be obtained in comparison to the individual classifiers. For some parameters (for example log-rank analysis within the high risk subgroup) the performance of the combined classifier is better than the individual parameters.

### Validation of the classifiers through RT-qPCR

Using real-time PCR, the prognostic gene lists and derived classifiers of the invention were tested on a large set of 345 European tumors and validated on an independent set of 257 USA tumors. The applied methods and classifiers are suitable for routine lab testing and ready to be evaluated in prospective studies. The multigene signatures are independent risk predictors after controlling for other known risk factors, identifying patients with increased risk in the current clinical risk groups. It should be clear of course that other means of detecting the expression levels of said RNAs known in the art can be used.

### Extraction of RNA from the biological sample of a patient

Extraction of the nucleic acid biological material as defined herein from the biological sample can be done using any the nucleic acid extraction and purification protocols well known to those skilled in the art. A non-limiting example of such a method typically comprises the following general method steps:
Lysis of the cells present in the biological sample, in order to release the nucleic acids contained in the cells of the patient using any known method in the art is always the first step. By way of non-limiting example, lysis methods as described in patent applications: WO00/05338 regarding mixed magnetic and mechanical lysis, WO99/53304 regarding electrical lysis, WO99/15321 regarding mechanical lysis, thermal or osmotic shocks or chemical lysis with chaotropic agents such as guanidium salts (US 5,234,809) can be used.

The second step is purification allowing separation of the nucleic acids and the other cellular constituents released in the lysis step. This step generally makes it possible to concentrate the nucleic acids, and can be adapted to the purification of both DNA or RNA. By way of non-limiting example, it is possible to use magnetic particles optionally coated with oligonucleotides, by adsorption or covalence (cf. US 4,672,040 and US 5,750,338), and thus to purify the nucleic acids which are bound to these magnetic particles, by means of a washing step. This nucleic acid purification step is particularly advantageous if it is desired to subsequently amplify said nucleic acids by means of e.g. PCR. A particularly advantageous embodiment of these magnetic particles is described in patent applications: WO97/45202 and WO99/35500. Another advantageous example of a method of purifying nucleic acids is the use of silica either in the form of a column, or in the form of inert particles (Boom R. et al., J. Clin. Microbiol., 1990;28(3):495-503) or magnetic particles (Merck: MagPrep® Silica, Promega: MagneSil™ Paramagnetic particles,...). Further methods are based on ion exchange resins in a column or in a paramagnetic particulate format (Whatman: DEAE-Magarose; Levison et al., J. Chromatography, 1998, p. 337-344). Another method for the invention is that of adsorption onto a metal oxide support (e.g. from Xtrana: Xtra-Bind™ matrix).

Additionally, when the DNA is to be extracted from a biological sample exclusively, it is in particular possible to carry out an extraction with phenol, chloroform and alcohol in order to remove the proteins, and to precipitate the DNA with 100% ethanol. The DNA can then be pelleted by centrifugation, washed, and re-dissolved.

Alternatively, when it is desired to specifically extract RNAs from a biological sample, it is in particular possible to carry out an extraction with phenol, chloroform and alcohol in order to remove the proteins, and to precipitate the RNAs with 100% ethanol. The RNAs can then be pelleted by centrifugation, washed, and re-dissolved.

### PCR-based detection of gene expression

According to one specific embodiment of the invention, the means for determining expression analysis of mRNAs or miRNAs used in the methods, kits and tools of the invention, comprises at least one amplification primer. For the purpose of the present invention, the term amplification primer is intended to mean a nucleotide fragment comprising from 5 to 100 nucleic units, preferably from 15 to 30 nucleic units, allowing the initiation of an enzymatic polymerization, such as in particular an enzymatic amplification reaction.

According to one specific embodiment of the invention, the amplification primer comprises a sequence chosen from SEQ ID Nos. 1 - 128 (cf. Table 2). The term enzymatic amplification reaction is intended to mean a process generating multiple copies of a nucleotide fragment by the action of at least one enzyme.

In most amplification reactions however a pair of two primers is used to amplify a specific region of a target RNA or DNA lying in-between the two primers of the pair.

For standardisation and reduction of cross-platform differences and to allow data exchange between different labs using different qPCR instruments, standard oligonucleotides such as the ones described in Table 3 can be included in the PCR reaction.

Such amplification reactions are well known to those skilled in the art and mention may in particular be made of the following techniques:
- PCR (Polymerase Chain Reaction), as described e.g. in patents US 4,683,195, US 4,683,202 and US 4,800,159, including reverse transcript PCR (RT-PCR), Real-Time quantitative PCR (RT-qPCR), and the like,
- LCR(Ligase Chain Reaction), disclosed, for example, in patent application EP 0201 184,
- RCR (Repair Chain Reaction), described in patent application WO90/01069,
- 3SR (Self Sustained Sequence Replication) with patent application WO90/06995, NASBA (Nucleic Acid Sequence-Based Amplification) with patent application WO91/02818, and
- TMA (Transcription Mediated Amplification) as described in e.g. patent US 5,399,491.

In a preferred embodiment, **RT-qPCR** is used to quantify the expression level of the target genes. The term "reverse transcription quantitative polymerase chain reaction" or "RT-qPCR", also called quantitative real time polymerase chain reaction (Q-PCR/qPCR) or kinetic polymerase chain reaction, is a laboratory technique based on the polymerase chain reaction, which is used to amplify and simultaneously quantify a targeted DNA molecule. It enables both detection and quantification (as absolute number of copies or relative amount when normalized to DNA input or additional normalizing genes) of a specific sequence in a DNA sample.

The procedure follows the general principle of polymerase chain reaction. Its key feature is that the amplified DNA is quantified as it accumulates in the reaction in *real time* after each amplification cycle. Two common methods of quantification are the use of fluorescent dyes that intercalate with double-stranded DNA, and modified DNA oligonucleotide probes that fluoresce when hybridized with a complementary DNA.

Frequently, real-time polymerase chain reaction is combined with reverse transcription polymerase chain reaction to quantify low abundance messenger RNA (mRNA), enabling a researcher to quantify relative gene expression at a particular time, or in a particular cell or tissue type.

Using PCR-technology as described, combinations of gene expression profiles can be analysed. Combined gene expression analysis of mRNA, miRNA and/or T-UCR expression analysis can also be carried out in a single PCR-based experiment, further improving the prognostic power of the tools and methods of the invention.

**End-Point PCR** technology can of course also be used in the methods of the invention. The difference between End-Point and Real-Time PCR lies in the moment of detection of amplified PCR product, i.e. during the reaction in case of Real-Time PCR and after the reaction has been stopped in case of End-Point-PCR. Amplification of DNA is an exponential procedure in the early and middle cycles of a PCR, a property that is exploited to infer the starting amount of PCR template. During this exponential or log phase each copy of DNA is being amplified. Detecting this amplification in Real-Time clearly is a better measure than in End-Point PCR, where reagents such a nucleotides may become exhausted and result in inefficient amplification, resulting in inaccurate quantification of the gene of interest (cf. Schmittgen TD et al., Anal Biochem. 2000 Oct 15;285(2):194-204).

In addition to RT-qPCR or End-Point PCR and others, **digital PCR** can be used (dPCR or dePCR), which is a refinement of conventional polymerase chain reaction methods that can be used to directly quantify and clonally amplify nucleic acids including DNA, cDNA or RNA. Digital PCR (dPCR) amplifies nucleic acids by temperature cycling of a nucleic acid molecule with the enzyme DNA polymerase. In digital emulsion PCR (dePCR), the reaction is carried out in the dispersed phase of an emulsion. Theoretically, PCR exponentially amplifies nucleic acids, and the number of amplification cycles and the amount of PCR end-product should allow the computation of starting quantity. However, many factors complicate this calculation, creating uncertainties and inaccuracies. These factors include: initial amplification cycles may not be exponential; PCR amplification eventually plateaus after an uncertain number of cycles; low initial concentrations of target nucleic acid molecules may not amplify to detectable levels; and PCR amplification efficiency in a sample of interest may be different from that of reference samples. Digital PCR overcomes these difficulties by transforming unreliable exponential data from conventional PCR to digital signals that simply indicate whether or not amplification has occurred. Digital PCR is achieved by capturing or isolating each individual nucleic acid molecule present in a sample within many separate chambers, zones or regions that are able to localize and concentrate the amplification product to detectable levels. After PCR amplification, a count of chambers, zones or regions containing PCR end-product is a direct measure of the absolute nucleic acids quantity. The capture or isolation of individual nucleic acid molecules may be effected in capillaries, microemulsions, arrays of miniaturized chambers, or on nucleic acid binding surfaces (Sykes, P.J. et al., Biotechniques 13 (3): 444-9 and Vogelstein B, Kinzler KW, Proc Natl Acad Sci USA. 1999 Aug 3;96(16):9236-41).

In addition, **High-Throughput Quantitative PCR** can be used according to e.g. Kiss MM et al. (Anal Chem. 2008 Oct 29)

Alternatively, **On-chip, real-time, single-copy PCR** can be used as described by e.g. Beer NR et al. (Anal Chem. 2007 Nov 15;79(22):8471-5).

When the enzymatic amplification is a PCR, the specific reagent comprises at least 2 amplification primers specific for a target gene (e.g. the primes pairs as listed in Table 2), and which allow the amplification of the material specific for the target gene. The material specific for the target gene then preferably comprises a complementary DNA obtained by reverse transcription of messenger RNA derived from the target gene (then described as cDNA specific for the target gene) or a complementary RNA obtained by transcription of the cDNA specific for a target gene (then described as cRNA specific for the target gene). When the enzymatic amplification is a PCR carried out after a reverse transcription reaction, it is referred to as RT-PCR.

When amplification reactions as indicated above are used it is possible to determine the expression of a target gene in the following way:

On the extracted biological material (i.e. the total RNA comprising the transfer RNA (tRNA), the ribosomal RNA (rRNA) and the messenger RNA (mRNA)) from a biological sample as presented above, a reverse transcription step is carried out in order to obtain the complementary DNAs (or cDNA) of said mRNAs using standard techniques.

By way of indication, this reverse transcription reaction can be carried out using a reverse transcriptase enzyme which makes it possible to obtain, from an RNA fragment, a complementary DNA fragment. cDNA complementary to the mRNAs derived from a target gene (cDNA specific for the target gene) and cDNA complementary to the mRNAs derived from genes other than the target gene (cDNA not specific to the target gene) are then obtained.

The amplification primer(s) specific for each of the target gene is (are) brought into contact with the cDNA. The amplification primer(s) specific for a target gene hybridize(s) with the cDNA specific for the target gene and a predetermined region, of known length, of the cDNA originating from the mRNAs derived from the target gene is specifically amplified. The cDNA not specific for the target genes are not amplified. For the purpose of the present invention, reference is made, without distinction, to "cDNA specific for the target gene" or to "cDNA originating from the mRNA derived from the target gene". This step can be carried out in particular by means of a PCR-type amplification reaction or by any other amplification technique as defined above. By PCR, it is also possible to simultaneously amplify several different cDNA, each one being specific for a different target gene, by using several pairs of different amplification primers, each one being specific for a target gene: reference is then made to multiplex amplification.

The expression of the target gene is then determined by detecting and quantifying the cDNA specific for the target gene that is obtained according to the method above. This detection can be carried out after electrophoretic migration of the cDNA specific for the target gene according to their size. The gel and the medium for migration can include ethydium bromide in order to allow direct detection of the cDNA specific for the target gene when the gel is placed, after a given migration period, on a UV (ultraviolet)-ray light table, through the emission of a light signal. The greater the amount of cDNA specific for the target gene, the brighter this light signal. These electrophoresis techniques are well known to those skilled in the art. Other fluorescent DNA-incorporating dyes may of course be used. The cDNA specific for the target gene can also be detected and quantified using a quantification range obtained by means of an amplification reaction carried out until saturation. In order to take into account the variability in enzymatic effectiveness which may be observed during the various steps (reverse transcription, PCR, etc.), the expression of a target gene of several groups of patients can be normalized by simultaneously determining the expression of a "housekeeping" gene, the expression of which is similar in the various groups of patients. By realizing a ratio of the expression of the target gene to the expression of the housekeeping gene, i.e. by realizing a ratio of the amount of cDNA specific for the target gene to the amount of cDNA specific for the housekeeping gene, any variability between the various experiments is thus corrected. Those skilled in the art may refer in particular to the following publications: Bustin, Journal of molecular endocrinology, 2002, 29: 23-39; Giulietti, Methods, 2001, 25: 386-401.

**Table 2: primers**

| **symbol** | **primer** | **efficiency %** | **Sense Primer** | **SEQ ID NO** | **Anti-sense Primer** | **SEQ ID NO** |
|---|---|---|---|---|---|---|
| NHLH2 | 4478 | 96 | TCAGCAACACTAGCACTTCAC | 1 | AAGGACTTCTCAGACATAACTACAG | 2 |
| MRPL3 | 4479 | 90,7 | GAACTGCCAGAAGATTTGTATGATG | 3 | GTCCAAAGATGTTAGGCAAATGTAA | 4 |
| CDCA5 | 4480 | 95,6 | TTGTCACCCATACCCATTTCTTAC | 5 | CTGGGACTCTTCAACTTTCTCTTC | 6 |
| ARHGEF7 | 4481 | 96,3 | GGAGCACCTACAGAAGCAAAC | 7 | GGTATGAGATGGCACTGAATGAG | 8 |
| ECEL1 | 4862-4863 | 98,4 | CTGCAGGTGCTGACTGACAAG | 9 | GAAAGCCCGGCCAAACTC | 10 |
| PTPRF | 4483 | 95 | TCGGAGCCTGTAACCTACTATG | 11 | CACACCATCCACCTCCTGAA | 12 |
| PLAGL1 | 4484 | 95,1 | GGCTAAGGGAAATGCTGGTAAAG | 13 | GAGGCAGGTATTGTTAGGTTCAC | 14 |
| ODC1 | 4860-4861 | 95,5 | AAGAGATCACCGGCGTAATCAA | 15 | CGGGCTCAGCTATGATTCTCA | 16 |
| DPYSL3 | 4486 | 93,7 | CAGCCAGCATTCATTGTAAGTTC | 17 | CCATACCCACCAGACACAGAA | 18 |
| PMP22 | 4487 | 93 | TACTCCTACGGTTTCGCCTAC | 19 | ACATAGATGACACCGCTGAGAA | 20 |
| CLSTN1 | 4488 | 95,8 | GATGGACTGGGACGACTCTG | 21 | CTGCTGTGCTGGTCCTCATA | 22 |
| MTSS1 | 4490 | 91 | TAGTGTTTAAGAAAGCAAGCAAGTC | 23 | GAGGGTTCGGTCAGAAATGTG | 24 |
| CAMTA2 | 4491 | 94,9 | ATGATGAGTGGCTGTCTTGTG | 25 | TGCGATTGTAGAGGATGATGGA | 26 |
| PTN | 4492 | 103,7 | ACAATGCCGAATGCCAGAAG | 27 | AGGTTTGGGCTTGGTCAGTT | 28 |
| SNAPC1 | 4493 | 91,1 | CATACAGGCATCCAAATCAAGAAG | 29 | GCAGAATCAGAGTCAGAAGAGTC | 30 |
| QPCT | 4494 | 90,6 | GGAACTTGCTCGTGCCTTAG | 31 | TCTGGCTTGGAGTCTGAAACA | 32 |
| EPB41L3 | 4495 | 96,9 | ACCACCACTACGCACATCAC | 33 | TCGCTTCTCAATTCTTGTCTCTG | 34 |
| GNB1 | 4496 | 96,9 | TGACCCTGTTTTGTGGCATTC | 35 | GGACACGATGACCAGATGAAC | 36 |
| PTPRH | 4497 | 96,6 | GCTTCGGCTTCAGAGAACAAC | 37 | CCAGTCATAGGGCAGCACAT | 38 |
| MAPT | 4498 | 97,6 | TTTGGTGGTGGTTAGAGATATGC | 39 | CCGAGGTGCGTGAAGAAATG | 40 |
| SLC25A5 | 4499 | 96,2 | CGCCTACTTCGGTATCTATGAC | 41 | CTGATGACGATGTGAGTGTTCTT | 42 |
| BIRC5 | 4500 | 93,7 | AAACTAAGCACAAAGCCATTCTAAG | 43 | CACTCTATTCTGTCTCCTCATCCA | 44 |
| AHCY | 4501 | 97,7 | ACAGGTCCAGTGGTTCTTCAG | 45 | ACAGTTCCTCTTTGCCCTTCA | 46 |
| ULK2 | 4503 | 101,8 | GGCTCTCCTACTAAGACCACAG | 47 | GACGAGTAACCAAGGCTAACAG | 48 |
| PTPRN2 | 4504 | 95,8 | AACAAAGACAAACTGGAGGAAAC | 49 | TGAGGAGGCAGGAACTTGAG | 50 |
| INPP1 | 4505 | 95,8 | GCAAAGTCCTCAATGGTAACAAG | 51 | TGGGTCAGTAAAGGCAACATC | 52 |
| HIVEP2 | 4506 | 95,3 | GCCAACTTCTTCAGCAACTAATC | 53 | CATCGTCGGAACCAGTCATC | 54 |
| EPN2 | 4507 | 95,9 | TCAGAGGCAGAAATCAAAGTCC | 55 | TGTAGGTCAGGTCGGCAATC | 56 |
| EPHA5 | 4508 | 90,9 | ATTTATTGGATTCACGCACTGTC | 57 | TCATCCACTTCACCAATCTCTTC | 58 |
| CHD5 | 4786-4787 | 97,6 | CGACTTCTACGTGGTCACCTACAC | 59 | CACTCCGAATGGCGTTGTC | 60 |
| PDE4DIP | 4510 | 92,3 | GCAGATACCTTCCAGAGATGATAG | 61 | CTGTGTCCAAGTCTCCTAATGTG | 62 |
| TYMS | 4511 | 84,2 | TGTGCCAGTTCTTTCCATAATAAA | 63 | ATTTCATTCTCCTCACTTTGTTCAT | 64 |
| MAP2K4 | 4512 | 91 | GCTGCCAACTTGATGTTCCA | 65 | CATCTGTAAACTTTGCCTTCTGTA | 66 |
| CPSG3 | 4513 | 96,7 | CCAGCATCACCACCACAAAT | 67 | GCAGAAATTCCCTTCGTCCTT | 68 |
| MCM2 | 4514 | 87,8 | TTGGCGTGAGTTGCGTATTC | 69 | GAGACTGAAAACGATTACAAACATC | 70 |
| PRDM2 | 4516 | 91,4 | TTCTTCTTCATCTTCCTCCTCTTC | 71 | AGCCTCCAGATTATCACCAGA | 72 |
| SLC6A8 | 4517 | 95,4 | GCCTCCTACTACTTCCGTTTC | 73 | TACATCCCGCCATCAGTCAC | 74 |
| NME1 | 4518 | 93,9 | GAGGAACTGGTAGATTACACGAG | 75 | GTCTGCCCTCCTGTCATTCA | 76 |
| TNFRSF25 | 4519 | 97 | CAAGGCGAAGAAGCACGAAC | 77 | GCCGAGAAGTTGAGAAATGTCT | 78 |
| PRKCZ | 4520 | 96,2 | AGACGATGAGGATGCCATAAAG | 79 | CCTCGGTGGACAGCAATAATG | 80 |
| CAMTA1 | 4521 | 89,2 | AAATGACAGATGGTAGAGACTTCC | 81 | ACTGGTAGGTTACACTGGTAGG | 82 |
| AKR1C1 | 4522 | 89,7 | GGATTATGTTGACCTCTACCTTATT | 83 | TTTTTCCATTTTCATCTTTTGGGAT | 84 |
| ELAVL4 | 919-920 | 92,6 | GCTACGGAACCGATTACTGTGAA | 85 | GACTGGTAGAGCTGGGAGAGCA | 86 |
| NTRK1 | 4545-4546 | 90,5 | CGAGAGCATCCTGTACCGTAAGT | 87 | TGCTTGCCGTAGGTGAAGATC | 88 |
| PRAME | 3197-3198 | 93,5 | CGTAGACTCCTCCTCTCCCACAT | 89 | TGGGCGATATACTGCTCTTCCT | 90 |
| WSB1 | 3193-3194 | 91,5 | CCAGAAAAACAGAGTCGCTGTGT | 91 | ACCCTGTAGCAAGAAGTAGCTGATC | 92 |
| DDC | 4663-4664 | 94 | CGCAAGTGAATTCCGAAGGA | 93 | ACCTGGCGTCCCTCAATG | 94 |
| MYCN | 4795-4796 | 94,3 | GCGAGCTGATCCTCAAACG | 95 | CGCCTCGCTCTTTATCTTCTTC | 96 |
| PLAT | 2978-2979 | 87,8 | CCGGCTACGGCAAGCA | 97 | TGGATGGGTACAGTCTGACATGA | 98 |
| CD44 | 651-652 | 92,3 | TGCCGCTTTGCAGGTGTAT | 99 | GGCCTCCGTCCGAGAGA | 100 |
| CDKN3 | 4533-4534 | 87,1 | TCCAGTAGCTGCTTGTCTCCTACTATA | 101 | TCTTAGGTCTCGCAGGCTGTCT | 102 |
| FYN | 4525-4526 | 92,6 | CCTTTCTTATCCGCGAGAGTGA | 103 | GGTCTCCTTTCATATCATCCCAAT | 104 |
| PAICS | 4527-4528 | 88,8 | TGTCACCCAGGTTCGTCTCA | 105 | CATGTCTGTAACCCTAGCACTTTGG | 106 |
| SCG2 | 4529-4530 | 91,5 | ACTTTCCAATGGACATGAGTGATG | 107 | TAGGAGGGAATTGCATGTGCTT | 108 |
| MAP7 | 4502 | 98,8 | ATCCCAAAGCAAGACAACCAG | 109 | CCAGGCAAATGAGGAAGAGAC | 110 |
| CADM1 | 5386-5387 | 94,9 | CGCGCTTGAGTTAACATGTGAA | 111 | TCGACTCTCACCCAAGTTACCA | 112 |
| PRKACB | 5392-5393 | 90,9 | GGGCATTAGGAGTGCTAATCTATGA | 113 | ATCTGAATTGGTTGGTCTGCAA | 114 |
| NRCAM | 5394-5395 | 98,7 | GAGAACACAGGGCAAGACACATAC | 115 | GGACCAGCTGATACAGAATGCA | 116 |
| PIK3R1 | 5398-5399 | 94,8 | GGGAAGCGAGATGGCACTT | 117 | CACCACTACAGAGCAGGCATAGC | 118 |
| UBC | 431/432 | 93,7 | ATTTGGGTCGCGGTTCTTG | 119 | TGCCTTGACATTCTCGATGGT | 120 |
| HPRT1 | 523/524 | 95,3 | TGACACTGGCAAAACAATGCA | 121 | GGTCCTTTTCACCAGCAAGCT | 122 |
| HMBS | 410/411 | 97,6 | GGCAATGCGGCTGCAA | 123 | GGGTACCCACGCGAATCAC | 124 |
| SDHA | 687-688 | 98,1 | TGGGAACAAGAGGGCATCTG | 125 | CCACCACTGCATCAAATTCATG | 126 |
| ALUsq | 2159/216 0 | NA | CATGGTGAAACCCCGTCTCTA | 127 | GCCTCAGCCTCCCGAGTAG | 128 |

**Table 3: Oligonucleotides**

| **symbol** | **oligonr** | **SEQ ID NO** | **oligo** |
|---|---|---|---|
| NHLH2 | 4981 | 129 | TCAGCAACACTAGCACTTCACactgactgaCTGTAGTTATGTCTGAGAAGTCCTT |
| MRPL3 | 4982 | 130 | GAACTGCCAGAAGATTTGTATGATGactgaTTACATTTGCCTAACATCTTTGGAC |
| CDCA5 | 4983 | 131 | TTGTCACCCATACCCATTTCTTACactgactGAAGAGAAAGTTGAAGAGTCCCAG |
| ARHGEF7 | 4984 | 132 | GGAGCACCTACAGAAGCAAACtctgactgactCTCATTCAGTGCCATCTCATACC |
| ECEL1 | 4985 | 133 | CTGCAGGTGCTGACTGACAAGactgactgactgactgGAGTTTGGCCGGGCTTTC |
| PTPRF | 4986 | 134 | TCGGAGCCTGTAACCTACTATGactgactgactgaTTCAGGAGGTGGATGGTGTG |
| PLAGL1 | 4987 | 135 | GGCTAAGGGAAATGCTGGTAAAGactgactgaGTGAACCTAACAATACCTGCCTC |
| ODC1 | 4988 | 136 | AAGAGATCACCGGCGTAATCAAactgactgactgTGAGAATCATAGCTGAGCCCG |
| DPYSL3 | 4989 | 137 | CAGCCAGCATTCATTGTAAGTTCactgactgactTTCTGTGTCTGGTGGGTATGG |
| PMP22 | 4990 | 138 | TACTCCTACGGTTTCGCCTACactgactgactgTTCTCAGCGGTGTCATCTATGT |
| CLSTN1 | 4991 | 139 | GATGGACTGGGACGACTCTGactgactgactgactTATGAGGACCAGCACAGCAG |
| MTSS1 | 4993 | 140 | TAGTGTTTAAGAAAGCAAGCAAGTCactgactgaCACATTTCTGACCGAACCCTC |
| CAMTA2 | 4994 | 141 | ATGATGAGTGGCTGTCTTGTGactgactgactgTCCATCATCCTCTACAATCGCA |
| PTN | 4995 | 142 | ACAATGCCGAATGCCAGAAGactgactgactgactAACTGACCAAGCCCAAACCT |
| SNAPC1 | 4996 | 143 | CATACAGGCATCCAAATCAAGAAGactgactgGACTCTTCTGACTCTGATTCTGC |
| QPCT | 4997 | 144 | GGAACTTGCTCGTGCCTTAGactgactgactgacTGTTTCAGACTCCAAGCCAGA |
| EPB41L3 | 4998 | 145 | ACCACCACTACGCACATCACactgactgactgCAGAGACAAGAATTGAGAAGCGA |
| GNB1 | 4999 | 146 | TGACCCTGTTTTGTGGCATTCactgactgactgaGTTCATCTGGTCATCGTGTCC |
| PTPRH | 5000 | 147 | GCTTCGGCTTCAGAGAACAACactgactgactgacATGTGCTGCCCTATGACTGG |
| MAPT | 5001 | 148 | TTTGGTGGTGGTTAGAGATATGCactgactgactgCATTTCTTCACGCACCTCGG |
| SLC25A5 | 5002 | 149 | CGCCTACTTCGGTATCTATGACactgactgacAAGAACACTCACATCGTCATCAG |
| BIRC5 | 5003 | 150 | AAACTAAGCACAAAGCCATTCTAAGactgacTGGATGAGGAGACAGAATAGAGTG |
| AHCY | 5004 | 151 | ACAGGTCCAGTGGTTCTTCAGactgactgactgaTGAAGGGCAAAGAGGAACTGT |
| ULK2 | 5006 | 152 | GGCTCTCCTACTAAGACCACAGactgactgactCTGTTAGCCTTGGTTACTCGTC |
| PTPRN2 | 5007 | 153 | AACAAAGACAAACTGGAGGAAACactgactgactgCTCAAGTTCCTGCCTCCTCA |
| INPP1 | 5008 | 154 | GCAAAGTCCTCAATGGTAACAAGactgactgactGATGTTGCCTTTACTGACCCA |
| HIVEP2 | 5009 | 155 | GCCAACTTCTTCAGCAACTAATCactgactgactgGATGACTGGTTCCGACGATG |
| EPN2 | 5010 | 156 | TCAGAGGCAGAAATCAAAGTCCactgactgactgaGATTGCCGACCTGACCTACA |
| EPHA5 | 5011 | 157 | ATTTATTGGATTCACGCACTGTCactgactgaGAAGAGATTGGTGAAGTGGATGA |
| CDH5 | 5012 | 158 | CGACTTCTACGTGGTCACCTACACactgactgactgGACAACGCCATTCGGAGTG |
| PDE4DIP | 5013 | 159 | GCAGATACCTTCCAGAGATGATAGactgactgCACATTAGGAGACTTGGACACAG |
| TYMS | 5014 | 160 | TGTGCCAGTTCTTTCCATAATAAAactgacATGAACAAAGTGAGGAGAATGAAAT |
| MAP2K4 | 5015 | 161 | GCTGCCAACTTGATGTTCCAactgactgactTACAGAAGGCAAAGTTTACAGATG |
| CPSG3 | 5016 | 162 | CCAGCATCACCACCACAAATactgactgactgacAAGGACGAAGGGAATTTCTGC |
| MCM2 | 5017 | 163 | TTGGCGTGAGTTGCGTATTCactgactgacGATGTTTGTAATCGTTTTCAGTCTC |
| PRDM2 | 5019 | 164 | TTCTTCTTCATCTTCCTCCTCTTCactgactgacTCTGGTGATAATCTGGAGGCT |
| SLC6A8 | 5020 | 165 | GCCTCCTACTACTTCCGTTTCactgactgactgacGTGACTGATGGCGGGATGTA |
| NME1 | 5021 | 166 | GAGGAACTGGTAGATTACACGAGactgactgactgTGAATGACAGGAGGGCAGAC |
| TNFRSF25 | 5022 | 167 | CAAGGCGAAGAAGCACGAACactgactgactgaAGACATTTCTCAACTTCTCGGC |
| PRKCZ | 5023 | 168 | AGACGATGAGGATGCCATAAAGactgactgactgCATTATTGCTGTCCACCGAGG |
| CAMTA1 | 5024 | 169 | AAATGACAGATGGTAGAGACTTCCactgactgaCCTACCAGTGTAACCTACCAGT |
| AKR1C1 | 5025 | 170 | GGATTATGTTGACCTCTACCTTATTactgaATCCCAAAAGATGAAAATGGAAAAA |
| ELAVL4 | 5026 | 171 | GCTACGGAACCGATTACTGTGAAactgactgacTGCTCTCCCAGCTCTACCAGTC |
| NTRK1 | 5027 | 172 | CGAGAGCATCCTGTACCGTAAGTactgactgactGATCTTCACCTACGGCAAGCA |
| PRAME | 5028 | 173 | CGTAGACTCCTCCTCTCCCACATactgactgacAGGAAGAGCAGTATATCGCCCA |
| WSB1 | 5029 | 174 | CCAGAAAAACAGAGTCGCTGTGTactgactGATCAGCTACTTCTTGCTACAGGGT |
| DDC | 5030 | 175 | CGCAAGTGAATTCCGAAGGAactgactgactgactgaCATTGAGGGACGCCAGGT |
| MYCN | 5032 | 176 | GCGAGCTGATCCTCAAACGactgactgactgacGAAGAAGATAAAGAGCGAGGCG |
| PLAT | 5033 | 177 | CCGGCTACGGCAAGCAactgactgactgaatgTCATGTCAGACTGTACCCATCCA |
| CD44 | 5034 | 178 | TGCCGCTTTGCAGGTGTATactgactgactgactgactTCTCTCGGACGGAGGCC |
| CDKN3 | 5035 | 179 | TCCAGTAGCTGCTTGTCTCCTACTATAactgacAGACAGCCTGCGAGACCTAAGA |
| FYN | 5036 | 180 | CCTTTCTTATCCGCGAGAGTGAactgactgaATTGGGATGATATGAAAGGAGACC |
| PAICS | 5037 | 181 | TGTCACCCAGGTTCGTCTCAactgactgaaCCAAAGTGCTAGGGTTACAGACATG |
| SCG2 | 5039 | 182 | ACTTTCCAATGGACATGAGTGATGactgactgaAAGCACATGCAATTCCCTCCTA |
| MAP7 | 5406 | 183 | ATCCCAAAGCAAGACAACCAGactgactgactgaGTCTCTTCCTCATTTGCCTGG |
| CADM1 | 5407 | 184 | CGCGCTTGAGTTAACATGTGAAactgactgactTGGTAACTTGGGTGAGAGTCGA |
| PRKACB | 5408 | 185 | GGGCATTAGGAGTGCTAATCTATGAactgactgTTGCAGACCAACCAATTCAGAT |
| NRCAM | 5409 | 186 | GAGAACACAGGGCAAGACACATACactgactgaTGCATTCTGTATCAGCTGGTCC |
| PIK3R1 | 5410 | 187 | GGGAAGCGAGATGGCACTTactgactgactgaGCTATGCCTGCTCTGTAGTGGTG |
| UBC | 5411 | 188 | ATTTGGGTCGCGGTTCTTGactgactgactgactACCATCGAGAATGTCAAGGCA |
| HPRT1 | 5412 | 189 | TGACACTGGCAAAACAATGCAactgactgactgaAGCTTGCTGGTGAAAAGGACC |
| HMBS | 5413 | 190 | GGCAATGCGGCTGCAAactgactgactgactgactgGTGATTCGCGTGGGTACCC |
| SDHA | 5414 | 191 | TGGGAACAAGAGGGCATCTGactgactgactgaCATGAATTTGATGCAGTGGTGG |
| ALUsq | 5415 | 192 | CATGGTGAAACCCCGTCTCTAactgactgactgactCTACTCGGGAGGCTGAGGC |

### Expression analysis by sequencing-based methods

Sequencing-based strategies are also possible. RNA is sequenced directly (RNAseq) using sequencing tools known in the art. Preferably, the so called "next generation sequencing tools" are used. The expression level of a certain target is then determined based on the number of specific target sequences occurring in the total pool of sequences retrieved from the reaction. Non-limiting examples of next generation sequencing platforms or technologies are:
- Supported oligonucleotide detection (SOLiD™) from Applied Biosystems. This method, which determines mRNA expression levels by sequencing unique sequence tags isolated from the 5' untranslated region of full-length mRNAs, has significant advantages over microarray technologies since it can detect known and novel mRNAs in a single cell, enables analysis of 1-256 samples per run, is highly reproducible, with a dynamic range that is orders of magnitude greater than microarrays, detects expression of transcripts from < 1 copy per cell to over 100,000 copies per cell, corresponding to a dynamic range of > 105 and detects transcripts present at levels 100X lower than microarrays.
- 454 Life Sciences / Roche provides for a method of rapid and accurate Pyrosequencing of Serial Analysis of Gene Expression (SAGE) using Ditags (Eveland et al. Plant Physiology 146: 32-44. January 2008.)
- Pyrosequencing as such can also be used as described by Agaton C et al. (Gene. 2002 May 1;289(1-2):31-9)
- Solexa™ from Illumina as described in Barski A et al. Cell. 129(4):823-37.)
- Heliscope™ from Helicos BioSciences
- Polony Cyclic Sequencing by Synthesis described by Mitra, R. and Church, G.M. Nucleic Acids Res. 27(24):e34; pp.1-6.)
- Genovoxx AnyGene Technology™
- Simultaneous Bi-directional Sequencing (SBS™) from LI-COR
- Single-molecule sequencing (e.g. Single molecule real time (SMRTtm) from Pacific Biosciences or Helicos True Single Molecule Sequencing (tSMS)™and the like)
- VisiGen Biotechnologies sequencing technology
- Hybridization-Assisted Nanopore Sequencing (HANS™) from Nabsys or SEQUENOM Inc. San Diego, CA USA (cf. Cantor CR, Nucleic Acids Symp Ser (Oxford Press). 2008;(52):15)). The technology uses an automated mass spectrometry platform for the quantitative analysis of DNA and RNA in a variety of settings including genotyping, gene copy number measurements, gene expression, epigenetics, and automated bacterial and viral identification.
- Sequencing by synthesis is another method. One example is the Four-color DNA sequencing by synthesis using cleavable fluorescent nucleotide reversible terminators reported by Juet al., (PNAS December 26, 2006 vol. 103 no. 52 19635-19640).

### Expression analysis by hybridisation

### Microarray detection of gene expression

### Microarrays

According to another embodiment of the invention, microarrays comprising gene-specific hybridization probes attached to a solid support are provided as means for determining the expression level of target mRNAs or miRNAs. The term "hybridization probe" or "probe" or "gene-specific probe" is intended to mean a nucleotide fragment comprising from 5 to 100 nucleic units, in particular from 10 to 35 nucleic units, having a hybridization specificity under given conditions so as to form a hybridization complex with the material specific for a target gene. In the present invention, the material specific for the target gene can be a nucleotide sequence included in a messenger RNA derived from the target gene (reference is then made to an mRNA specific for the target gene), a nucleotide sequence included in a complementary DNA obtained by reverse transcription of said messenger RNA (reference is then made to a cDNA specific for the target gene), or else a nucleotide sequence included in a complementary RNA (cRNA)obtained by transcription of said DNA or cDNA.

The hybridization probe can comprise a label for its detection or the material specific for the target gene can be labeled due to its synthesis. For the purpose of the present invention, the hybridization probe may thus either be a "detection" probe, in which case the "detection" probe is labelled with a label as defined above, or a "capture" probe, in which case the "capture" probe is immobilized or can be immobilized on a solid support by any appropriate means, i.e. directly or indirectly, for example by covalence or adsorption.

The term "detection" is intended to mean either a direct detection by a physical method, or an indirect detection by a method of detection using a label. Methods for the detection of nucleic acids are well known in the art (cf. e.g. Kricka et al., Clinical Chemistry, 1999, No. 45(4), p. 453-458 or Keller G. H. et al., DNA Probes, 2nd Ed., Stockton Press, 1993, sections 5 and 6, p. 173-249).

The term "label" is intended to mean a tracer capable of engendering a signal that can be detected. A non-limiting list of these tracers includes the enzymes which produce a signal detectable, for example, by colorimetry, fluorescence or luminescence, such as horseradish peroxydase, alkaline phosphatase, beta galactosidase, or glucose-6-phosphate dehydrogenase; chromophores, such as fluorescent, luminescent or dye compounds; electron-dense groups that can be detected by electron microscopy or by means of their electrical properties such as conductivity, by amperometry or voltammetry methods, or by impedance measurements; groups that can be detected by optical methods such as diffraction, surface plasmon resonance, or contact angle variation, or by physical methods such as atomic force spectroscopy, tunnel effect, etc.; and radioactive molecules such as ³²P, ³⁵S or ¹²⁵I.

As a solid support, use may be made of synthetic materials or natural materials, that are optionally chemically modified, in particular polysaccharides, such as cellulose-based materials, for example paper, cellulose derivatives such as cellulose acetate and nitrocellulose or dextran, polymers, copolymers, in particular based on styrene-type monomers, natural fibres such as cotton, and synthetic fibres such as nylon; mineral materials such as silica, quartz, glasses or ceramics; latices; magnetic particles; metal derivatives, gels, etc. The solid support can be in the form of a microtitration plate, of a membrane as described in application WO94/12670, or of a particle. It is also possible to immobilize several different capture probes on the support, each one being specific for a target gene. In particular, it is possible to use, as support, a biochip or microarray on which a large number of probes can be immobilized. The term biochip or microarray is intended to mean a solid support that is small in size and to which a multitude of capture probes are attached at predetermined positions. The operating principle is based on a foundation of molecular biology: the hybridization phenomenon, i.e. the pairing by complementarity of the bases of two DNA and/or RNA sequences. The detection or capture probes are positioned specifically on the support or chip and each hybridization gives a specific piece of information, in relation to the target nucleotide fragment. The pieces of information obtained are, cumulative, and make it possible, for example, to quantify the level of expression of a target gene or of several target genes. To analyse the expression of a target gene, it is therefore possible to prepare a biochip carrying a very large number of probes which correspond to all or part of the target gene, which is transcribed into mRNA. The cDNAs or the cRNAs specific for a target gene that it is desired to analyse, for example, are then hybridized on specific capture or detection probes. After hybridization, the support or chip is washed, and the labelled cDNA or cRNA/capture probe complexes are revealed with a high-affinity ligand bound, for example, to a fluorochrome-type label. The fluorescence or radioactivity is read, for example, with a scanner and the analysis of the fluorescence or radioactivity is processed by computer technology. By way of indication, mention may be made of the DNA chips developed by the company Affymetrix (M. Chee et al., Science, 1996, 274, 610-614; A. Caviani Pease et al., Proc. Natl. Acad. Sci. USA, 1994, 91, 5022-5026), for molecular diagnoses. In this technology, the capture probes are generally small in size, around 25 nucleotides. Other examples of biochips are given in the publications by G. Ramsay, Nature Biotechnology, 1998, No. 16, p. 40-44; F. Ginot, Human Mutation, 1997, No. 10, p. 1-10; J. Cheng et al, Molecular diagnosis, 1996, No. 1(3), p. 183-200; T. Livache et al, Nucleic Acids Research, 1994, No. 22(15), p. 2915-2921; J. Cheng et al, Nature Biotechnology, 1998, No. 16, p. 541-546 or in patents US 4,981,783, US 5,700,637, US 5,445,934, US 5,744,305 and US 5,807,522. The main characteristic of the solid support must be that of conserving the characteristics of hybridization of the capture probes on the target nucleotide fragments while at the same time generating a minimum background noise for the method of detection.

For the immobilization of the detection or capture probes on the support, three major types of fabrication are distinguished:

First of all, there is a first technique which consists in depositing presynthesized probes. The attachment of the probes occurs by direct transfer, by means of micropipettes or of microtips, or by means of an inkjet-type device. This technique makes it possible to attach probes having a size ranging from a few bases (5 to 10) up to relatively large sizes of 60 bases (printing) to a few hundred bases (microdeposition):

Printing is an adaptation of the method used by inkjet printers. It is based on the propulsion of very small spheres of fluid (volume<1 nl) at a rate that can reach 4000 drops/second. The printing does not involve any contact between the system releasing the fluid and the surface on which it is deposited.

Microdeposition consists in attaching long probes of from a few tens of bases to several hundred bases to the surface of a glass slide. These probes are generally extracted from databases and are in the form of amplified and purified products. This technique makes it possible to produce chips called microarrays that carry approximately ten thousand spots, called recognition zones, of DNA on a surface area of slightly less than 4 cm². The use of nylon membranes, called "macroarrays", which carry amplified products, generally PCR-amplified products, with a diameter of 0.5 to 1 mm, and the maximum density of which is 25 spots/cm², should not, however, be forgotten. This very flexible technique is used by many laboratories. In the present invention, this latter technique is considered to be part of the biochips. It is, however, possible to deposit at the bottom of a microtitration plate a certain volume of sample in each well, as is the case in patent applications WO00/71750 and FR 00/14896, or to deposit at the bottom of the same Petri dish a certain number of drops that are separated from one another, according to another patent application FR 00/14691.

The second technique for attaching the probes to the support or chip is called in situ synthesis. This technique results in the development of short probes directly at the surface of the chip. It is based on the synthesis of oligonucleotides in situ (see, in particular, patent applications WO89/10977 and WO90/03382), and is based on the oligonucleotide synthesizer method. It consists in moving a reaction chamber, in which the oligonucleotide elongation reaction takes place, along the glass surface.

Finally, the third technique is called photolithography, which is a process used for the biochips developed by Affymetrix. It is also an in situ synthesis. Photolithography is derived from microprocessor techniques. The surface of the chip is modified by the attachment of photolabile chemical groups that can be light-activated. Once illuminated, these groups are capable of reacting with the 3' end of an oligonucleotide. By protecting this surface with masks of defined shapes, it is possible to selectively illuminate and therefore activate areas of the chip where it is desired to attach one or other of the four nucleotides. The successive use of different masks makes it possible to alternate cycles of protection/reaction and therefore to produce the oligonucleotide probes on spots of approximately a few tens of a micrometre squared. (µm²). This resolution makes it possible to create up to several hundred thousand spots on a surface area of a few centimetres squared (cm²). Photolithography has advantages: in bulk in parallel, it makes it possible to create a chip of N-mers in only 4×N cycles. All these techniques can of course be used in the present invention.

### Other hybridization-based techniques

Other non-microarray-based hybridisation techniques can also be used for expression analysis. Non-limiting examples are the known northern-blot hybridization technologies (cf. Sambrook, J. et al. in "Expression of cloned genes in E. coli", Molecular Cloning: A Laboratory Manual (1989), Cold Spring Harbor Laboratory Press, New York, USA, 9.47-9.62 and 11.45-11.61), RNA-in-situ hybridization (RISH) and the recently developed digital analysis of gene expression (DAGE) such as the e.g. the nCounter™ technology by Nanostring. DAGE is e.g. described in Mikkilineni V et al (Biotechnol Bioeng. 2004 Apr 20;86(2):117-24)) and in Velculescu and Kinzler (Nature Biotechnology 25, 878 - 880 (2007)), and is an accurate and precise technology for measuring digital gene expression on a relative or absolute scale by simply counting the number of transcripts of a gene being expressed at a given time by means of "barcode"-labelled probes, wherein the barcode can e.g. be made through a range of fluorescent spectra each linked to a specific probe. The technology uses molecular barcodes and single molecule imaging to detect and count hundreds of unique transcripts in a single reaction, and eliminates the need for enzymology. Because the assay directly targets RNA molecules through hybridization, there is no need to even purify RNA, let alone make cDNA or PCR amplify saving serious time and money.

### cDNA detection of gene expression

After having extracted, as biological material, the RNA from a biological sample as presented above, a reverse transcription step is carried out according to standard procedures in order to obtain cDNAs complementary to the mRNAs derived from a-target gene (cDNA specific for the target gene) and cDNAs complementary to the mRNAs derived from genes other than the target gene (cDNA not specific for the target gene).

All the cDNAs are brought into contact with a microarray according to the invention, on which are immobilized capture probes specific for the target gene whose expression it is desired to analyse, in order to carry out a hybridization reaction between the cDNAs specific for the target gene and the capture probes.

The hybridization reaction can be preceded by a step consisting of enzymatic amplification of the cDNAs specific for the target gene as described above, so as to obtain a large amount of cDNAs specific for the target gene and to increase the probability of a cDNA specific for a target gene hybridizing with a capture probe specific for the target gene.

The hybridization reaction can also be preceded by a step consisting in labelling and/or cleaving the cDNAs specific for the target gene as described above, for example using a labelled deoxyribonucleotide triphosphate for the amplification reaction. The cleavage can be carried out in particular by the action of imidazole and manganese chloride. The cDNA specific for the target gene can also be labelled after the amplification step, for example by hybridizing a labelled probe according to the sandwich hybridization technique described in document WO91/19812. Other preferred specific methods for labelling and/or cleaving nucleic acids are described in applications WO99/65926, WO01/44507, WO01/44506, WO02/090584 and WO02/090319.

A step consisting of detection of the hybridization reaction is subsequently carried out. The detection can be carried out by bringing the support on which the capture probes specific for the target gene are hybridized with the cDNAs specific for the target gene into contact with a "detection" probe labelled with a label, and detecting the signal emitted by the label. When the cDNA specific for the target gene has been labelled beforehand with a label, the signal emitted by the label is detected directly.

### cRNA detection of gene expression

The expression of a target gene in the biological material or sample obtained from the patient can also be determined by analyzing cRNA based on the extracted biological material e.g. the mRNA from a biological sample. First, cDNAs is prepared from the mRNA template from the biological material obtained by carrying out a reverse transcription step. The polymerization of the complementary RNA (cRNA) of the cDNA is subsequently carried out using a T7 polymerase enzyme which functions under the dependency of a promoter and which makes it possible to obtain, from a DNA template, the complementary RNA. The cRNAs of the cDNAs of the mRNAs specific for the target gene (reference is then made to cRNA specific for the target gene) and the cRNAs of the cDNAs of the mRNAs not specific for the target gene are then obtained.

All the cRNAs are brought into contact with the microarray of the invention on which are immobilized capture probes specific for the target genes whose expression it is desired to analyse in order to carry out a hybridization reaction between the cRNAs specific for the target gene and the capture probes. The hybridization reaction can also be preceded by a step consisting in labelling and/or cleaving the cRNAs specific for the target gene, as described above.

A step consisting of detection of the hybridization reaction is subsequently carried out. The detection can be carried out by bringing the support on which the capture probes specific for the target gene are hybridized with the cRNA specific for the target gene into contact with a "detection" probe labelled with a label, and detecting the signal emitted by the label. When the cRNA specific for the target gene has been labelled beforehand with a label, the signal emitted by the label is detected directly. The use of cRNA is particularly advantageous when a support of biochip type on which a large number of probes are hybridized is used.

### Gene expression analysis

Typically, the expression analysis comprises the steps of labeling a gene transcript obtained from a tumor cell of a patient diagnosed as having neuroblastoma (e.g. labeled RNA, mRNA, ncRNA or cDNA based thereon); bringing said labeled gene transcript into contact with any one of the microarrays according to the invention and measuring the labeling signal of each of the gene transcripts hybridized to the probes related to good prognosis and the probes related to poor prognosis on the microarray.

The determination of the expression of a target gene can be carried out by any of the protocols known to those skilled in the art. In general, the expression of a target gene can be analysed by detecting the mRNAs (messenger RNAs) that are transcribed from the target gene at a given moment or by detecting the proteins derived from these mRNAs. This can be done through PCR analysis of copy DNA (cDNA) obtained from the template mRNA from the sample or by hybridization of a mixture of a microarray coated with gene specific probes.

The invention relates preferably to the determination of the expression of a target gene by detection of the mRNAs derived from this target gene according to any of the protocols well known to those skilled in the art. According to one specific embodiment of the invention, the expression of several target genes is determined simultaneously, by detection of several different mRNAs, each mRNA being derived from a target gene.

### Prognosis

The prognostic method according to the invention thus determines the expression levels of the genes identified in the gene signature of the invention, and indicates that the prognosis of the patient is good if the correlation between the prognostic multi-gene signature and the expression profile of the given patient is negative and poor if the correlation is positive.

Table 1 gives the values of the prognostic multi-gene signature. As this signature is based on the expression levels of a certain set of training samples (2 times 15), it might slightly change if another training set is used.

The methods and tools of the present invention will make it possible to make a better choice of risk-related therapy. The current stratification methods or parameters allow a rather general classification and are sometimes not correct. This results in children being subjected to heavy treatment regimes or experimental treatments because they were classified in the high risk group, while they should have been classified in the lower risk group. Similarly, patients stratified as being in the low risk group may receive a treatment that does not suffice or is inefficient since the patient should have been classified as having a high risk. The classifier of the invention makes it possible to distinguish better between the high and low risk groups and can to some extent also subdivide the high risk group of patients into very high and high risk groups. The first group of very high patients may for instance be treated with experimental drugs, while the "normal" high group is not. Similarly, the low risk group can be subdivided according the method of the invention into very low risk and "normal" low risk patient. Similarly, patients who have a poor gene signature and who are currently treated with surgery alone or mild chemotherapy might benefit from more appropriate therapies, i.e. according to the current intensive multimodal protocol (HR-NBL1).

The term "good prognosis" as used in the present specification refers to a condition of human neuroblastoma in which the tumor is localized or has become a regressing or benign sympathetic ganglion neoplasm, and is judged to have low malignancy based on N-myc or other tumor markers. Typically, a "good prognosis" case is a case of stage 1 or 2, with an onset age of less than one year and survival without recurrence for 5 or more years after surgery, and with no noted amplification of *MYCN* in the clinical tissue; however, there is no limitation to such specific cases.

The term "poor prognosis" as used in the present specification refers to a condition of human neuroblastoma in which progression of the tumor has been observed, and it is judged to have high malignancy based on *MYCN* or other tumor markers. Typically, a "poor prognosis" case is a case of stage 4, with an onset age of greater than one year, death within 3 years after surgery and noted amplification of *MYCN* in the clinical tissue; however, there is no limitation to such specific cases.

The terms "predicting prognosis" or "prognosing" mean to predict whether the postoperative or post-treatment status of a patient with neuroblastoma is good or poor. More specifically, the term "good prognosis" indicates the status in which a neuroblastoma is localized or regressed, or it becomes a benign sympathetic ganglion cell tumor. Examples include the case where the patient is alive 5 years or more after the operation without recurrence. The term "poor prognosis" indicates the status in which the progression or relapse of neuroblastoma is confirmed, and examples include the status where there is a risk that the patient will die within 3 years after the operation.

The term "low risk" indicates a neuroblastoma tumor of the following characteristics: stage 1,2 or 4s, having *MYCN* single copy, and the patient being alive (progression free survival (PFS)> 1000 days)

The term "high risk" indicates a neuroblastoma tumor of the following characteristics: stage 2/3, having an amplified *MYCN,* > 1 year, dead of disease or stage 4 (= disseminated disease, > 1 year, dead of disease.

The term "intermediate risk" means any stage in between the low and high risk groups.

The term "Progression Free Survival" or "PFS" indicates: Progression free survival (PFS) was calculated from the day of diagnosis until the date of last follow-up or tumor progression or relapse.

The term "Overall survival" or "OS" indicates: Overall survival (OS) was calculated from the day of diagnosis to the last follow-up or disease-related death. Deaths due to toxicity were censored.

### Neuroblastoma and neuroblastoma-like tumors

The term "Neuroblastoma" as used in the present application means a tumor consisting of at least 60% tumor cells (neuroblastic or schwannian cells). Neuroblastoma (NB) is one of the most frequent extracranial solid tumors in children. It is a neuroendocrine tumor, arising from any neural crest element of the sympathetic nervous system (SNS). Its solid tumors, which take the form of a lump or mass, commonly begin in one of the adrenal glands, though they can also develop in nerve tissues in the neck, chest, abdomen, or pelvis. Neuroblastoma is one of the peripheral neuroblastic tumors (pNTs) that have similar origins and show a wide pattern of differentiation ranging from benign ganglioneuroma to stroma-rich ganglioneuroblastoma with differentiating neuroblastic cells intermixed or in nodules, to highly malignant neuroblastoma.

This distinction in the pre-treatment tumor pathology is an important prognostic factor, along with age and mitosis-karyorrhexis index (MKI). This pathology classification system describes tumors as having a "good" or "favorable" and "poor" or "unfavorable" prognosis by the International Neuroblastoma Pathology Committee (INPC, also called Shimada system). Cancer cells from neuroblastoma with good prognosis are characterized by having a very slow rate of proliferation, with spontaneous regression beginning at some point. Findings to date have confirmed that nerve cell differentiation and apoptosis (nerve cell death) occur in the spontaneous regression, and that the differentiation which occurs in the maturation stages of normal nerve cells and programmed cell death are phenomena very closely resembling each other. Consequently, it is highly probable that the analysis of genes expressed in such tumors will lead to obtaining important information relating to nerve cell differentiation and apoptosis.

Currently neuroblastoma can be classified in several stages, making it possible to define prognostic groups as specifically as possible. These groups theoretically make it possible to define the therapeutic indications in a manner that is adapted to the risk of the disease (Brodeur et al. (1993) J. Clin. Oncol. 11, 1466-77). According to this classification, the following stages are distinguished:
o stage 1: localized tumor with complete gross excision; ipsilateral and controlateral lymph nodes examined and negative microscopically,
o stage 2A: unilateral tumor with incomplete excision, ipsilateral and controlateral lymph nodes examined and negative;
o stage 2B: unilateral tumor with ipsilateral lymph node involvement but controlateral lymph nodes negative;
o stage 3: inoperable unilateral tumor infiltrating across the midline, or unilateral tumor with controlateral lymph node involvement, or midline tumor with bilateral extension by infiltration or by adenopathy;
o stage 4: primary tumor with distant dissemination: to lymph nodes, bone, bone marrow, liver;
o stage 4S: local stage 1 or 2 tumor with dissemination limited to liver, skin or bone marrow. The 4S stages are children less than 1 year old.

Recently, a new staging system has been established (INRGSS: international neuroblastoma risk group staging system):
o Stage L1: Locoregional tumor not involving vital structures as defined by the list of Image Defined Risk Factors (IDRF)
o Stage L2: Locoregional tumor with presence of one or more Image Defined Risk Factor
o Stage M: Distant metastatic disease (except Stage Ms)
o Stage Ms: Metastatic disease confined to skin and/or liver and/or bone marrow

The term "neuroblastoma-like tumor" encompasses tumors which are pathologically identified as being so called "small-blue cells" due to their basophilic characteristics. Such tumors include nephroblastomas medulloblastomas, ewing tumors, lymphoblastic lymphomas, pneumoblastomas and desmoplastic small round cell tumors.

### Other definitions

The term "nucleic acid(s)" as used in this invention refers to, for example, DNA or RNA, or polynucleotides derived therefrom which are active as DNA or RNA, and preferably they are DNA or RNA, but also encompasses micro-RNA (miRNA). The term "polynucleotide" used herein means a molecule in which a plurality of, preferably not less than 30 purine or pyrimidine bases are incorporated.

The term "isolated nucleic acid(s)" as used in the present specification refers to a nucleic acid or a polynucleotide containing substantially no cellular substances or culture medium, if prepared by recombinant DNA techniques, or containing substantially no precursor chemical substances or other chemical substances, if prepared by chemical synthesis.

The term "hybridize under stringent conditions" means that two nucleic acid fragments hybridize to each other under the hybridization conditions described by Sambrook, J. et al. in "Expression of cloned genes in E. coli", Molecular Cloning: A Laboratory Manual (1989), Cold Spring Harbor Laboratory Press, New York, USA, 9.47-9.62 and 11.45-11.61.

More specifically, the "stringent conditions" refers to hybridization at approximately 45° C., 6.0×SSC, followed by washing at 50° C., 2.0×SSC. The stringency may be selected by choosing a salt concentration in the washing step from approximately 2.0×SSC, 50° C. as low stringency to approximately 0.2×SSC, 50° C. as high stringency. Also, the temperature in the washing step may be increased from room temperature, or approximately 22° C. as low stringency conditions, to approximately 65° C. as high stringency conditions.

The term "gene transcript" is referred to as an RNA transcribed from genomic gene or a cDNA synthesized from this mRNA or can be a non-coding RNA (ncRNA) such as a micro-RNA (miRNA).

The term "biological sample" or "sample" or "patients' sample" is intended to mean any sample taken from a patient, and which may contain a biological material as defined hereinafter. This biological sample can in particular be a sample of tumor, i.e. a biopsy or a dissected cell thereof, a tissue sample of the patient such as bone-marrow or circulating cells from the patient, or bodily fluids from the patient such as cerebrospinal fluid, peritoneal fluid and intraperitoneal fluid, blood, blood components, serum or plasma, saliva, urine. This biological sample is provided by any type of means for taking a sample known to those skilled in the art. According to a preferred embodiment of the invention, the biological sample taken from the patient is a tumor sample, i.e. a biopsy or a dissected cell thereof, bone marrow sample, or other tissue sample.

The term "biological material" used in the present invention can comprise any material that makes it possible to detect the expression of a target gene. The biological material can in particular comprise nucleic acids such as, in particular, deoxyribonucleic acids (DNA) or ribonucleic acids (RNA) or proteins. The nucleic acid is preferably a RNA (ribonucleic acid). According to a preferred embodiment of the invention, the biological material comprises nucleic acids, preferably RNAs, and even more preferably total RNAs. The total RNAs comprise the transfer RNAs, the messenger RNAs (mRNAs), such as the mRNAs transcribed from the target gene, but also transcribed from any other gene and the ribosomal RNAs and non-coding RNAs such as microRNAs (miRNA).

microRNAs (miRNA) are single-stranded RNA molecules of about 21-23 nucleotides in length, which regulate gene expression. miRNAs are encoded by genes that are transcribed from DNA but not translated into protein (non-coding RNA); instead they are processed from primary transcripts known as *pri-miRNA* to short stem-loop structures called *pre-miRNA* and finally to functional miRNA. Mature miRNA molecules are partially complementary to one or more messenger RNA (mRNA) molecules, and their main function is to downregulate gene expression (Ruvkun G et al., 2001, Science 294:797-9 and Mestdagh P. et al., 2008, Nucleic Acid Research 2008, 1-8).

### Algorithms used

Generally speaking, two steps are taken in order to come to the classifiers of the invention. It should be clear that the skilled person could use any suitable algorithm or software program known in the art and that the algorithms used in the examples are merely non-limiting examples.

Step 1 encompasses the gene reduction, i.e. the reduction of the number of genes that have prognostic value, starting from a larger list of several hundreds of genes that are e.g. reported as being differentially expressed in neuroblastoma tumors of different types.

In Step 2 said reduced gene list is trained in order to obtain a workable prognostic classifier. This training is done based on the expression data of the genes from the reduced gene list obtained in step 1 in a panel of training samples that are specifically selected to have clearly different phenotypes. In this case, the training samples usually are two groups of tumors that are clearly distinct in phenotype, i.e. one group of tumors that can clearly be classified as being high risk versus another group of tumors that can clearly be classified as being low risk. Based on the expression data in these two distinct tumour sample pools, the expression level of each of the genes from the reduced list gets its prognostic value.

When comparing the expression levels of the genes of the reduced list in a random sample with the values obtained for the training samples, one can classify the random sample as belonging either to the high risk or low risk group. Appropriate algorithms are well known in the art and some non-limiting examples are used in the experimental section.

Similar approaches were used for building and validating the classification signature based on miRNA expression.

Another object of the invention is to perform an integrative analysis of the established multigene mRNA expression classifier with the miRNA expression data, available gene copy number profiles, the generated methylation marker profiles and the currently used clinico-biological factors. Combining information from each of those individual information layers should lead to the design of a powerful multi-level information predictor for risk stratification. Such prognostic classifiers are of crucial importance for more accurate assessment of prognosis in children with neuroblastoma, in order to make a better choice of risk-related therapy and to identify subgroups of patients for inclusion in clinical trials for new therapeutic compounds.

With regard to other tumor entities, the same workflow can be applied on economically more interesting malignancies, such as leukaemia, lung, breast and colon cancer.

Reference is made herein to the accompanying examples and drawings that show, by way of illustration only, specific embodiments in which the invention may be practiced. These embodiments are described in sufficient detail to enable those skilled in the art to practice the invention. It is to be understood that the various embodiments of the invention, although different, are not necessarily mutually exclusive. For example, a particular use, method, feature, or characteristic described herein in connection with one embodiment may be implemented within other embodiments without departing from the spirit and scope of the invention. In addition, it is to be understood that each disclosed embodiment may be modified without departing from the spirit and scope of the invention. The detailed description, drawings and examples are, therefore, not to be taken in a limiting sense, and the scope of the present invention is defined only by the appended claims, appropriately interpreted, along with the full range of equivalents to which the claims are entitled.

### EXAMPLES

The invention is illustrated by the following non-limiting examples

### Example 1: Establishing a 42 gene classifier

In this study, we established a sensitive and specific prognostic 42-gene classifier for children with NB by re-analysis of four published gene expression studies from four different microarray platforms analyzing in total 582 patients (Oberthuer et al., J Clin Oncol. 2006 Nov 1;24(31):5070-8; Wang Q et al., Cancer Research 15;66(12):6050-62 2006); Berwanger et al., Cancer Cell 2002, 2(5):377-386; Ohira et al., Cancer Cell. 2005 Apr;7(4):337-50). In order to facilitate data comparison across different platforms, probe annotations were updated with respect to the original publications. When available, clinical follow-up information was updated. All these aspects critically contribute to the success of our multi-gene signature. Successful validation of the multi-gene signature in four independent unpublished datasets demonstrates its robust performance and platform independence.

### Materials and Methods

### Gene expression datasets

Four published studies were used for selecting the genes and building the prognostic classifier (phase 1 datasets) and four unpublished datasets were used as independent validation sets (phase 2 datasets).

The phase 1 datasets were either downloaded from the NCBI GEO (GSE2283 and GSE3960), or from the EBI ArrayExpress database (E-TABM-38), or from the authors' website (http://www.imt.uni-marburg.de/microarray/download.html).

A trained multi-gene correlation signature was validated on the four independent phase 2 datasets from which the 42 genes (when present) were extracted and standardized (per gene, the median value across the samples was subtracted, followed by division by the gene's standard deviation): 1) hgu95av2 Affymetrix gene expression data from 106 NB patients (validation set 1) (40 genes present), 2) hgu133plus2 Affymetrix gene expression data from 53 NB patients (validation set 2) (40 genes present), 3) dataset for 91 NB patients obtained using an 11K custom Agilent oligonucleotide micro-array (validation set 3) (41 genes present) and 4) Human Exon 1.0 ST Affymetrix expression data from 101 NB patients (validation set 4) (42 genes present) (Figure 1).

For the remainder of the manuscript, we will label the datasets according to the first author for the published phase 1 studies (Oberthuer, Wang, Berwanger and Ohira) and as validation set 1, 2, 3 and 4 for the unpublished phase 2 studies.

### Data-preprocessing

In order to make the data from the different micro-array platforms maximally comparable, annotation information of the probes was updated using the MatchMiner tool for the custom-made cDNA or oligonucleotide arrays and using the latest version of the R packages hgu95av2 and hgu133plus2 for the Affymetrix array data. Probe identification numbers were converted into gene symbols to enable straightforward comparison of the gene lists between the different studies. Throughout the text, the number of unique gene symbols (represented by one or more array-probes) in each study is indicated.

Updated clinical information with regard to progression free and overall survival times were obtained from the authors or were publically available. For the Berwanger and Ohira studies and validation set 1, only overall survival data were available.

Patients were divided in 2 clearly defined risk groups. The low-risk subgroup was defined by stage 1, 2 or 4S without *MYCN* amplification and the high-risk subgroup comprised patients with age of diagnosis higher than 1 year with stage 4 tumours (irrespective of *MYCN* status) or with stage 2 and 3 tumours with *MYCN* amplification. To develop our classifier, as many patients as possible from the four phase 1 datasets were divided in the two risk groups with maximally divergent clinical course (Table 4), i.e. low-risk patients with progression-free survival time (PFS) (or overall survival time (OS) for Berwanger and Ohira datasets) of at least 1000 days and high-risk patients that died from disease. The patients that did not belong to the above mentioned low- or high-risk subgroups were used as independent test set.

### Statistical analysis

Identification and validation of prognostic classifiers (for each single phase 1 dataset) was performed by PAM (prediction analysis of micro-arrays) classification with 10-times-repeated 10-fold cross-validation in the R statistical language using the Bioconductor package MCRestimate (Figure 1A). Fourty-two genes were present in at least 2 of the 4 resulting gene lists.

A cross-platform gene signature was built using standardised expression data of the 42 genes (if present on the respective arrays) from four published phase 1 studies. The correlation method was used to build and test a cross-platform prognostic signature (Figure 1B). Log transformed data were merged in one file (if more than one probe was present for a certain gene, the probe with the highest expression value was selected) and for each of the 42 genes, the mean expression value in low-risk NB patients with PFS of at least 1000 days was subtracted from the mean expression value in high-risk NB patients that died of disease. For classification, the Pearson's correlation coefficient of the signature with the standardized expression values of independent test patients was calculated. Patients with a correlation coefficient below 0 were predicted to have good prognosis, while the other patients were predicted to have bad prognosis.

Kaplan-Meier survival analysis was performed with the R survival package (R version 2.6.1). The area under the ROC curve (AUC) was used as a measure for the accuracy of the classifiers (ROCR R-package). Multivariate forward conditional logistic regression analysis was performed using SPSSv16.

### Results

### Gene prioritization for inclusion in a robust prognostic classifier

A complete 10-times-repeated 10-fold cross-validation using the PAM algorithm was performed on the training patients belonging to one of the two clearly defined risk groups from the four published phase 1 studies separately, in order to identify robust prognostic markers (Figure 1). This process was accompanied by determination of the classification accuracy, providing a first estimation of the utility of the expression data to predict outcome (Table 4).

For each dataset we selected the probes that were included in at least 65 of the 100 cross-validation gene lists as these genes are likely to be the ones with the highest prognostic value as previously published (Oberthuer et al). The resulting prognostic gene lists from the four studies showed significant overlap (Table 5). Two genes were in common between 3 lists (i.e. *MYCN* and *NTRK1*), while 40 genes were in common between 2 lists. Thirty-two were previously reported in at least one of 10 published prognostic gene lists, of which only 10 were found in 2 or more published prognostic lists. The occurrence of the 42 genes in at least 2 of the 4 lists makes them robust platform independent, prognostic markers.

### Classification performance of the 42-gene list

Next, we investigated whether the 42-gene list is able to predict prognosis across different datasets. The classification performance was estimated in the different phase 1 datasets using a complete, 10-times-repeated 10-fold cross-validation method using all patients from the 2 clearly defined risk groups. For this analysis, it is important to note that not all 42 genes are present on all platforms; hence the performance test was inherently done with a different number of genes for the different datasets (Table 7). As already indicated, the 10-times repeated 10-fold cross-validation provides a good estimate for the classification performance using the expression data of the selected gene list.

As a reference, the 35-, 330-, 81- and 82-gene lists obtained through single PAM analysis of each of the 4 phase 1 datasets were evaluated in the same way as the 42-gene list. The classification performance was also tested for a subset of 11 genes (from the 42-gene list) that were present on all 4 platforms. This analysis showed that all performance parameters for the 42-gene list are best or second best for all studies compared to the other gene lists whereby the overall accuracy is highest for the 42-gene list subset (AUC=0.935) (Table 7). This analysis also shows that the performance of a classifier built for a given dataset is not always best, which indicates the power and utility of our meta-analysis for the identification of a prognostic gene list by employing expression data of 250 training samples (170 low-risk and 80 high-risk). When only 11 genes of the 42-gene list were selected that are present on all 4 platforms, the overall accuracy was lower due to loss in sensitivity and positive predictive value. The 42-gene classifier was also compared with 2 published classifiers and demonstrated that the 42-gene classifier performs best.

### Validation of a cross-platform prognostic 42-gene correlation signature for neuroblastoma

A major disadvantage of the PAM classification method is the need for a training set of samples that are analysed on the same gene expression measurement platform as the one used to evaluate the test samples. We therefore applied an alternative method to build a classifier based on the 42-gene list that can be used for completely independent datasets, even on other platforms.

The prognostic signature is determined using 250 training samples from the four phase 1 studies. A 42-gene classification vector was created and tested using the correlation method (see Material and Methods) (Figure 1).

First, the correlation signature was tested on the 129 test samples (patients not belonging to the low and high-risk subgroup) from the four phase 1 studies and revealed a very high predictive power for overall survival (OS) (log-rank p=2.41 E-4) and progression-free survival (PFS) (log-rank p=3.40E-7) (Figure 2).

Next, this correlation signature was evaluated on the 4 independent phase 2 datasets (351 patients), whereby the patients could be clearly separated into groups with significant differences in OS (log-rank p=2.17E-23) and PFS (log-rank p=2.03E-21) (Figure 3A). Kaplan-Meier analysis of patients stratified using known risk factors, i.e. age, stage and *MYCN-gene* status, showed that the correlation signature outperforms these risk factors (p<0.001 except for *MYCN* amplified samples) (Figure 4). This was confirmed using multivariate logistic regression analysis evaluating age, stage, *MYCN* status and the gene classifier, indicating that the 42-gene signature is an independent predictor for PFS and OS in the 4 phase 2 datasets as well as in the test samples of the phase 1 datasets (Table 6). Of note, whereas phase 2 datasets are representative of the general NB population, test samples from the phase 1 datasets only represent intermediate risk patients.

As the different validation datasets include patients stratified using different risk stratification systems (Europe, USA and Germany), we defined a common low- and high-risk group. As there was only 1 patient out of 50 that died of disease within the common low-risk group of patients, we did not perform Kaplan-Meier analysis. However, we could show that this single patient was classified in the high-molecular risk group using our classifier. Most interestingly, the correlation signature could partition patients within the common high-risk subgroup into groups with significant differences in OS and PFS (Figure 3B) and was an independent prognostic marker (Odds' ratios above 4). In order to exclude that the significant survival differences in high-risk tumours is solely due to the effect of the *MYCN* amplification and related downstream *MYCN* signalling, we also tested the survival in high-risk tumours without *MYCN* amplification and could show that the classifier also significantly discriminates these patients with respect to outcome (Figure 3C). In line with this, inspection of the 42-gene list indicated that not all 42-genes are related to *MYCN* amplification.

**Table 4: Published phase 1 studies used for training the classifier, with indication of number of (training) samples, median overall survival (OS) or progression-free survival (PFS) (in days), and estimation of the performance of the study specific PAM classifier for prediction of unfavourable outcome**

| | Berwanger | Oberthuer | Ohira | Wang |
|---|---|---|---|---|
| number of patients | 94 | 251 | 136 | 101 |
| number of low risk training samples | 22 | 87 | 43 | 18 |
| number of high risk training samples | 13 | 25 | 20 | 22 |
| median OS/PFS (months) | OS = 43 | PFS = 55 | OS = 46 | PFS = 48 |
| specificity | 0.955 | 0.977 | 0.814 | 1.000 |
| sensitivity | 1.000 | 0.960 | 0.950 | 0.773 |
| negative predictive value | 0.929 | 0.923 | 0.704 | 1.000 |
| positive predictive value | 1.000 | 0.988 | 0.972 | 0.783 |
| accuracy | 0.971 | 0.973 | 0.857 | 0.875 |

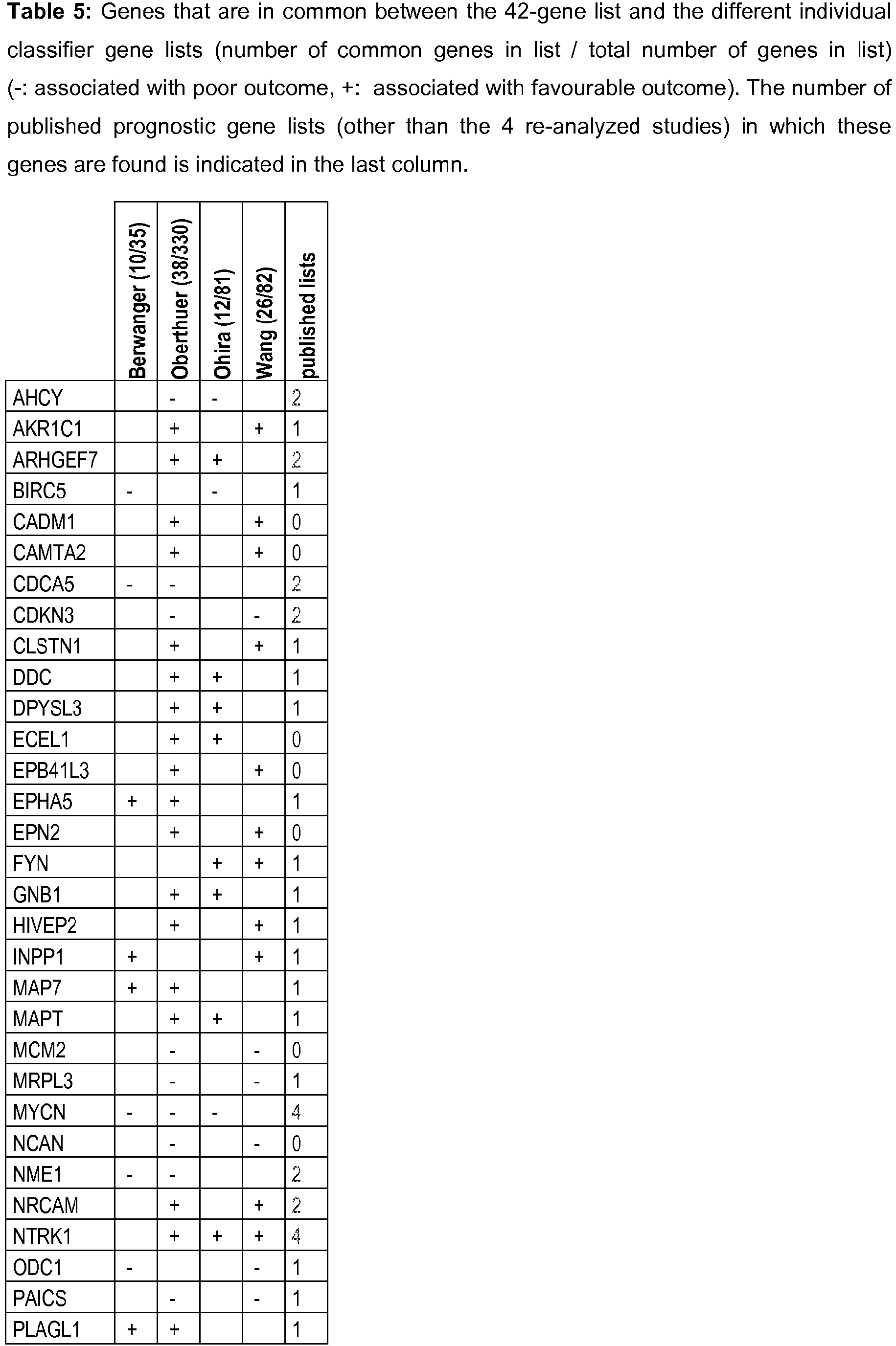

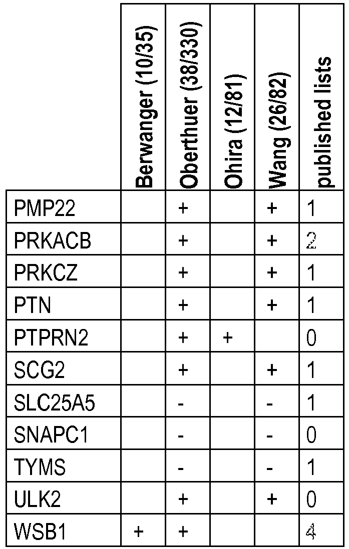

**Table 6: Multivariate logistic regression analysis (with correlation signature classification, MYCN status, INSS stage and age at diagnosis) (A) and sensitivity, specificity and accuracy (AUC with 95% Cl) results (follow-up time of at least 36 months) (B) for correlation signature prediction in the independent test samples from the phase 1 datasets and in the phase 2 validation datasets (OR = odds ratio, Cl = confidence interval, - = not analysed).**

| A | | **overall survival (OS)** | | | | **progression-free survival (PFS)** | | | |
|---|---|---|---|---|---|---|---|---|---|
| | | **p-value** | **OR** | **95% CI** | | **p-value** | **OR** | **95% CI** | |
| ***test samples from phase 1 datasets*** | **correlation signature** | 3.16E-2 | 5.11 | 1.16 | 22.58 | 3.12E-4 | 54.00 | 6.17 | 472.41 |
| | ***MYCN* amp** | 7.80E-5 | 21.50 | 4.69 | 98.54 | 1.26E-1 | - | - | - |
| | **stage (4 vs other)** | 1.80E-1 | - | - | - | 2.65E-1 | - | - | - |
| | **age (<> 1 year)** | 1.52E-1 | - | - | - | 8.65E-1 | - | - | - |
| ***phase 2 validation datasets*** | **correlation signature** | 9.07E-7 | 7.02 | 3.23 | 15.28 | 1.1E-14 | 16.45 | 8.09 | 33.48 |
| | ***MYCN* amp** | 4.19E-2 | 2.23 | 1.03 | 4.84 | 3.13E-1 | - | - | - |
| | **stage (4 vs other)** | 1.35E-2 | 2.50 | 1.21 | 5.16 | 2.16E-1 | - | - | - |
| | **age (<> 1 year)** | 1.45E-4 | 4.14 | 1.99 | 3.66 | 1.1E-4 | 4.18 2.03 | | 8.64 |

| **B** | **test samples from phase 1 datasets** | | **phase 2 validation datasets** | | | | | | |
|---|---|---|---|---|---|---|---|---|---|
| **sensitivity OS** | **17/20** | **=0.85** | **89/102** | **=0.87** | | | | | |
| **specificity OS** | **41/67** | **=0.61** | **140/195** | **=0.72** | | | | | |
| **performance (AUC) (95%Cl) (OS)** | **0.731 (0.612-0.850)** | | **0.795 (0.742-0.849)** | | | | | | |
| **sensitivity PFS** | **16/17** | **=0.94** | **93/110** | **=0.85** | | | | | |
| **specificity PFS** | **27/35** | **=0.77** | **95/119** | **=0.80** | | | | | |
| **performance (AUC) (95%Cl) (PFS)** | **0.856 (0.748-0.964)** | | **0.822 (0.764-0.879)** | | | | | | |

### Overlap between published gene lists from prognostic neuroblastoma microarray studies

In order to demonstrate the power of our re-analysis strategy to identify common prognostic markers, we first determined the overlap of genes between published neuroblastoma (NB) micro-array prognostic gene lists.

Comparison of published prognostic gene lists from 10 different studies contained in the NBGS (NB gene server) (http://medgen.ugent.be/NBGS, Pattyn et al., in preparation) revealed only 36 genes to be found in more than 1 study, i.e. *AHCY, ARHGEF7, BAK1*, *BTBD3, CAMK2B, CAMTA1, CASP8, CASP9, CCND1, CD44, CDCA5, CDKN3, CNR1, CYB561, DBH, DDX1, GNAI1, HIST1H1C, IGF2, ITGB1*, *MAD2L1, MYCN, NME1, NRCAM, NTRK1, NXPH1, PGM2L1, PHF1, PRKACB, PRPH, RFC4, STMN2, TP53, TUBA3, UBE2C* and *WSB1* of which *MYCN, NTRK1* and *WSB1* were found in 4 lists, and *CCND1, CNR1, IGF2* and *UBE2C* in 3 lists. For a fair comparison between the gene overlap in 10 published gene lists and the 4 gene list obtained after re-analyzing raw data with updated probe annotations and clinical patient information, all 210 combinations of 4 lists from the available 10 lists were made and for each combination the percent of overlapping genes was calculated (number of genes in common divided by the total number of prognostic genes in the 4 lists together). The mean overlap was 2.9% (minimum 0.5%, maximum 5.9%).

The percentage of overlapping genes of the four generated PAM classifier gene lists of the training datasets was 8.7% (42/485) (Table 5) which is significantly higher than the mean percentage of overlapping genes in any set of 4 published gene lists (2.9%) (p=1.222E-149, z-score = 5.06).

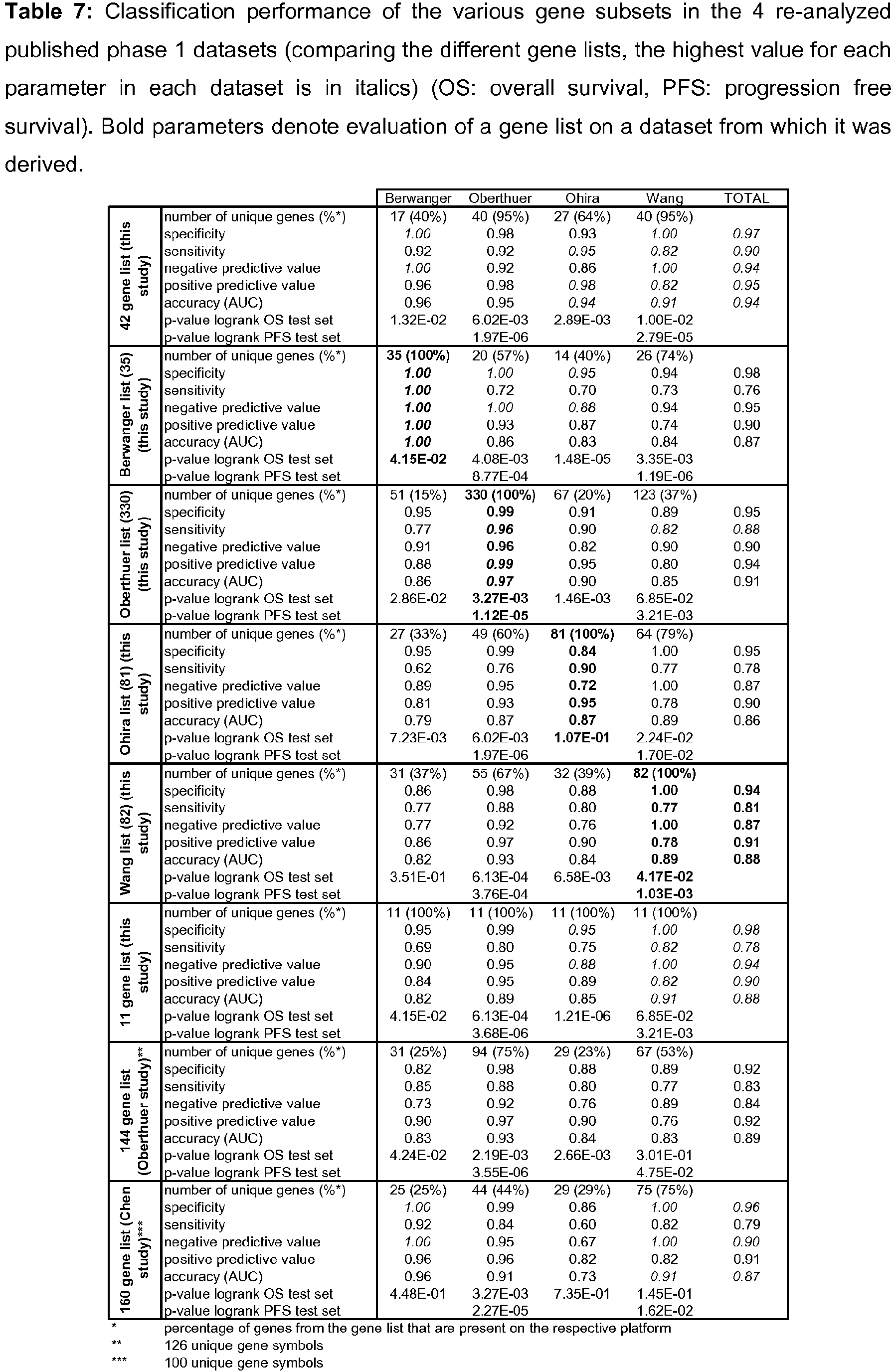

In conclusion, the inventors identified a robust set of 42 prognostic marker genes for outcome prediction in NB. The invention provides a NB prognostic signature that was shown to be significantly associated with outcome prediction in NB samples from independent studies on different technological platforms, making it a useful and practical classifier for risk stratification in NB patients. The high prognostic value despite its low number of genes makes this signature very well suited for cost-effective and fast PCR based analysis requiring only minimal amounts of tumor material in clinical settings.

### Example 2: Establishing a 59 gene classifier for Neuroblastoma

### Patients and methods

### Study population

The initial cohort comprised 343 NB patients from the Society International Oncology Pediatric European Neuroblastoma (SIOPEN) from whose primary untreated NB tumor (at least 60% tumor cells) RNA was available and of sufficient quality. Almost all of the patients (n = 324) were uniformly treated according to the SIOPEN protocols. The median follow-up was 55 months (range 1 - 143 months). Of the total group of 343 patients, 290 patients are alive

The validation cohort comprised 236 patients from the Children Oncology Group (COG)

This study was approved by the Ghent University Hospital Ethical Committee (EC2008/159).

### RNA extraction and amplification

Total RNA extraction of NB tumor samples was performed in individual laboratories by silica gel-based membrane purification methods (RNeasy Mini kit or MicroRNeasy kit, Qiagen), by phenol-based (TRIzol reagent, Invitrogen or Tri Reagent product, Sigma) or by chaotropic solution-based isolation methods (Perfect Eukaryotic RNA kit, Eppendorf) according to the manufacturer's instructions.

Starting from 20 ng of total RNA, a validated sample pre-amplification method was applied generating approximately 6 µg of cDNA to profile up to 1000 target genes (WT-Ovation, NuGEN).

### Assessment of RNA purity and integrity

In order to assess the RNA quality of the 740 collected tumor samples, we used 30 ng of each RNA isolate to perform two PCR-based assays (5'-3' mRNA integrity assay to establish a 5'-3'-delta-Ct, and a SPUD assay for the detection of enzymatic inhibitors in nucleic acid preparations [Nolan et al 1]) and a capillary gel electrophoresis analysis (high sensitivity chips, Experion, Bio-Rad) to establish a RNA quality index (RQI) based on the ribosomal RNA profile. Based on these tests, we retained 90 % of samples with acceptable quality (ROI ≥ 3 and absence of enzymatic inhibitors). Impact of RNA quality on performance will be published elsewhere [Vermeulen et al].

### High-throughput real-time quantitative PCR based gene expression

A real-time quantitative polymerase chain reaction (RT-qPCR) assay was designed for all 59 markers and 5 reference genes by PrimerDesign and went through an extensive in silico validated analysis using BLAST and BiSearch specificity, amplicon secondary structure, SNP presence and splice variant analysis. Average primer efficiency was 95% (cf. Table 2).

RT-qPCR was performed on a high throughput 384-well plate instrument (Roche LC480). PCR plates were prepared using a 96-well head pipetting robot (Sciclone ALH 3000). RT-qPCR amplifications were performed in 8 µl containing 4 µl 2X SYBR Green I master mix (Roche), 0.4 µl forward and reverse primer (5 µM each), 0.2 µl nuclease-free water and 3 µl cDNA (corresponding to 4.5 ng unamplified cDNA). The cycling conditions comprised 3 min polymerase activation at 95°C and 40 cycles of 15 sec at 95°C and 30 sec at 60°C, followed by a dissociation curve analysis from 60°C to 95°C. To detect and correct inter-run variation and allow future data comparison with different labs, we included a dilution series of absolute standards consisting of 55 bp oligo's (Biolegio, the Netherlands) run in parallel with patient samples (cf. Table 3).

For data pre-processing all samples without signal for a particular gene were set to minimum Cq of the gene across all samples. The Cq values were converted to relative quantities and converted to log₂. Relative gene expression levels were then normalised using the geometric mean of 4 reference genes and 1 repeated sequence (HPRT1, SDHA, UBC, HMBS and ALUsq). Data handling and calculations (normalization, rescaling and inter-run calibration and error propagation), were done in qBasePlus (www.qbaseplus.com)

Possible sample permutation was excluded by measuring the MYCN copy number and determining sex using the TPSY1 gene expressed on the Y chromosome, by RT-qPCR.

### Multigene correlation signature

The multigene expression signature was built using 30 training samples, tested on the remaining SIOPEN samples and validated in a blind manner using COG samples (Figure 6).

For the SIOPEN cohort, the R-language for statistical computing (version 2•6•2) was used to train and test the prognostic signature, to evaluate its performance by receiver operating characteristic (ROC) curve and area under the curve (AUC) analyses and for Kaplan-Meier survival analyses using the Bioconductor MCRestimate, the ROC and survival packages, respectively. Multivariate logistic regression analyses were performed using SPSS (version 16). Currently used risk factors such as age at diagnosis (≥12 months vs. <12 months), INSS (International Neuroblastoma Staging System) stage (stage 4 vs. not stage 4), and *MYCN* status (amplified vs. not amplified) were tested and variables with p<0•05 were retained in the model. Since an interaction between the signature and risk factors was not expected to occur, interaction terms were not included in the models. For ROC and multivariate analyses, only patients with an event and patients with sufficient follow-up time (≥36 months) were included if no event occurred since 95% of events in neuroblastoma are expected to occur within the 36 months after diagnosis.

For the validation of the signature on the COG cohort, a case-control study was set up. This was done in order to ensure a sufficient number of events in each risk group, i.e., to increase the power from what would have resulted from a random sample. A case was defined as failure (relapse, progression, or death from disease for progression-free survival (PFS), and death for overall survival (OS)) prior to two years and control as non-failure prior to two years in patients with at least two years of follow-up. Controls and cases with complete data were selected 2 to 1 to increase the sample size and power. Multivariate logistic regression analyses were performed to determine if the signature was a significant independent predictor after controlling for known risk factors. Statistical analyses were conducted in SAS (version 9).

### Results

### Establishment and testing of a 59 gene-expression signature

Based on an innovative strategy consisting of re-analysis of published microarray gene-expression studies (Oberthuer et al., J Clin Oncol. 2006 Nov 1;24(31):5070-8; Wang Q et al., Lancet 365, 671-679 (2005); Schramm et al., Oncogene 2005, 24(53):7902-7912; Berwanger et al., Cancer Cell 2002, 2(5):377-386; Ohira et al., Cancer Cell. 2005 Apr;7(4):337-50; De Preter et al., Genome Biology 2006, 7(9):R84 McArdle et al., Carcinogenesis 2004, 25(9):1599-1609) and on a review of the literature, a core set of 59 genes with prognostic power in at least 2 independent studies was selected (Table 8).

A prognostic multigene signature was subsequently built based on the expression of the 59 genes using 15 deceased high-risk and 15 low-risk patients with a long progression-free survival time. Patients with a low- or high-risk based on the expression of the 59 genes will be defined as low or high molecular risk respectively throughout the rest of the text.

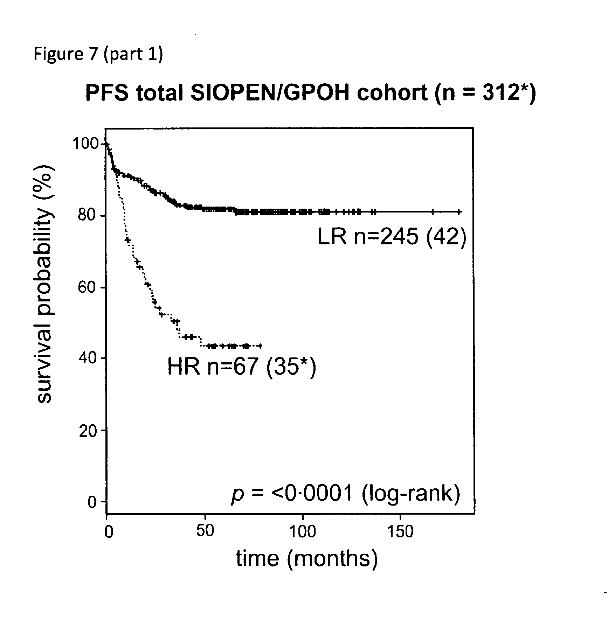

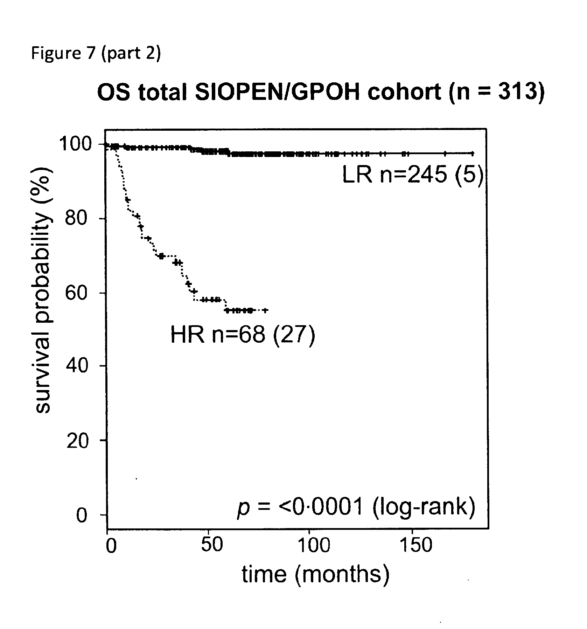

This multigene expression signature significantly distinguished the remaining 313 (missing relapse date for one high molecular risk case) patients with respect to PFS and OS (p<0•0001) (Figure 7). PFS at five years from the date of diagnosis was 81•2% (95%CI: 76•8-87•0) for the group of patients at low molecular risk compared to 43•6% (95%CI: 32•4-58•6) for the group of patients at high molecular risk. The five-year OS was 98•0% (95%CI: 96•1-100) and 55•0% (95%CI: 43•1-70•1) respectively.

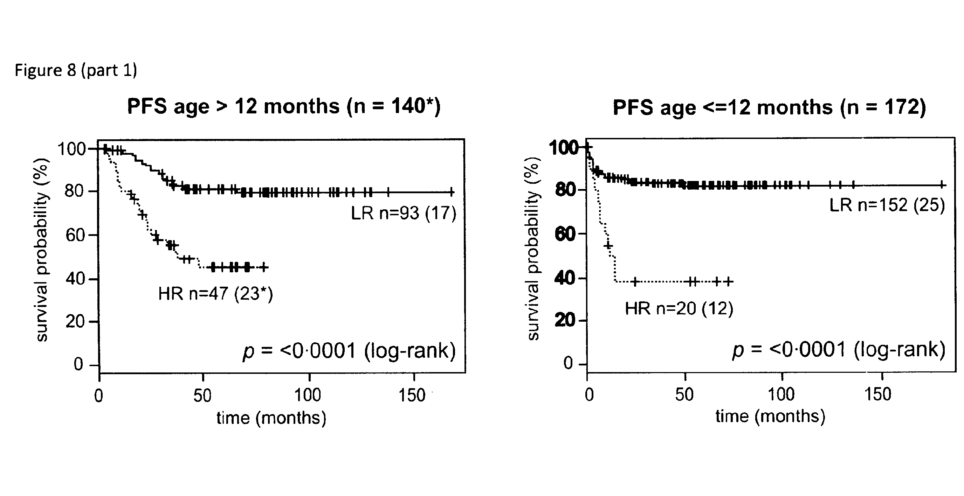

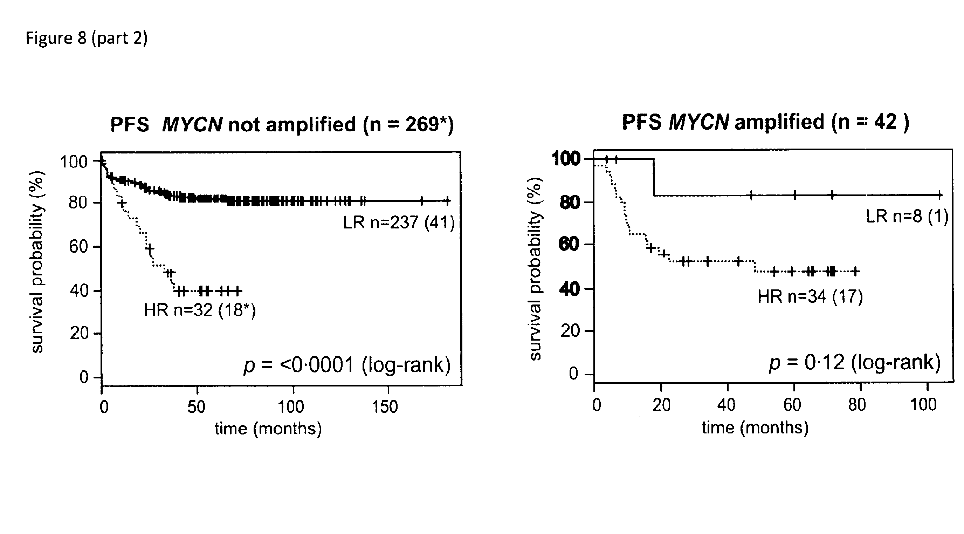

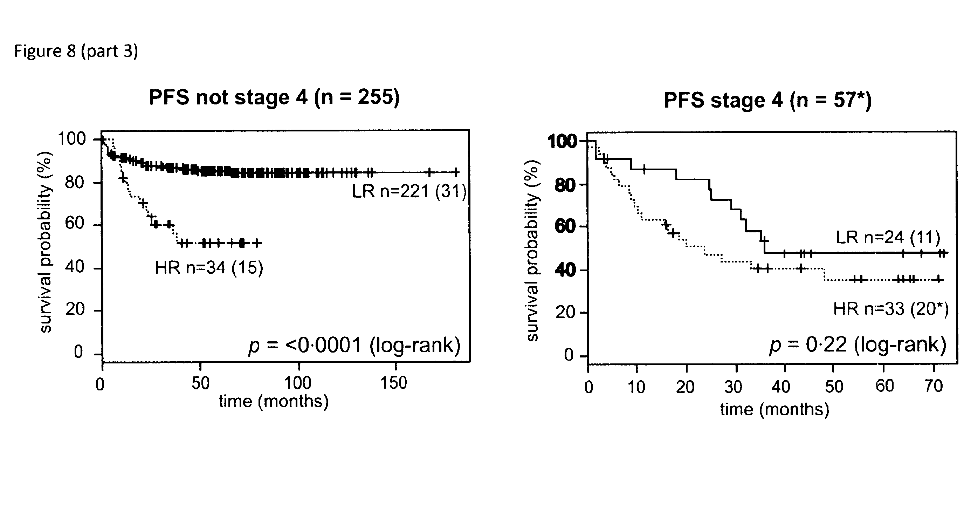

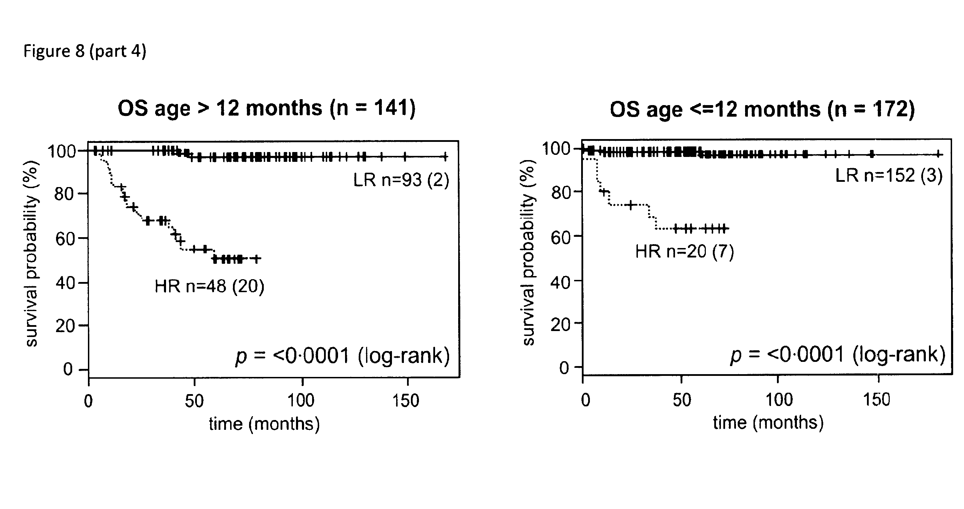

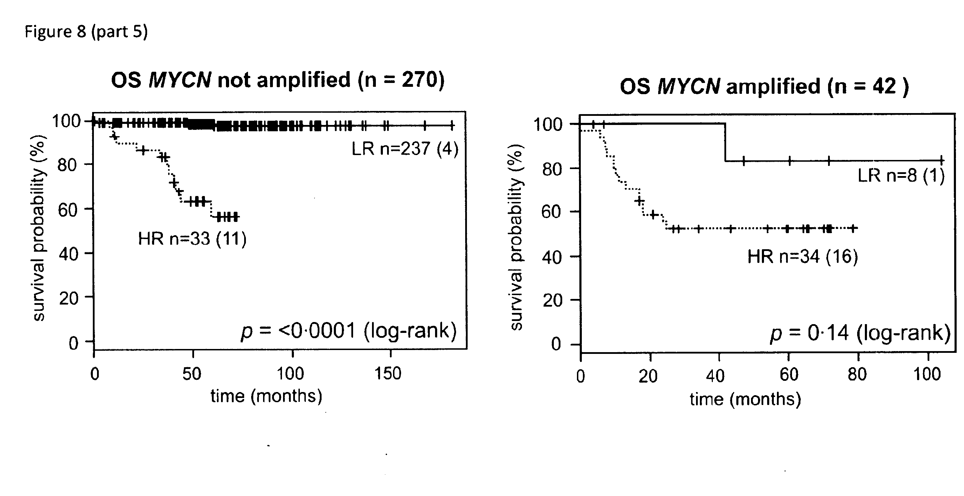

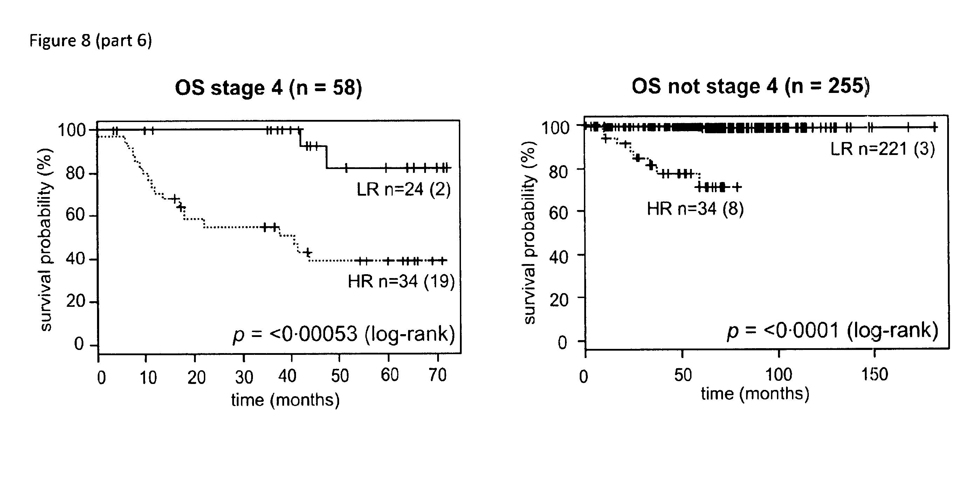

Patients with increased risk for both a shorter PFS and OS could also be identified after stratification by currently used European risk factors such as age, MYCN status, and INSS stage (Figure 8).

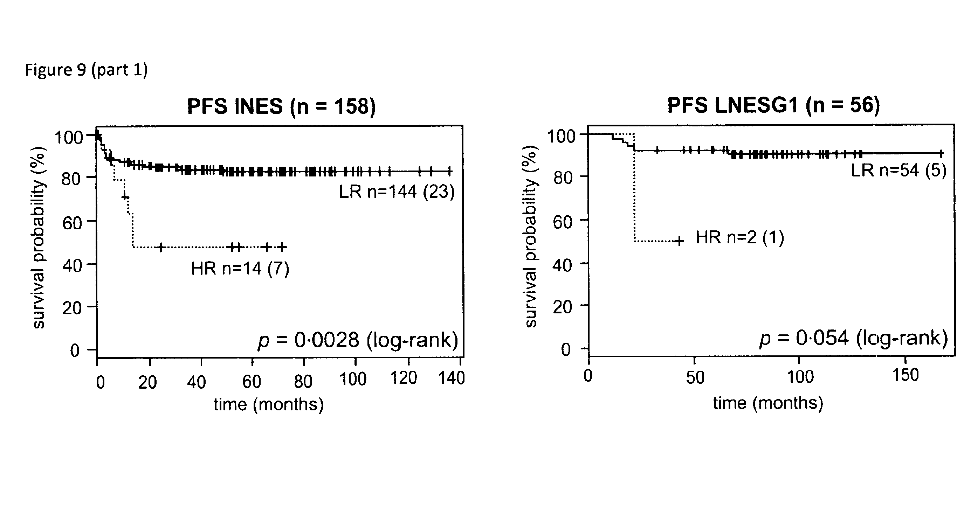

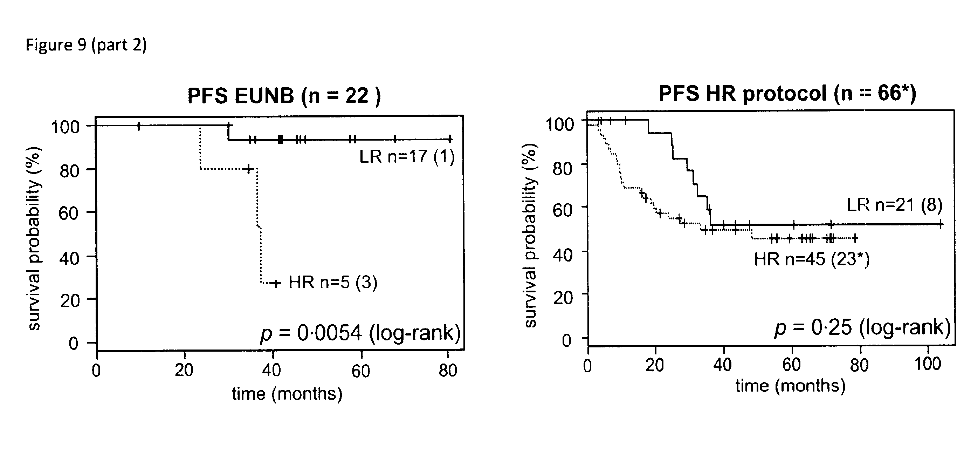

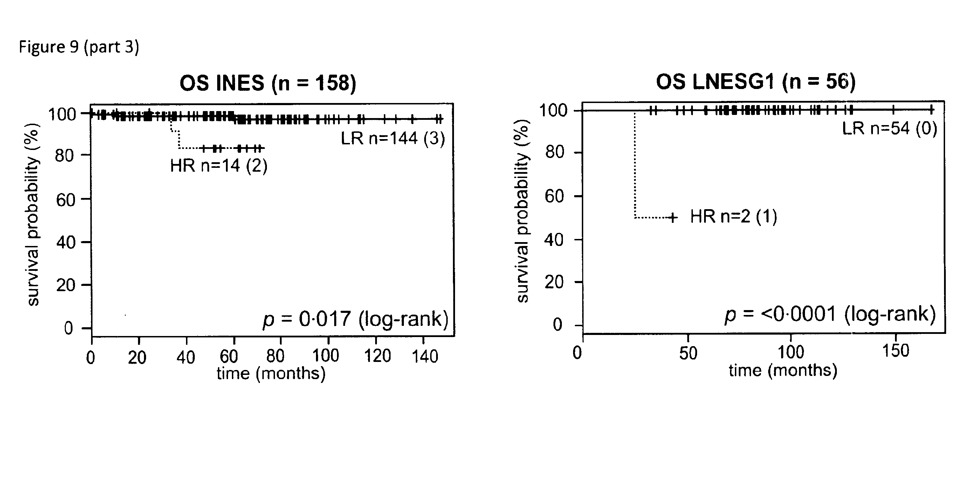

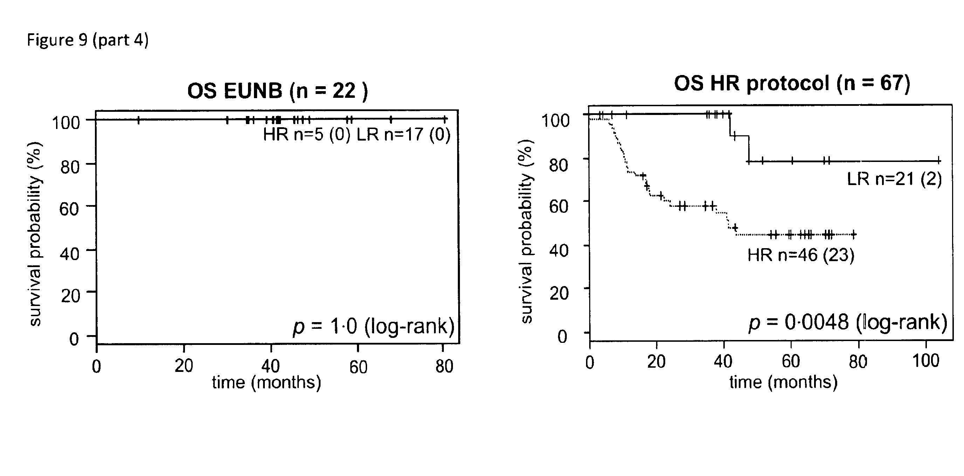

Subsequently, we tested the signature within each SIOPEN treatment protocol. In the group of patients treated according to the INES (NB99•study, NB99•1, NB99•2, and NB99•3), LNEGS1 protocols, and HR-NBL1 protocol (with inclusion of patients sharing the same high-risk features as described in Supplemental Material 5 and treated according to similar protocols) patients with increased risk for death could be identified (p=0•017, p<0•0001, and p=0•0048 respectively). While the signature was useful in identifying those patients at risk of a progression or relapse amongst patients treated according to the INES, LNESG1, and EUNB protocols (p=0•0028, p=0•054, and p=0•0054 respectively), there was no difference in PFS between patients at high and low molecular risk treated according to the HR-NBL1 protocol (Figure 9).

Multivariate logistic regression analysis of the SIOPEN patients was performed within a subset of the overall SIOPEN cohort as described in the patients and methods section. Table 9 shows that the signature and INSS stage were the only significant independent predictors (odds ratio of 19•32 (95%CI: 6•50-57•43) and 3•96 (95%CI: 1•97-7•97) for OS and PFS, in case of an adverse outcome signature). Further, within the INES and HR protocols, multivariate logistic regression analysis demonstrated that the signature was the only significant independent predictor for OS (odds ratio of 7•00 (95%CI: 1•04-46•95) and 9•20(95%CI: 1•80-47•06), respectively).

The probability that a patient will be correctly classified by the signature based on a ROC-curve analysis (AUC) was 85•4% (95%CI: 77•7-93•2) and 66•9% (95%CI: 59•2-74•6) for OS and PFS, outperforming current risk factors (age (62•3% (95%CI: 52•2-72•4) and 53•5% (95%CI: 45•8-61•2)), INSS stage (77•0% (95%CI: 66•8-87•1) and 65•4% (95%CI: 57•6-73•2)), and MYCN status (72•7% (95%CI: 61•7-83•8) and 57•2% (95%CI: 49•3-65•2))). For prediction of OS, the signature had a sensitivity of 84•4% (27/32) (95%CI: 66•5-94•1) (= the percentage of patients at high molecular risk that had an adverse outcome) and a specificity of 86•5% (192/222) (95%CI: 81•1-90•6) (= the percentage of patients at low molecular risk that had a good outcome).

### Blind validation of the multigene expression signature

In order to validate the multigene expression signature in a completely independent patient cohort, 236 COG tumours were tested in a blind manner. The same signature as used for the SIOPEN cohort identified COG patients who were at greater risk for progression, relapse, or death. Multivariate logistic regression analysis showed that the signature was independently statistically significant in a model adjusted for MYCN status, age, INSS stage, ploidy, INPC grade of differentiation, and MKI. The signature was the only independent significant predictor for PFS, with complete data for 139 controls and 70 cases. Patients at high molecular risk had a greater risk for relapse or progression (odds ratio of 3•68 (95%CI: 2•01-6•71)). In terms of OS, there were not enough deaths to power the fit of a logistic regression model with forced inclusion of all factors. Therefore, separate models testing the signature with adjustment for one risk factor at a time were fit, with complete data for 74 controls and 37 cases. In each model comparing the signature to a given risk factor, the odds ratio of the expression signature always had a higher significance (smaller P-value) than any other variable (Table 10).

**Table 8: Top ranking list of 59 prognostic markers and their correlation with good or poor prognosis.**

| **symbol** | **Accession Number/Name** | **Sequence Definition** | **chromosomal position** | **correlatio n signature vector** | **higher expresse d in HR or LR tumors** |
|---|---|---|---|---|---|
| NHLH2 | NM_005599 | nescient helix loop helix 2 mRNA. | 1p12-p11 | 6,098 | HR |
| MRPL3 | NM_007208 | mitochondrial ribosomal protein L3 (MRPL3), nuclear gene encoding mitochondrial protein, mRNA. | 3q21-q23 | 0,552 | HR |
| CDCA5 | NM_080668 | cell division cycle associated 5 (CDCA5), mRNA. | 11q12.1 | 2,046 | HR |
| ARHGEF7 | NM_145735 | Rho guanine nucleotide exchange factor (GEF) 7 (ARHGEF7), transcript variant 2, mRNA. | 13q34 | -0,466 | LR |
| ECEL1 | NM_004826 | endothelin converting enzyme-like 1 (ECEL1), mRNA. | 2q36-q37 | -4,106 | LR |
| PTPRF | NM_002840 | protein tyrosine phosphatase, receptor type, F (PTPRF), transcript variant 1, mRNA. | 1p34 | -1,38 | LR |
| PLAGL1 | NM_002656 | pleiomorphic adenoma gene-like 1 (PLAGL1), transcript variant 1, mRNA. | 6q24-q25 | -2 | LR |
| ODC1 | NM_002539 | ornithine decarboxylase 1 (ODC1), mRNA. | 2p25 | 1,986 | HR |
| DPYSL3 | NM_001387 | dihydropyrimidinase-like 3 (DPYSL3), mRNA. | 5q32 | -1,888 | LR |
| PMP22 | NM_000304 | peripheral myelin protein 22 (PMP22), transcript variant 1, mRNA. | 17p12-p11.2 | -2,274 | LR |
| CLSTN1 | NM_001009566 | calsyntenin 1 (CLSTN1), transcript variant 1, mRNA. | 1 p36.22 | -2,634 | LR |
| NRCAM | NM_001037132 | neuronal cell adhesion molecule (NRCAM), transcript variant 1, mRNA. | 7q31.1-q31.2 | -2,072 | LR |
| MTSS1 | NM_014751 | metastasis suppressor 1 (MTSS1), mRNA. | 8p22 | -1,258 | LR |
| CAMTA2 | NM_015099 | calmodulin binding transcription activator 2 (CAMTA2), mRNA. | 17p13.2 | -1,232 | LR |
| PTN | NM_002825 | pleiotrophin (heparin binding growth factor 8, neurite growth-promoting factor 1) (PTN), mRNA. | 7q33-q34 | -1,566 | LR |
| SNAPC1 | NM_003082 | small nuclear RNA activating complex, polypeptide 1, 43kDa (SNAPC1), mRNA. | 14q22 | 0,248 | HR |
| QPCT | NM_012413 | glutaminyl-peptide cyclotransferase (glutaminyl cyclase) (QPCT), mRNA. | 2p22.2 | -3,518 | LR |
| EPB41L3 | NM_012307 | erythrocyte membrane protein band 4.1-like 3 (EPB41L3), mRNA. | 18p11.32 | -2,64 | LR |
| GNB1 | NM_002074 | guanine nucleotide binding protein (G protein), beta polypeptide 1 (GNB1), mRNA. | 1p36.33 | -0,772 | LR |
| PTPRH | NM_002842 | protein tyrosine phosphatase, receptor type, H (PTPRH), mRNA. | 19q13.4 | -4,938 | LR |
| MAPT | NM_016835 | microtubule-associated protein tau (MAPT), transcript variant 1, mRNA. | 17q21.1 | -2,036 | LR |
| SLC25A5 | NM_001152 | solute carrier family 25 (mitochondrial carrier; adenine nucleotide translocator), member 5 (SLC25A5), mRNA. | Xq24-q26 | 1,374 | HR |
| BIRC5 | NM_001168 | baculoviral IAP repeat-containing 5 (survivin) (BIRC5), transcript variant 1, mRNA. | 17q25 | 2,428 | HR |
| AHCY | NM_000687 | S-adenosyl homocysteine hydrolase (AHCY), mRNA. | 20cen-q 13.1 | 1,8 | HR |
| MAP7 | NM_003980 | microtubule-associated protein 7 (MAP7), mRNA. | 6q23.3 | -2,232 | LR |
| ULK2 | NM_014683 | unc-51-like kinase 2 (C. elegans) (ULK2), mRNA. | 17p11.2 | -0,82 | LR |
| PTPRN2 | NM_002847 | protein tyrosine phosphatase, receptor type, N polypeptide 2 (PTPRN2), transcript variant 1, mRNA. | 7q36 | -2,876 | LR |
| INPP1 | NM_002194 | inositol polyphosphate-1-phosphatase (INPP1), mRNA. | 6q22-q23 | -1,384 | LR |
| HIVEP2 | NM_006734 | human immunodeficiency virus type I enhancer binding protein 2 (HIVEP2), mRNA. | 6q23-q24 | -1,796 | LR |
| EPN2 | NM_014964 | epsin 2 (EPN2), transcript variant 2, mRNA. | 17p11.2 | -2,448 | LR |
| EPHA5 | NM_004439 | EPH receptor A5 (EPHA5), transcript variant 1, mRNA. | 4q13.1 | -5,752 | LR |
| CHD5 | NM_015557 | chromodomain helicase DNA binding protein 5 (CHD5), mRNA. | 1 p36.31 | -7,766 | LR |
| PDE4DIP | NM_014644 | phosphodiesterase 4D interacting protein (myomegalin) (PDE4DIP), transcript variant 1, mRNA. | 1q12 | -1,654 | LR |
| TYMS | NM_001071 | thymidylate synthetase (TYMS), mRNA. | 18p11.32 | 1,116 | HR |
| MAP2K4 | NM_003010 | mitogen-activated protein kinase kinase 4 (MAP2K4), mRNA. | 17p11.2 | -0,56 | LR |
| CPSG3 | NM_004386 | chondroitin sulfate proteoglycan 3 (neurocan) (CSPG3)(NCAN), mRNA. | 19p12 | 4,704 | HR |
| MCM2 | NM_004526 | MCM2 minichromosome maintenance deficient 2, mitotin (S. cerevisiae) (MCM2), mRNA. | 3q21 | 1,874 | HR |
| PIK3R1 | NM_181523 | phosphoinositide-3-kinase, regulatory subunit 1 (p85 alpha) (PIK3R1), transcript variant 1, mRNA. | 5q13.1 | -1,828 | LR |
| PRDM2 | NM_012231 | PR domain containing 2, with ZNF domain (PRDM2), transcript variant 1, mRNA. | 1 p36.21 | 0,078 | LR |
| SLC6A8 | NM_005629 | solute carrier family 6 (neurotransmitter transporter, creatine), member 8 (SLC6A8), mRNA. | Xq28 | 0,308 | HR |
| NME1 | NM_198175 | non-metastatic cells 1, protein (NMA(23); NM23A) expressed in (NME1), transcript variant 1, mRNA. | 17q21.3 | 1,298 | HR |
| TNFRSF2 5 | NM_148965 | tumor necrosis factor receptor superfamily, member 25 (TNFRSF25), transcript variant 1, mRNA. | 1 p36.2 | 0,178 | LR |
| PRKCZ | NM_002744 | protein kinase C, zeta (PRKCZ), transcript variant 1, mRNA. | 1 p36.33-p36.2 | -1,284 | LR |
| CAMTA1 | NM_015215 | calmodulin binding transcription activator 1 (CAMTA1), mRNA. | 1p36.31-p36.23 | -2,164 | LR |
| AKR1C1 | NM_001353 | aldo-keto reductase family 1, member C1 (dihydrodiol dehydrogenase 1; 20-alpha (3-alpha)-hydroxysteroid dehydrogenase) (AKR1C1), mRNA. | 10p15-p14 | -3,994 | LR |
| ELAVL4 | NM_021952 | Embryonic lethal, abnormal vision, Drosophila)-like 4 (Hu antigen D) | 1p34 | -1,29 | LR |
| NTRK1 | NM_001012331 | Neurotrophic tyrosine kinase receptor type 1 | 1q21-q22 | -7,79 | LR |
| PRAME | NM_006115 | preferentially expressed antigen in melanoma | 22q 11.22 | 5,57 | HR |
| WSB1 | NM_134265 | WD repeat and SOCS box-containing 1 | 17q11.1 | 0,194 | LR |
| DDC | NM_000790 | dopa decarboxylase (aromatic L-amino acid decarboxylase) | 7p11 | -3,462 | LR |
| CADM1 | NM_014333 | cell adhesion molecule 1 (IGSF4) | 11q23.2 | -0,414 | LR |

| **symbol** | **Accession Number/Name** | **Sequence Definition** | **chromosoma l position** | **correlation signature vector** | **higher expresse d in HR or LR tumors** |
|---|---|---|---|---|---|
| MYCN | NM_005378 | v-myc myelocytomatosis viral related oncogene, neuroblastoma derived | 2p24.1 | 3,91 | HR |
| PLAT | NM_033011 | plasminogen activator, tissue | 8p12 | -2,06 | LR |
| CD44 | NM_001001392 | CD44 molecule (Indian blood group) | 11p13 | -1,952 | LR |
| CDKN3 | NM_005192 | cyclin-dependent kinase inhibitor 3 | 14q22 | 1,378 | HR |
| FYN | NM_153048 | proto-oncogene tyrosine-protein kinase fyn | 6q21 | -1,078 | LR |
| PAICS | NM_001079525. 1 | phosphoribosylaminoimidazol e carboxylase, phosphoribosylaminoimidazol e succinocarboxamide synthetase | 4q12 | 1,458 | HR |
| PRKACB | NM_182948 | protein kinase, cAMP-dependent, catalytic, beta | 1p36.1 | -1,098 | LR |
| SCG2 | NM_003469 | secretogranin II (chromogranin C) | 2q35-q36 | -2,634 | LR |

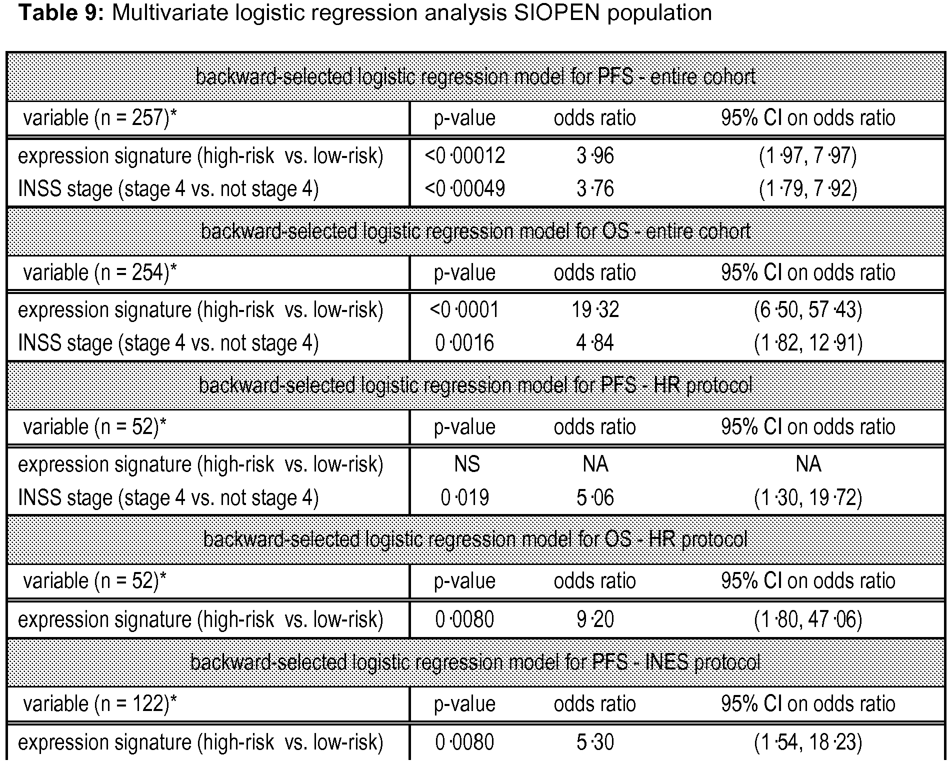

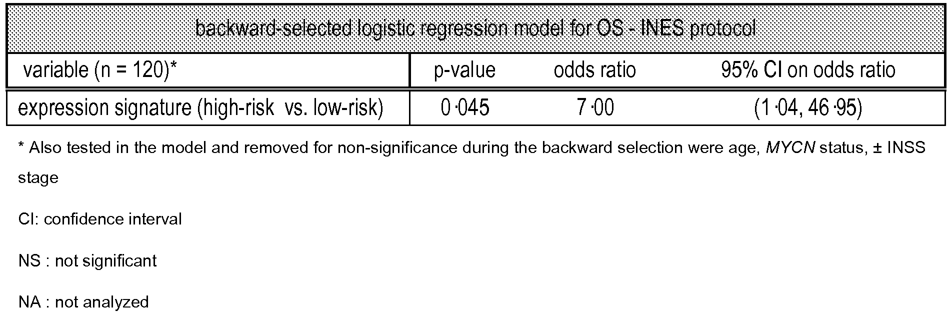

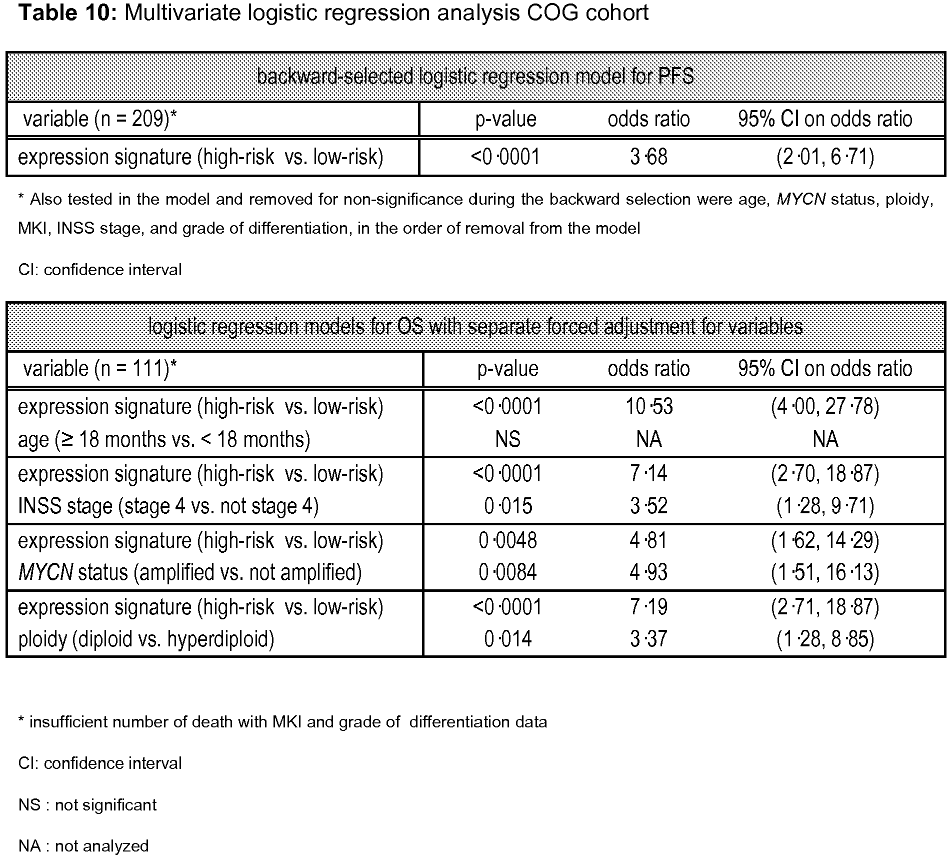

In conclusion, using a fast and sensitive qPCR technology, a robust prognostic multigene expression signature was established and validated which is an independent risk predictor, able to identify patients with increased risk in the current risk groups. This study might form the basis for future research, i.e. large well-defined prospective studies with international collaboration

### Example 3: Reduction of the gene list

In the complete 59 gene set, 12 genes were identified that had not previously been linked to neuroblastoma prognosis at all. The predictive power of the group of 12 genes was also tested (cf. Table 1, gene set "12") and shown to have good prognostic power, but performs inferior to the best subsets (i.e. the 59 and 42 gene lists)

As indicated above already, the inventors identified also a 42 genes list and the genes overlapping in this 42 gene list and the 12 gene list, ie a 6 gene list was also evaluated for their predictive power. The performance of this smallest gene list of the invention is still reasonably good especially in view of its size, but does not perform as well as the longer lists such as the 12 gene list (cf. Table 1A, gene list "6").

### Example 4: Establishing an miRNA classifier

We have developed a molecular signature to predict overall or progression free survival in patients with neuroblastoma (NB). This signature involves 25 miRNAs of which the whole list or subsets can be used in a signature that can accurately predict neuroblastoma patient outcome (progression, relapse as well as overall survival).

In a first step, 430 different human mature miRNAs were profiled on 268 primary neuroblastoma tumour samples. In brief, mature miRNAs were reverse transcribed using the megaplex stem-loop reverse transcription method in combination with a limited cycle pre-amplification. The evaluation of this procedure was performed at our lab and was published recently in Nucleic Acids Research (see Mestdagh P et al., Nucleic Acid Research 2008, 1-8). Subsequent miRNA expression profiling was performed using quantitative PCR with miRNA specific Taqman probes. RT-qPCR data was normalized using mean normalisation (see Mestdagh P et al., Genome Biology 2009). Logistic regression analysis of miRNA expression on 30 high risk and 30 low risk patients (randomly selected from the group of 268 patients) was used to select the top 25 miRNAs with highest correlation to overall patient survival.

The performance of a 25 miRNA PAM classifier for the prognostic classification of the remaining 208 samples is summarised in Table 2B and Figure 10. In addition we tested the performance of a classifier based on a subselection of 12 genes, as well as the remaining 13 genes. 7 miRNAs of the 25 miRNA set were identified as not being previously linked to neuroblastoma prognosis at all. The predictive power of this list as well as the 5 miRNAs of the 7 miRNA list that overlap with the 12 gene list and the 2 miRNAs of the 7 miRNA list that overlap with the 13 gene list is summarised in the Table. Overall these analyses show that the 25 miRNA list performs best and that all other lists perform relatively good.

### Example 5: Establishing a combined classifier containing both mRNAs and miRNAs

The mRNA and miRNA prognostic gene lists can be used in isolation or in combination with each other and/or in combination with the mRNA classifiers. In such a combination, one can for example use the 6, 12, 42 or 59 gene set in combination with any one of miRNA sets of the present invention..

For 178 samples, mRNA expression profiling data (on the Affymetrix platforms) as well as miRNA expression profiling data (stem-loop RT-qPCR platform) were available. mRNA expression data of 56 of the claimed 59 mRNA list and all of the 25 miRNA list were present in the profiles. In Table 1C performance results are shown for a PAM classifier combining the expression data of the 59 mRNA list and the 25 miRNA list, showing that comparable results could be obtained in comparison to the individual classifiers. For some parameters (for example log-rank analysis within the high risk subgroup) the performance of the combined classifier is better than the individual parameters.

## Claims

1. A kit for prognostic stratification of neuroblastoma patients consisting of:
a. oligonucleotides or primer pairs which are specific for each of the following 42 mRNAs: CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2 and WSB1; or
b. oligonucleotides or primer pairs which are specific for each of the following 59 mRNAs: CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2, WSB1 CAMTA1, CD44, CHD5, ELAVL4, MAP2K4, MTSS1, NHLH2, PDE4DIP, PIK3R1, PLAT, PRAME, PRDM2, PTPRF, PTPRH, QPCT, SLC6A8 and TNFRSF25; or
c. oligonucleotides or primer pairs which are specific for each of the following 12 miRNAs: hsa-mir-345, hsa-mir-320, hsa-mir-485-5p, hsa-mir-542-3p, hsa-mir-192, hsa-mir-25, hsa-mir-15b, hsa-mir-326, hsa-mir-93, hsa-mir-572, hsa-mir-17-5p and hsa-mir-20b; or
d. oligonucleotides or primer pairs which are specific for each miRNA of the following 25 miRNAs: hsa-mir-628, hsa-mir-345, hsa-mir-500, hsa-mir-320, hsa-mir-485-5p, hsa-mir-542-3p, hsa-mir-192, hsa-mir-92, hsa-mir-26a, hsa-mir-30c, hsa-mir-190, hsa-mir-204, hsa-mir-488, hsa-mir-125b, hsa-mir-18a^{*}, hsa-mir-20a, hsa-mir-19a, hsa-mir-18a, hsa-mir-25, hsa-mir-15b, hsa-mir-326, hsa-mir-93, hsa-mir-572, hsa-mir-17-5p and hsa-mir-20b; or
e. a combination of said oligonucleotides which are specific for each of said 42 mRNAs or 59 mRNAs with said oligonucleotides which are specific for each of said 12 miRNAs or 25 miRNAs.

2. The kit according to claim 1, wherein said oligonucleotides are for use in hybridization-based analysis, microarray, digital gene expression (DGE), RNA-in-situ hybridization (RISH), or Northern-blot analysis.

3. The kit according to claim 1, wherein said primer pairs are for use in PCR, RT-PCR, RT-qPCR, end-point PCR, or digital PCR.

4. A method for prognostic stratification of neuroblastoma patients comprising the steps of:
a)analyzing the expression level of the genes and/or miRNAs which are part of the kits according to any of claims 1-3 in a sample obtained from a patient,
b) comparing the expression level obtained in step a) with the expression level as established for neuroblastoma tumors with poor prognosis and with the expression level as established for neuroblastoma tumors with good prognosis, and
c) determining whether the expression level(s) as determined in step a) correlate(s) with the expression levels in neuroblastoma tumors with either good or poor prognosis, thereby stratifying the patient into the group of good prognosis or poor prognosis.

5. The method according to claim 4, wherein the sample is selected from the group comprising: tumor tissue, bone-marrow, bodily fluids, blood, serum, plasma, cerebrospinal fluid, peritoneal fluid and intraperitoneal fluid.

6. The method according to any one of claims 4-5, wherein the expression analysis is performed using any one of the technologies selected from the group comprising:
Polymerase Chain Reaction (PCR), Real-Time quantitative PCR (RT-qPCR), End-Point PCR, digital PCR (dPCR), RNA or cDNA hybridization techniques, microarrays, RNA-in-situ hybridization (RISH), Northern-Blotting, digital gene expression (DGE), sequence-analysis based expression analysis, Supported oligonucleotide detection, Pyrosequencing, Polony Cyclic Sequencing by Synthesis, Simultaneous Bi-directional Sequencing, Single-molecule sequencing, Single molecule real time sequencing, True Single Molecule Sequencing, Hybridization-Assisted Nanopore Sequencing or Sequencing by synthesis.

7. The method according to any one of claims 4-6, wherein the patient was previously diagnosed as being a high risk patient, an intermediate patient, a low risk patient or was not classified previously.

## Patentansprüche

1. Kit zur prognostischen Stratifizierung von Neuroblastom-Patienten, bestehend aus:
a. Oligonukleotiden oder Primer-Paaren, die für jede der folgenden 42 mRNAs spezifisch sind: CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2 und WSB1; oder
b. Oligonukleotiden oder Primer-Paaren, die für jede der folgenden 59 mRNAs spezifisch sind: CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2, WSB1, CAMTA1, CD44, CHD5, ELAVL4, MAP2K4, MTSS1, NHLH2, PDE4DIP, PIK3R1, PLAT, PRAME, PRDM2, PTPRF, PTPRH, QPCT, SLC6A8 und TNFRSF25; oder
c. Oligonukleotiden oder Primer-Paaren, die für jede der folgenden 12 miRNAs spezifisch sind: hsa-mir-345, hsa-mir-320, hsa-mir-485-5p, hsa-mir-542-3p, hsa-mir-192, hsa-mir-25, hsa-mir-15b, hsa-mir-326, hsa-mir-93, hsa-mir-572, hsa-mir-17-5p und hsa-mir-20b; oder
d. Oligonukleotiden oder Primer-Paaren, die für jede miRNA der folgenden 25 miRNAs spezifisch sind: hsa-mir-628, hsa-mir-345, hsa-mir-500, hsa-mir-320, hsa-mir-485-5p, hsa-mir-542-3p, hsa-mir-192, hsa-mir-92, hsa-mir-26a, hsa-mir-30c, hsa-mir-190, hsa-mir-204, hsa-mir-488, hsa-mir-125b, hsa-mir-18a*, hsa-mir-20a, hsa-mir-19a, hsa-mir-18a, hsa-mir-25, hsa-mir-15b, hsa-mir-326, hsa-mir-93, hsa-mir-572, hsa-mir-17-5p und hsa-mir-20b; oder
e. einer Kombination der Oligonukleotide, die für jede der 42 mRNAs oder 59 mRNAs spezifisch sind, mit den Oligonukleotiden, die für jede der 12 miRNAs oder 25 miRNAs spezifisch sind.

2. Kit nach Anspruch 1, wobei die Oligonukleotide zur Verwendung bei der Analyse auf Hybridisierungsbasis, im Mikroarray, bei der digitalen Genexpression (DGE), RNA-In-situ-Hybridisierung (RISH) oder Northern-Blot-Analyse vorgesehen sind.

3. Kit nach Anspruch 1, wobei die Primer-Paare zur Verwendung bei PCR, RT-PCR, RT-qPCR, Endpunkt-PCR oder digitaler PCR vorgesehen sind.

4. Verfahren zur prognostischen Stratifizierung von Neuroblastom-Patienten, umfassend die Schritte:
a) Analysieren des Expressionsniveaus der Gene und/oder miRNAs, die Teil der Kits nach einem der Ansprüche 1-3 sind, in einer von einem Patienten erhaltenen Probe,
b) Vergleichen des in Schritt a) erhaltenen Expressionsniveaus mit dem Expressionsniveau, wie es für Neuroblastomtumoren mit schlechter Prognose ermittelt wurde, und mit dem Expressionsniveau, wie es für Neuroblastomtumoren mit guter Prognose ermittelt wurde, und
c) Bestimmen, ob das bzw. die wie in Schritt a) bestimmte(n) Expressionsniveau(s) mit den Expressionsniveaus in Neuroblastomtumoren mit entweder guter oder schlechter Prognose korreliert bzw. korrelieren, und damit Stratifizieren des Patienten in die Gruppe mit guter Prognose oder schlechter Prognose.

5. Verfahren nach Anspruch 4, wobei die Probe aus der Tumorgewebe, Knochenmark, Körperflüssigkeiten, Blut, Serum, Plasma, Liquor, Peritonealflüssigkeit und Intraperitonealflüssigkeit umfassenden Gruppe ausgewählt ist.

6. Verfahren nach einem der Ansprüche 4-5, wobei die Expressionsanalyse unter Verwendung einer der aus der Folgendes umfassenden Gruppe aus-gewählten Technologien durchgeführt wird: PCR (Polymerase Chain Reaction), RT-qPCR (Real-Time quantitative PCR), End-Point-PCR, digitale PCR (dPCR), RNA- oder cDNA-Hybridisierungstechniken, Mikroarrays, RNA-In-situ-Hybridisierung (RISH), Northern-Blotting, digitale Genexpression (DGE), Expressionsanalyse auf Sequenzanalysebasis, geträgerten Oligonukleotidnachweis, Pyrosequencing, Polony Cyclic Sequencing by Synthesis, Simultaneous Bi-directional Sequencing, Einzelmolekül-Sequenzierung, Einzelmolekül-Echtzeit-Sequenzierung, True Single Molecule Sequencing, Hybridization-Assisted Nanopore Sequencing oder Sequenzierung mittels Synthese.

7. Verfahren nach einem der Ansprüche 4-6, wobei der Patient zuvor als Patient mit hohem Risiko, Durchschnittspatient, Patient mit geringem Risiko diagnostiziert wurde oder nicht zuvor klassifiziert wurde.

## Revendications

1. Trousse pour la stratification pronostique de patients présentant un neuroblastome, consistant en :
a. les oligonucléotides ou paires d'amorces qui sont spécifiques de chacun des 42 ARNm suivants : CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2 et WSB1 ; ou
b. les oligonucléotides ou paires d'amorces qui sont spécifiques de chacun des 59 ARNm suivants : CAMTA2, EPHA5, EPN2, PLAGL1, PTPRN2, SNAPC1, AHCY, AKR1C1, ARHGEF7, BIRC5, CADM1, CDCA5, CDKN3, CLSTN1, CPSG3, DDC, DPYSL3, ECEL1, EPB41L3, FYN, GNB1, HIVEP2, INPP1, MAP7, MAPT, MCM2, MRPL3, MYCN, NME1, NRCAM, NTRK1, ODC1, PAICS, PMP22, PRKACB, PRKCZ, PTN, SCG2, SLC25A5, TYMS, ULK2, WSB1, CAMTA1, CD44, CHD5, ELAVL4, MAP2K4, MTSS1, NHLH2, PDE4DIP, PIK3R1, PLAT, PRAME, PRDM2, PTPRF, PTPRH, QPCT, SLC6A8 et TNFRSF25 ; ou
c. les oligonucléotides ou paires d'amorces qui sont spécifiques de chacun des 12 miARN suivants : hsa-mir-345, hsa-mir-320, hsa-mir-485-5p, hsa-mir-542-3p, hsa-mir-192, hsa-mir-25, hsa-mir-15b, hsa-mir-326, hsa-mir-93, hsa-mir-572, hsa-mir-17-5p et hsa-mir-20b ; ou
d. les oligonucléotides ou paires d'amorces qui sont spécifiques de chaque miARN des 25 miARN suivants : hsa-mir-628, hsa-mir-345, hsa-mir-500, hsa-mir-320, hsa-mir-485-5p, hsa-mir-542-3p, hsa-mir-192, hsa-mir-92, hsa-mir-26a, hsa-mir-30c, hsa-mir-190, hsa-mir-204, hsa-mir-488, hsa-mir-125b, hsa-mir-18a*, hsa-mir-20a, hsa-mir-19a, hsa-mir-18a, hsa-mir-25, hsa-mir-15b, hsa-mir-326, hsa-mir-93, hsa-mir-572, hsa-mir-17-5p et hsa-mir-20b ; ou
e. une combinaison desdits oligonucléotides qui sont spécifiques de chacun desdits 42 ARNm ou desdits 59 ARNm, avec lesdits nucléotides qui sont spécifiques de chacun desdits 12 miARN ou desdits 25 miARN.

2. Trousse selon la revendication 1, dans laquelle lesdits oligonucléotides sont destinés à une utilisation dans une analyse par hybridation, un microréseau, une quantification de l'expression génique (DGE), une hybridation in situ de l'ARN (RISH) ou une analyse par transfert de Northern.

3. Trousse selon la revendication 1, dans laquelle lesdites paires d'amorces sont destinées à une utilisation en PCR, en RT-PCR, en RT-qPCR, en PCR en point final ou en PCR numérique.

4. Procédé pour la stratification pronostique de patients présentant un neuroblastome, comprenant les étapes de :
a) analyse du niveau d'expression des gènes et/ou des miARN qui font partie des trousses selon l'une quelconque des revendication 1 à 3 dans un échantillon obtenu d'un patient,
b) comparaison du niveau d'expression obtenu dans l'étape a) au niveau d'expression tel qu'établi pour des tumeurs neuroblastiques à pronostic médiocre, et au niveau d'expression tel qu'établi pour des tumeurs neuroblastiques à bon pronostic, et
c) détermination si le ou les niveaux d'expression tels que déterminés dans l'étape a) présentent une corrélation avec les niveaux d'expression dans des tumeurs neuroblastiques ayant un pronostic bon ou médiocre, en stratifiant de ce fait le patient dans le groupe des bons pronostics ou des pronostics médiocres.

5. Procédé selon la revendication 4, dans lequel l'échantillon est choisi dans le groupe comprenant un tissu tumoral, la moelle osseuse, les fluides corporels, le sang, le sérum, le plasma, le liquide céphalorachidien, le liquide péritonéal et le liquide intrapéritonéal.

6. Procédé selon l'une quelconque des revendications 4-5, dans lequel l'analyse de l'expression est réalisée par utilisation de l'une quelconque des technologies choisies dans le groupe consistant en la réaction en chaîne par polymérase (PCR), la PCR quantitative en temps réel (RT-qPCR), la PCR en point final, la PCR numérique (dPCR), les techniques d'hybridation de l'ARN ou de l'ADNc, les microréseaux, l'hybridation in situ de l'ARN (RISH), le transfert de Northern, la quantification de l'expression génique (DGE), l'analyse de l'expression par analyse des séquences, la détection des oligonucléotides supportés, le pyroséquençage, le séquençage cyclique des polonies par synthèse, le séquençage bidirectionnel simultané, le séquençage sur molécule unique, le séquençage en temps réel sur molécule unique, le séquençage sur molécule unique vrai, le séquençage par nanopore assisté par hybridation, ou le séquençage par synthèse.

7. Procédé selon l'une quelconque des revendications 4-6, dans lequel le patient a été préalablement diagnostiqué comme étant un patient à risque élevé, un patient intermédiaire, un patient à faible risque, ou n'a pas été préalablement classifié.


### SEQUENCE LISTING

<110> Ghent University
<120> NEUROBLASTOMA PROGNOSTIC MULTIGENE EXPRESSION SIGNATURE
<130> UG-025-PCT
<150> EP 08171163.2
   <151> 2008-12-10
<160> 192
<170> PatentIn version 3.3
<210> 1
   <211> 21
   <212> DNA
   <213> Artificial
<220>
   <223> primer
<400> 1
   tcagcaacac tagcacttca c 21
<210> 2
   <211> 25
   <212> DNA
   <213> Artificial
<220>
   <223> primer
<400> 2
   aaggacttct cagacataac tacag 25
<210> 3
   <211> 25
   <212> DNA
   <213> Artificial
<220>
   <223> primer
<400> 3
   gaactgccag aagatttgta tgatg 25
<210> 4
   <211> 25
   <212> DNA
   <213> Artificial
<220>
   <223> primer
<400> 4
   gtccaaagat gttaggcaaa tgtaa 25
<210> 5
   <211> 24
   <212> DNA
   <213> Artificial
<220>
   <223> primer
<400> 5
   ttgtcaccca tacccatttc ttac 24
<210> 6
   <211> 24
   <212> DNA
   <213> Artificial
<220>
   <223> primer
<400> 6
   ctgggactct tcaactttct cttc 24
<210> 7
   <211> 21
   <212> DNA
   <213> Artificial
<220>
   <223> primer
<400> 7
   ggagcaccta cagaagcaaa c 21
<210> 8
   <211> 23
   <212> DNA
   <213> Artificial
<220>
   <223> primer
<400> 8
   ggtatgagat ggcactgaat gag 23
<210> 9
   <211> 21
   <212> DNA
   <213> Artificial
<220>
   <223> primer
<400> 9
   ctgcaggtgc tgactgacaa g 21
<210> 10
   <211> 18
   <212> DNA
   <213> Artificial
<220>
   <223> primer
<400> 10
   gaaagcccgg ccaaactc 18
<210> 11
   <211> 22
   <212> DNA
   <213> Artificial
<220>
   <223> primer
<400> 11
   tcggagcctg taacctacta tg 22
<210> 12
   <211> 20
   <212> DNA
   <213> Artificial
<220>
   <223> primer
<400> 12
   cacaccatcc acctcctgaa 20
<210> 13
   <211> 23
   <212> DNA
   <213> Artificial
<220>
   <223> primer
<400> 13
   ggctaaggga aatgctggta aag 23
<210> 14
   <211> 23
   <212> DNA
   <213> Artificial
<220>
   <223> primer
<400> 14
   gaggcaggta ttgttaggtt cac 23
<210> 15
   <211> 22
   <212> DNA
   <213> Artificial
<220>
   <223> primer
<400> 15
   aagagatcac cggcgtaatc aa 22
<210> 16
   <211> 21
   <212> DNA
   <213> Artificial
<220>
   <223> primer
<400> 16
   cgggctcagc tatgattctc a 21
<210> 17
   <211> 23
   <212> DNA
   <213> Artificial
<220>
   <223> primer
<400> 17
   cagccagcat tcattgtaag ttc 23
<210> 18
   <211> 21
   <212> DNA
   <213> Artificial
<220>
   <223> primer
<400> 18
   ccatacccac cagacacaga a 21
<210> 19
   <211> 21
   <212> DNA
   <213> Artificial
<220>
   <223> primer
<400> 19
   tactcctacg gtttcgccta c 21
<210> 20
   <211> 22
   <212> DNA
   <213> Artificial
<220>
   <223> primer
<400> 20
   acatagatga caccgctgag aa 22
<210> 21
   <211> 20
   <212> DNA
   <213> Artificial
<220>
   <223> primer
<400> 21
   gatggactgg gacgactctg 20
<210> 22
   <211> 20
   <212> DNA
   <213> Artificial
<220>
   <223> primer
<400> 22
   ctgctgtgct ggtcctcata 20
<210> 23
   <211> 25
   <212> DNA
   <213> Artificial
<220>
   <223> primer
<400> 23
   tagtgtttaa gaaagcaagc aagtc 25
<210> 24
   <211> 21
   <212> DNA
   <213> Artificial
<220>
   <223> primer
<400> 24
   gagggttcgg tcagaaatgt g 21
<210> 25
   <211> 21
   <212> DNA
   <213> Artificial
<220>
   <223> primer
<400> 25
   atgatgagtg gctgtcttgt g 21
<210> 26
   <211> 22
   <212> DNA
   <213> Artificial
<220>
   <223> primer
<400> 26
   tgcgattgta gaggatgatg ga 22
<210> 27
   <211> 20
   <212> DNA
   <213> Artificial
<220>
   <223> primer
<400> 27
   acaatgccga atgccagaag 20
<210> 28
   <211> 20
   <212> DNA
   <213> Artificial
<220>
   <223> primer
<400> 28
   aggtttgggc ttggtcagtt 20
<210> 29
   <211> 24
   <212> DNA
   <213> Artificial
<220>
   <223> primer
<400> 29
   catacaggca tccaaatcaa gaag 24
<210> 30
   <211> 23
   <212> DNA
   <213> Artificial
<220>
   <223> primer
<400> 30
   gcagaatcag agtcagaaga gtc 23
<210> 31
   <211> 20
   <212> DNA
   <213> Artificial
<220>
   <223> primer
<400> 31
   ggaacttgct cgtgccttag 20
<210> 32
   <211> 21
   <212> DNA
   <213> Artificial
<220>
   <223> primer
<400> 32
   tctggcttgg agtctgaaac a 21
<210> 33
   <211> 20
   <212> DNA
   <213> Artificial
<220>
   <223> primer
<400> 33
   accaccacta cgcacatcac 20
<210> 34
   <211> 23
   <212> DNA
   <213> Artificial
<220>
   <223> primer
<400> 34
   tcgcttctca attcttgtct ctg 23
<210> 35
   <211> 21
   <212> DNA
   <213> Artificial
<220>
   <223> primer
<400> 35
   tgaccctgtt ttgtggcatt c 21
<210> 36
   <211> 21
   <212> DNA
   <213> Artificial
<220>
   <223> primer
<400> 36
   ggacacgatg accagatgaa c 21
<210> 37
   <211> 21
   <212> DNA
   <213> Artificial
<220>
   <223> primer
<400> 37
   gcttcggctt cagagaacaa c 21
<210> 38
   <211> 20
   <212> DNA
   <213> Artificial
<220>
   <223> primer
<400> 38
   ccagtcatag ggcagcacat 20
<210> 39
   <211> 23
   <212> DNA
   <213> Artificial
<220>
   <223> primer
<400> 39
   tttggtggtg gttagagata tgc 23
<210> 40
   <211> 20
   <212> DNA
   <213> Artificial
<220>
   <223> primer
<400> 40
   ccgaggtgcg tgaagaaatg 20
<210> 41
   <211> 22
   <212> DNA
   <213> Artificial
<220>
   <223> primer
<400> 41
   cgcctacttc ggtatctatg ac 22
<210> 42
   <211> 23
   <212> DNA
   <213> Artificial
<220>
   <223> primer
<400> 42
   ctgatgacga tgtgagtgtt ctt 23
<210> 43
   <211> 25
   <212> DNA
   <213> Artificial
<220>
   <223> primer
<400> 43
   aaactaagca caaagccatt ctaag 25
<210> 44
   <211> 24
   <212> DNA
   <213> Artificial
<220>
   <223> primer
<400> 44
   cactctattc tgtctcctca tcca 24
<210> 45
   <211> 21
   <212> DNA
   <213> Artificial
<220>
   <223> primer
<400> 45
   acaggtccag tggttcttca g 21
<210> 46
   <211> 21
   <212> DNA
   <213> Artificial
<220>
   <223> primer
<400> 46
   acagttcctc tttgcccttc a 21
<210> 47
   <211> 22
   <212> DNA
   <213> Artificial
<220>
   <223> primer
<400> 47
   ggctctccta ctaagaccac ag 22
<210> 48
   <211> 22
   <212> DNA
   <213> Artificial
<220>
   <223> primer
<400> 48
   gacgagtaac caaggctaac ag 22
<210> 49
   <211> 23
   <212> DNA
   <213> Artificial
<220>
   <223> primer
<400> 49
   aacaaagaca aactggagga aac 23
<210> 50
   <211> 20
   <212> DNA
   <213> Artificial
<220>
   <223> primer
<400> 50
   tgaggaggca ggaacttgag 20
<210> 51
   <211> 23
   <212> DNA
   <213> Artificial
<220>
   <223> primer
<400> 51
   gcaaagtcct caatggtaac aag 23
<210> 52
   <211> 21
   <212> DNA
   <213> Artificial
<220>
   <223> primer
<400> 52
   tgggtcagta aaggcaacat c 21
<210> 53
   <211> 23
   <212> DNA
   <213> Artificial
<220>
   <223> primer
<400> 53
   gccaacttct tcagcaacta atc 23
<210> 54
   <211> 20
   <212> DNA
   <213> Artificial
<220>
   <223> primer
<400> 54
   catcgtcgga accagtcatc 20
<210> 55
   <211> 22
   <212> DNA
   <213> Artificial
<220>
   <223> primer
<400> 55
   tcagaggcag aaatcaaagt cc 22
<210> 56
   <211> 20
   <212> DNA
   <213> Artificial
<220>
   <223> primer
<400> 56
   tgtaggtcag gtcggcaatc 20
<210> 57
   <211> 23
   <212> DNA
   <213> Artificial
<220>
   <223> primer
<400> 57
   atttattgga ttcacgcact gtc 23
<210> 58
   <211> 23
   <212> DNA
   <213> Artificial
<220>
   <223> primer
<400> 58
   tcatccactt caccaatctc ttc 23
<210> 59
   <211> 24
   <212> DNA
   <213> Artificial
<220>
   <223> primer
<400> 59
   cgacttctac gtggtcacct acac 24
<210> 60
   <211> 19
   <212> DNA
   <213> Artificial
<220>
   <223> primer
<400> 60
   cactccgaat ggcgttgtc 19
<210> 61
   <211> 24
   <212> DNA
   <213> Artificial
<220>
   <223> primer
<400> 61
   gcagatacct tccagagatg atag 24
<210> 62
   <211> 23
   <212> DNA
   <213> Artificial
<220>
   <223> primer
<400> 62
   ctgtgtccaa gtctcctaat gtg 23
<210> 63
   <211> 24
   <212> DNA
   <213> Artificial
<220>
   <223> primer
<400> 63
   tgtgccagtt ctttccataa taaa 24
<210> 64
   <211> 25
   <212> DNA
   <213> Artificial
<220>
   <223> primer
<400> 64
   atttcattct cctcactttg ttcat 25
<210> 65
   <211> 20
   <212> DNA
   <213> Artificial
<220>
   <223> primer
<400> 65
   gctgccaact tgatgttcca 20
<210> 66
   <211> 24
   <212> DNA
   <213> Artificial
<220>
   <223> primer
<400> 66
   catctgtaaa ctttgccttc tgta 24
<210> 67
   <211> 20
   <212> DNA
   <213> Artificial
<220>
   <223> primer
<400> 67
   ccagcatcac caccacaaat 20
<210> 68
   <211> 21
   <212> DNA
   <213> Artificial
<220>
   <223> primer
<400> 68
   gcagaaattc ccttcgtcct t 21
<210> 69
   <211> 20
   <212> DNA
   <213> Artificial
<220>
   <223> primer
<400> 69
   ttggcgtgag ttgcgtattc 20
<210> 70
   <211> 25
   <212> DNA
   <213> Artificial
<220>
   <223> primer
<400> 70
   gagactgaaa acgattacaa acatc 25
<210> 71
   <211> 24
   <212> DNA
   <213> Artificial
<220>
   <223> primer
<400> 71
   ttcttcttca tcttcctcct cttc 24
<210> 72
   <211> 21
   <212> DNA
   <213> Artificial
<220>
   <223> primer
<400> 72
   agcctccaga ttatcaccag a 21
<210> 73
   <211> 21
   <212> DNA
   <213> Artificial
<220>
   <223> primer
<400> 73
   gcctcctact acttccgttt c 21
<210> 74
   <211> 20
   <212> DNA
   <213> Artificial
<220>
   <223> primer
<400> 74
   tacatcccgc catcagtcac 20
<210> 75
   <211> 23
   <212> DNA
   <213> Artificial
<220>
   <223> primer
<400> 75
   gaggaactgg tagattacac gag 23
<210> 76
   <211> 20
   <212> DNA
   <213> Artificial
<220>
   <223> primer
<400> 76
   gtctgccctc ctgtcattca 20
<210> 77
   <211> 20
   <212> DNA
   <213> Artificial
<220>
   <223> primer
<400> 77
   caaggcgaag aagcacgaac 20
<210> 78
   <211> 22
   <212> DNA
   <213> Artificial
<220>
   <223> primer
<400> 78
   gccgagaagt tgagaaatgt ct 22
<210> 79
   <211> 22
   <212> DNA
   <213> Artificial
<220>
   <223> primer
<400> 79
   agacgatgag gatgccataa ag 22
<210> 80
   <211> 21
   <212> DNA
   <213> Artificial
<220>
   <223> primer
<400> 80
   cctcggtgga cagcaataat g 21
<210> 81
   <211> 24
   <212> DNA
   <213> Artificial
<220>
   <223> primer
<400> 81
   aaatgacaga tggtagagac ttcc 24
<210> 82
   <211> 22
   <212> DNA
   <213> Artificial
<220>
   <223> primer
<400> 82
   actggtaggt tacactggta gg 22
<210> 83
   <211> 25
   <212> DNA
   <213> Artificial
<220>
   <223> primer
<400> 83
   ggattatgtt gacctctacc ttatt 25
<210> 84
   <211> 25
   <212> DNA
   <213> Artificial
<220>
   <223> primer
<400> 84
   tttttccatt ttcatctttt gggat 25
<210> 85
   <211> 23
   <212> DNA
   <213> Artificial
<220>
   <223> primer
<400> 85
   gctacggaac cgattactgt gaa 23
<210> 86
   <211> 22
   <212> DNA
   <213> Artificial
<220>
   <223> primer
<400> 86
   gactggtaga gctgggagag ca 22
<210> 87
   <211> 23
   <212> DNA
   <213> Artificial
<220>
   <223> primer
<400> 87
   cgagagcatc ctgtaccgta agt 23
<210> 88
   <211> 21
   <212> DNA
   <213> Artificial
<220>
   <223> primer
<400> 88
   tgcttgccgt aggtgaagat c 21
<210> 89
   <211> 23
   <212> DNA
   <213> Artificial
<220>
   <223> primer
<400> 89
   cgtagactcc tcctctccca cat 23
<210> 90
   <211> 22
   <212> DNA
   <213> Artificial
<220>
   <223> primer
<400> 90
   tgggcgatat actgctcttc ct 22
<210> 91
   <211> 23
   <212> DNA
   <213> Artificial
<220>
   <223> primer
<400> 91
   ccagaaaaac agagtcgctg tgt 23
<210> 92
   <211> 25
   <212> DNA
   <213> Artificial
<220>
   <223> primer
<400> 92
   accctgtagc aagaagtagc tgatc 25
<210> 93
   <211> 20
   <212> DNA
   <213> Artificial
<220>
   <223> primer
<400> 93
   cgcaagtgaa ttccgaagga 20
<210> 94
   <211> 18
   <212> DNA
   <213> Artificial
<220>
   <223> primer
<400> 94
   acctggcgtc cctcaatg 18
<210> 95
   <211> 19
   <212> DNA
   <213> Artificial
<220>
   <223> primer
<400> 95
   gcgagctgat cctcaaacg 19
<210> 96
   <211> 22
   <212> DNA
   <213> Artificial
<220>
   <223> primer
<400> 96
   cgcctcgctc tttatcttct tc 22
<210> 97
   <211> 16
   <212> DNA
   <213> Artificial
<220>
   <223> primer
<400> 97
   ccggctacgg caagca 16
<210> 98
   <211> 23
   <212> DNA
   <213> Artificial
<220>
   <223> primer
<400> 98
   tggatgggta cagtctgaca tga 23
<210> 99
   <211> 19
   <212> DNA
   <213> Artificial
<220>
   <223> primer
<400> 99
   tgccgctttg caggtgtat 19
<210> 100
   <211> 17
   <212> DNA
   <213> Artificial
<220>
   <223> primer
<400> 100
   ggcctccgtc cgagaga 17
<210> 101
   <211> 27
   <212> DNA
   <213> Artificial
<220>
   <223> primer
<400> 101
   tccagtagct gcttgtctcc tactata 27
<210> 102
   <211> 22
   <212> DNA
   <213> Artificial
<220>
   <223> primer
<400> 102
   tcttaggtct cgcaggctgt ct 22
<210> 103
   <211> 22
   <212> DNA
   <213> Artificial
<220>
   <223> primer
<400> 103
   cctttcttat ccgcgagagt ga 22
<210> 104
   <211> 24
   <212> DNA
   <213> Artificial
<220>
   <223> primer
<400> 104
   ggtctccttt catatcatcc caat 24
<210> 105
   <211> 20
   <212> DNA
   <213> Artificial
<220>
   <223> primer
<400> 105
   tgtcacccag gttcgtctca 20
<210> 106
   <211> 25
   <212> DNA
   <213> Artificial
<220>
   <223> primer
<400> 106
   catgtctgta accctagcac tttgg 25
<210> 107
   <211> 24
   <212> DNA
   <213> Artificial
<220>
   <223> primer
<400> 107
   actttccaat ggacatgagt gatg 24
<210> 108
   <211> 22
   <212> DNA
   <213> Artificial
<220>
   <223> primer
<400> 108
   taggagggaa ttgcatgtgc tt 22
<210> 109
   <211> 21
   <212> DNA
   <213> Artificial
<220>
   <223> primer
<400> 109
   atcccaaagc aagacaacca g 21
<210> 110
   <211> 21
   <212> DNA
   <213> Artificial
<220>
   <223> primer
<400> 110
   ccaggcaaat gaggaagaga c 21
<210> 111
   <211> 22
   <212> DNA
   <213> Artificial
<220>
   <223> primer
<400> 111
   cgcgcttgag ttaacatgtg aa 22
<210> 112
   <211> 22
   <212> DNA
   <213> Artificial
<220>
   <223> primer
<400> 112
   tcgactctca cccaagttac ca 22
<210> 113
   <211> 25
   <212> DNA
   <213> Artificial
<220>
   <223> primer
<400> 113
   gggcattagg agtgctaatc tatga 25
<210> 114
   <211> 22
   <212> DNA
   <213> Artificial
<220>
   <223> primer
<400> 114
   atctgaattg gttggtctgc aa 22
<210> 115
   <211> 24
   <212> DNA
   <213> Artificial
<220>
   <223> primer
<400> 115
   gagaacacag ggcaagacac atac 24
<210> 116
   <211> 22
   <212> DNA
   <213> Artificial
<220>
   <223> primer
<400> 116
   ggaccagctg atacagaatg ca 22
<210> 117
   <211> 19
   <212> DNA
   <213> Artificial
<220>
   <223> primer
<400> 117
   gggaagcgag atggcactt 19
<210> 118
   <211> 23
   <212> DNA
   <213> Artificial
<220>
   <223> primer
<400> 118
   caccactaca gagcaggcat agc 23
<210> 119
   <211> 19
   <212> DNA
   <213> Artificial
<220>
   <223> primer
<400> 119
   atttgggtcg cggttcttg 19
<210> 120
   <211> 21
   <212> DNA
   <213> Artificial
<220>
   <223> primer
<400> 120
   tgccttgaca ttctcgatgg t 21
<210> 121
   <211> 21
   <212> DNA
   <213> Artificial
<220>
   <223> primer
<400> 121
   tgacactggc aaaacaatgc a 21
<210> 122
   <211> 21
   <212> DNA
   <213> Artificial
<220>
   <223> primer
<400> 122
   ggtccttttc accagcaagc t 21
<210> 123
   <211> 16
   <212> DNA
   <213> Artificial
<220>
   <223> primer
<400> 123
   ggcaatgcgg ctgcaa 16
<210> 124
   <211> 19
   <212> DNA
   <213> Artificial
<220>
   <223> primer
<400> 124
   gggtacccac gcgaatcac 19
<210> 125
   <211> 20
   <212> DNA
   <213> Artificial
<220>
   <223> primer
<400> 125
   tgggaacaag agggcatctg 20
<210> 126
   <211> 22
   <212> DNA
   <213> Artificial
<220>
   <223> primer
<400> 126
   ccaccactgc atcaaattca tg 22
<210> 127
   <211> 21
   <212> DNA
   <213> Artificial
<220>
   <223> primer
<400> 127
   catggtgaaa ccccgtctct a 21
<210> 128
   <211> 19
   <212> DNA
   <213> Artificial
<220>
   <223> primer
<400> 128
   gcctcagcct cccgagtag 19
<210> 129
   <211> 55
   <212> DNA
   <213> Artificial
<220>
   <223> oligonucleotide
<400> 129
   tcagcaacac tagcacttca cactgactga ctgtagttat gtctgagaag tcctt 55
<210> 130
   <211> 55
   <212> DNA
   <213> Artificial
<220>
   <223> oligonucleotide
<400> 130
   gaactgccag aagatttgta tgatgactga ttacatttgc ctaacatctt tggac 55
<210> 131
   <211> 55
   <212> DNA
   <213> Artificial
<220>
   <223> oligonucleotide
<400> 131
   ttgtcaccca tacccatttc ttacactgac tgaagagaaa gttgaagagt cccag 55
<210> 132
   <211> 55
   <212> DNA
   <213> Artificial
<220>
   <223> oligonucleotide
<400> 132
   ggagcaccta cagaagcaaa ctctgactga ctctcattca gtgccatctc atacc 55
<210> 133
   <211> 55
   <212> DNA
   <213> Artificial
<220>
   <223> oligonucleotide
<400> 133
   ctgcaggtgc tgactgacaa gactgactga ctgactggag tttggccggg ctttc 55
<210> 134
   <211> 55
   <212> DNA
   <213> Artificial
<220>
   <223> oligonucleotide
<400> 134
   tcggagcctg taacctacta tgactgactg actgattcag gaggtggatg gtgtg 55
<210> 135
   <211> 55
   <212> DNA
   <213> Artificial
<220>
   <223> oligonucleotide
<400> 135
   ggctaaggga aatgctggta aagactgact gagtgaacct aacaatacct gcctc 55
<210> 136
   <211> 55
   <212> DNA
   <213> Artificial
<220>
   <223> oligonucleotide
<400> 136
   aagagatcac cggcgtaatc aaactgactg actgtgagaa tcatagctga gcccg 55
<210> 137
   <211> 55
   <212> DNA
   <213> Artificial
<220>
   <223> oligonucleotide
<400> 137
   cagccagcat tcattgtaag ttcactgact gactttctgt gtctggtggg tatgg 55
<210> 138
   <211> 55
   <212> DNA
   <213> Artificial
<220>
   <223> oligonucleotide
<400> 138
   tactcctacg gtttcgccta cactgactga ctgttctcag cggtgtcatc tatgt 55
<210> 139
   <211> 55
   <212> DNA
   <213> Artificial
<220>
   <223> oligonucleotide
<400> 139
   gatggactgg gacgactctg actgactgac tgacttatga ggaccagcac agcag 55
<210> 140
   <211> 55
   <212> DNA
   <213> Artificial
<220>
   <223> oligonucleotide
<400> 140
   tagtgtttaa gaaagcaagc aagtcactga ctgacacatt tctgaccgaa ccctc 55
<210> 141
   <211> 55
   <212> DNA
   <213> Artificial
<220>
   <223> oligonucleotide
<400> 141
   atgatgagtg gctgtcttgt gactgactga ctgtccatca tcctctacaa tcgca 55
<210> 142
   <211> 55
   <212> DNA
   <213> Artificial
<220>
   <223> oligonucleotide
<400> 142
   acaatgccga atgccagaag actgactgac tgactaactg accaagccca aacct 55
<210> 143
   <211> 55
   <212> DNA
   <213> Artificial
<220>
   <223> oligonucleotide
<400> 143
   catacaggca tccaaatcaa gaagactgac tggactcttc tgactctgat tctgc 55
<210> 144
   <211> 55
   <212> DNA
   <213> Artificial
<220>
   <223> oligonucleotide
<400> 144
   ggaacttgct cgtgccttag actgactgac tgactgtttc agactccaag ccaga 55
<210> 145
   <211> 55
   <212> DNA
   <213> Artificial
<220>
   <223> oligonucleotide
<400> 145
   accaccacta cgcacatcac actgactgac tgcagagaca agaattgaga agcga 55
<210> 146
   <211> 55
   <212> DNA
   <213> Artificial
<220>
   <223> oligonucleotide
<400> 146
   tgaccctgtt ttgtggcatt cactgactga ctgagttcat ctggtcatcg tgtcc 55
<210> 147
   <211> 55
   <212> DNA
   <213> Artificial
<220>
   <223> oligonucleotide
<400> 147
   gcttcggctt cagagaacaa cactgactga ctgacatgtg ctgccctatg actgg 55
<210> 148
   <211> 55
   <212> DNA
   <213> Artificial
<220>
   <223> oligonucleotide
<400> 148
   tttggtggtg gttagagata tgcactgact gactgcattt cttcacgcac ctcgg 55
<210> 149
   <211> 55
   <212> DNA
   <213> Artificial
<220>
   <223> oligonucleotide
<400> 149
   cgcctacttc ggtatctatg acactgactg acaagaacac tcacatcgtc atcag 55
<210> 150
   <211> 55
   <212> DNA
   <213> Artificial
<220>
   <223> oligonucleotide
<400> 150
   aaactaagca caaagccatt ctaagactga ctggatgagg agacagaata gagtg 55
<210> 151
   <211> 55
   <212> DNA
   <213> Artificial
<220>
   <223> oligonucleotide
<400> 151
   acaggtccag tggttcttca gactgactga ctgatgaagg gcaaagagga actgt 55
<210> 152
   <211> 55
   <212> DNA
   <213> Artificial
<220>
   <223> oligonucleotide
<400> 152
   ggctctccta ctaagaccac agactgactg actctgttag ccttggttac tcgtc 55
<210> 153
   <211> 55
   <212> DNA
   <213> Artificial
<220>
   <223> oligonucleotide
<400> 153
   aacaaagaca aactggagga aacactgact gactgctcaa gttcctgcct cctca 55
<210> 154
   <211> 55
   <212> DNA
   <213> Artificial
<220>
   <223> oligonucleotide
<400> 154
   gcaaagtcct caatggtaac aagactgact gactgatgtt gcctttactg accca 55
<210> 155
   <211> 55
   <212> DNA
   <213> Artificial
<220>
   <223> oligonucleotide
<400> 155
   gccaacttct tcagcaacta atcactgact gactggatga ctggttccga cgatg 55
<210> 156
   <211> 55
   <212> DNA
   <213> Artificial
<220>
   <223> oligonucleotide
<400> 156
   tcagaggcag aaatcaaagt ccactgactg actgagattg ccgacctgac ctaca 55
<210> 157
   <211> 55
   <212> DNA
   <213> Artificial
<220>
   <223> oligonucleotide
<400> 157
   atttattgga ttcacgcact gtcactgact gagaagagat tggtgaagtg gatga 55
<210> 158
   <211> 55
   <212> DNA
   <213> Artificial
<220>
   <223> oligonucleotide
<400> 158
   cgacttctac gtggtcacct acacactgac tgactggaca acgccattcg gagtg 55
<210> 159
   <211> 55
   <212> DNA
   <213> Artificial
<220>
   <223> oligonucleotide
<400> 159
   gcagatacct tccagagatg atagactgac tgcacattag gagacttgga cacag 55
<210> 160
   <211> 55
   <212> DNA
   <213> Artificial
<220>
   <223> oligonucleotide
<400> 160
   tgtgccagtt ctttccataa taaaactgac atgaacaaag tgaggagaat gaaat 55
<210> 161
   <211> 55
   <212> DNA
   <213> Artificial
<220>
   <223> oligonucleotide
<400> 161
   gctgccaact tgatgttcca actgactgac ttacagaagg caaagtttac agatg 55
<210> 162
   <211> 55
   <212> DNA
   <213> Artificial
<220>
   <223> oligonucleotide
<400> 162
   ccagcatcac caccacaaat actgactgac tgacaaggac gaagggaatt tctgc 55
<210> 163
   <211> 55
   <212> DNA
   <213> Artificial
<220>
   <223> oligonucleotide
<400> 163
   ttggcgtgag ttgcgtattc actgactgac gatgtttgta atcgttttca gtctc 55
<210> 164
   <211> 55
   <212> DNA
   <213> Artificial
<220>
   <223> oligonucleotide
<400> 164
   ttcttcttca tcttcctcct cttcactgac tgactctggt gataatctgg aggct 55
<210> 165
   <211> 55
   <212> DNA
   <213> Artificial
<220>
   <223> oligonucleotide
<400> 165
   gcctcctact acttccgttt cactgactga ctgacgtgac tgatggcggg atgta 55
<210> 166
   <211> 55
   <212> DNA
   <213> Artificial
<220>
   <223> oligonucleotide
<400> 166
   gaggaactgg tagattacac gagactgact gactgtgaat gacaggaggg cagac 55
<210> 167
   <211> 55
   <212> DNA
   <213> Artificial
<220>
   <223> oligonucleotide
<400> 167
   caaggcgaag aagcacgaac actgactgac tgaagacatt tctcaacttc tcggc 55
<210> 168
   <211> 55
   <212> DNA
   <213> Artificial
<220>
   <223> oligonucleotide
<400> 168
   agacgatgag gatgccataa agactgactg actgcattat tgctgtccac cgagg 55
<210> 169
   <211> 55
   <212> DNA
   <213> Artificial
<220>
   <223> oligonucleotide
<400> 169
   aaatgacaga tggtagagac ttccactgac tgacctacca gtgtaaccta ccagt 55
<210> 170
   <211> 55
   <212> DNA
   <213> Artificial
<220>
   <223> oligonucleotide
<400> 170
   ggattatgtt gacctctacc ttattactga atcccaaaag atgaaaatgg aaaaa 55
<210> 171
   <211> 55
   <212> DNA
   <213> Artificial
<220>
   <223> oligonucleotide
<400> 171
   gctacggaac cgattactgt gaaactgact gactgctctc ccagctctac cagtc 55
<210> 172
   <211> 55
   <212> DNA
   <213> Artificial
<220>
   <223> oligonucleotide
<400> 172
   cgagagcatc ctgtaccgta agtactgact gactgatctt cacctacggc aagca 55
<210> 173
   <211> 55
   <212> DNA
   <213> Artificial
<220>
   <223> oligonucleotide
<400> 173
   cgtagactcc tcctctccca catactgact gacaggaaga gcagtatatc gccca 55
<210> 174
   <211> 55
   <212> DNA
   <213> Artificial
<220>
   <223> oligonucleotide
<400> 174
   ccagaaaaac agagtcgctg tgtactgact gatcagctac ttcttgctac agggt 55
<210> 175
   <211> 55
   <212> DNA
   <213> Artificial
<220>
   <223> oligonucleotide
<400> 175
   cgcaagtgaa ttccgaagga actgactgac tgactgacat tgagggacgc caggt 55
<210> 176
   <211> 55
   <212> DNA
   <213> Artificial
<220>
   <223> oligonucleotide
<400> 176
   gcgagctgat cctcaaacga ctgactgact gacgaagaag ataaagagcg aggcg 55
<210> 177
   <211> 55
   <212> DNA
   <213> Artificial
<220>
   <223> oligonucleotide
<400> 177
   ccggctacgg caagcaactg actgactgaa tgtcatgtca gactgtaccc atcca 55
<210> 178
   <211> 55
   <212> DNA
   <213> Artificial
<220>
   <223> oligonucleotide
<400> 178
   tgccgctttg caggtgtata ctgactgact gactgacttc tctcggacgg aggcc 55
<210> 179
   <211> 55
   <212> DNA
   <213> Artificial
<220>
   <223> oligonucleotide
<400> 179
   tccagtagct gcttgtctcc tactataact gacagacagc ctgcgagacc taaga 55
<210> 180
   <211> 55
   <212> DNA
   <213> Artificial
<220>
   <223> oligonucleotide
<400> 180
   cctttcttat ccgcgagagt gaactgactg aattgggatg atatgaaagg agacc 55
<210> 181
   <211> 55
   <212> DNA
   <213> Artificial
<220>
   <223> oligonucleotide
<400> 181
   tgtcacccag gttcgtctca actgactgaa ccaaagtgct agggttacag acatg 55
<210> 182
   <211> 55
   <212> DNA
   <213> Artificial
<220>
   <223> oligonucleotide
<400> 182
   actttccaat ggacatgagt gatgactgac tgaaagcaca tgcaattccc tccta 55
<210> 183
   <211> 55
   <212> DNA
   <213> Artificial
<220>
   <223> oligonucleotide
<400> 183
   atcccaaagc aagacaacca gactgactga ctgagtctct tcctcatttg cctgg 55
<210> 184
   <211> 55
   <212> DNA
   <213> Artificial
<220>
   <223> oligonucleotide
<400> 184
   cgcgcttgag ttaacatgtg aaactgactg acttggtaac ttgggtgaga gtcga 55
<210> 185
   <211> 55
   <212> DNA
   <213> Artificial
<220>
   <223> oligonucleotide
<400> 185
   gggcattagg agtgctaatc tatgaactga ctgttgcaga ccaaccaatt cagat 55
<210> 186
   <211> 55
   <212> DNA
   <213> Artificial
<220>
   <223> oligonucleotide
<400> 186
   gagaacacag ggcaagacac atacactgac tgatgcattc tgtatcagct ggtcc 55
<210> 187
   <211> 55
   <212> DNA
   <213> Artificial
<220>
   <223> oligonucleotide
<400> 187
   gggaagcgag atggcactta ctgactgact gagctatgcc tgctctgtag tggtg 55
<210> 188
   <211> 55
   <212> DNA
   <213> Artificial
<220>
   <223> oligonucleotide
<400> 188
   atttgggtcg cggttcttga ctgactgact gactaccatc gagaatgtca aggca 55
<210> 189
   <211> 55
   <212> DNA
   <213> Artificial
<220>
   <223> oligonucleotide
<400> 189
   tgacactggc aaaacaatgc aactgactga ctgaagcttg ctggtgaaaa ggacc 55
<210> 190
   <211> 55
   <212> DNA
   <213> Artificial
<220>
   <223> oligonucleotide
<400> 190
   ggcaatgcgg ctgcaaactg actgactgac tgactggtga ttcgcgtggg taccc 55
<210> 191
   <211> 55
   <212> DNA
   <213> Artificial
<220>
   <223> oligonucleotide
<400> 191
   tgggaacaag agggcatctg actgactgac tgacatgaat ttgatgcagt ggtgg 55
<210> 192
   <211> 55
   <212> DNA
   <213> Artificial
<220>
   <223> oligonucleotide
<400> 192
   catggtgaaa ccccgtctct aactgactga ctgactctac tcgggaggct gaggc 55